# EUROPEAN PATENT SPECIFICATION

(11) **EP 1 335 976 B1**
(45) Date of publication and mention of the grant of the patent: **18.04.2007**
(21) Application number: 01989507.7
(22) Date of filing: 23.11.2001
(51) Int. Cl.: C12N 15/12, C07K 14/47, C07K 14/46, C07K 14/435, C12N 5/10, C12N 15/62, C07K 16/18, C12Q 1/68, A61K 38/00, A61K 39/00, A01K 67/027, A01K 67/033, G01N 33/50, G01N 33/53

(54) **MODIFIER OF ORGANELLE METALBOLISM**
MODIFIZIERUNGSMITTEL DES ORGANELL-STOFFWECHSELS
MODIFICATEUR DE METABOLISME D'ORGANELLE

(30) Priority: 23.11.2000 EP 00125693
(43) Date of publication of application: 20.08.2003
(73) Proprietor: DeveloGen Aktiengesellschaft, 37079 Göttingen (DE)
(72) Inventor: STEUERNAGEL, Arnd, 37085 Göttingen (DE); BRÖNNER, Günter, 37077 Göttingen (DE); DOHRMANN, Cord, 37077 Göttingen (DE); CIOSSEK, Thomas, 37083 Göttingen (DE); WEHR, Roland, 37075 Göttingen (DE); RUDOLPH, Bettina, 30161 Hannover (DE); RUDOLPH, Dorothea, 37083 Göttingen (DE)
(74) Representative: Weiss, Wolfgang
(86) International application number: PCT/EP2001/013663
(87) International publication number: WO 2002/042455

(56) References cited:
- DATABASE EMBL [Online] Preliminary; PRT; 360 AA, 1 May 2000 (2000-05-01) ADAMS M. ET AL.: "CG8026 protein" Database accession no. Q9V551 XP002205574
- DATABASE EMBL [Online] standard; DNA; HTG; 51430 bp, 14 December 1999 (1999-12-14) ADAMS M ET AL.: "Drosophila melanogaster, WORKING DRAFT SEQUENCE, in ordered pieces." Database accession no. AC017981 XP002205575
- DATABASE EMBL [Online] standard; RNA; EST; 731 bp, 17 November 1999 (1999-11-17) SUGANO ET AL.: "fi40c12.y1 Sugano Kawakami zebrafish DRA Danio rerion cDNA clone 2640118 5' smilar to WP:K01C8.7 CE02268 MITOCHONDRIAL CARRIER PROTEINS; mRNA sequence" Database accession no. AW174196 XP002205576
- TITUS S. AND MORAN R.: "Retrovirally Mediated Complementation of the glyB Phenotype" JOURNAL OF BIOLOGICAL CHEMISTRY, PAPERS IN PRESS, vol. 275, no. 47, 7 September 2000 (2000-09-07), pages 36811-36817, XP002205572 USA -& DATABASE EMBL [Online] standard; RNA; HUM; 2534 bp, TITUS S. AND MORAN R.: "Homo sapiens folate transporter/carrier mRNA, complete cds; nuclear gene for mitochondrial product" XP002174537
- DATABASE EMBL [Online] standard; cDNA; 2676 bp, RUBEN S.M ET AL.: "Human uncoupling protein cDNA#8" Database accession no. AAC90459 XP002205577 -& WO 00 61614 A (HUMAN GENOME SCIENCES INC ;NI JIAN (US); ROSEN CRAIG A (US); RUBEN) 19 October 2000 (2000-10-19)
- RICQUIER DANIEL ET AL: "The uncoupling protein homologues: UCP1, UCP2, UCP3, StUCP and AtUCP" BIOCHEMICAL JOURNAL, THE BIOCHEMICAL SOCIETY, LONDON, GB, vol. 345, no. 2, 15 January 2000 (2000-01-15), pages 161-179, XP002180562 ISSN: 0264-6021 cited in the application
- ROQUES B P: "PEPTIDOMIMETICS AS RECEPTORS AGOINSTS OR PEPTIDASE INHIBITORS: A STRUCTURAL APPROACH IN THE FIELD OF ENKELPHALINS, ANP AND CCK" BIOPOLYMERS, NEW YORK, NY, US, vol. 32, no. 4, April 1992 (1992-04), pages 407-410, XP001041605 ISSN: 0006-3525
- DATABASE EMBL [Online] standard; cDNA; 2357 bp, OTA T. ET AL.: "Human cDNA sequence SEQ ID NO: 14686" Database accession no. AAH16028 XP002205578 -& EP 1 074 617 A (HELIX RES INST.) 7 February 2001 (2001-02-07)
- DATABASE EMBL [Online] standard; Protein; 315 AA, OTA T. ET AL.: "Human protein sequence SEQ ID NO:14687" Database accession no. AAB94269 XP002205579 -& EP 1 074 617 A (HELIX RES INST) 7 February 2001 (2001-02-07)
- DATABASE EMBL [Online] standard DNA; HUM; 3599 bp, YUE ET AL.: "Sequence 14 from Patent WO0162923" Database accession no. AX230560 XP002205580 -& WO 01 62923 A (HERNANDEZ ROBERTO ;INCYTE GENOMICS INC (US); PATTERSON CHANDRA (US) 30 August 2001 (2001-08-30)
- HANAK P. AND JEZEK P.: "Mitochondrial uncoupling proteins and phylogenesis - UCP4 as the ancestral uncoupling protein" FEBS LETTERS, ELSEVIER SCIENCE B.V., vol. 495, 28 March 2001 (2001-03-28), pages 137-141, XP002205573 Amsterdam

## Description

The present invention relates to the use of (i) a nucleic acid molecule encoding a polypeptide contributing to membrane stability and/or function of organelles, wherein said nucleic acid molecule (a) remains hybridized under herein defined stringent conditions to a nucleic acid molecule encoding the amino acid sequence as disclosed herein and/or the complementary strand thereof; (b) remains hybridized under herein defined conditions to a nucleic acid molecule as disclosed herein and/or the complementary strand thereof; (c) is degenerate with respect to the nucleic acid molecule of (a) or (b); (d) encodes a polypeptide which is at least 90% identical to a herein disclosed amino acid sequence representing a polynucleotide contributing to membrane stability and/or function of organelles; (e) has the sequence as disclosed herein; (ii) a polypeptide encoded by said nucleic acid molecule; (iii) a fusion protein comprising said polypeptide; (iv) an antibody, fragment or derivative thereof or an aptamer specifically recognizing said nucleic acid molecule, polypeptide or fusion protein; or (v) an antisense oligonucleotide, ribozyme, hybridization probe or amplification primer of said nucleic acid molecule for the manufacture of a diagnostic and/or pharmaceutical composition for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction. Furthermore, the invention provides for the use of a non-human transgenic animal comprising a nucleic acid molecule encoding a polypeptide contributing to membrane stability and/or function of organelles as defined above; expressing a polypeptide encoded by said nucleic acid molecule or a fusion protein comprising said polypeptide;or transfected with a vector comprising said nucleic acid molecule as a research model for obesity, adipositas, eating disorders, wasting and/or other disorders of body weight/body mass. The invention also relates to an in vitro method of identifying a polypeptide or (a) substance(s) involved in a metabolic disorder selected from obesity, adipositas, eating disorders (bulimia nervosa, anorexia nervosa), cachexia (wasting) and pancreatic dysfunction. The invention also describes an *in vitro* method for identifying a polypeptide involved in the regulation of body weight in a mammal and a method of identifying a compound for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction. Furthermore, the invention provides for a method of assessing the impact of the expression of one or more polypeptides encoded by a nucleic acid molecule encoding a polypeptide contributing to membrane stability and/or function of organelles as defined above or of one or more fusion proteins comprising said polypeptide in a non-human animal. The invention also relates to a method of screening for an agent which modulates the interaction of a polypeptide encoded by a nucleic acid molecule encoding a polypeptide contributing to membrane stability and/or function of organelles as defined above, which is capable of detecting, verifying, treating, alleviating and/or preventing a metabolic disorder selected from obesity, adipositas, eating disorders (bulimia nervosa, anorexia nervosa), cachexia (wasting) and pancreatic dysfunction, with a binding target/agent. Finally, the invention provides for a kit for the detection and/or verification or for the treatment, alleviation and/or prevention of a metabolic disorder, selected from obesity, adipositas, eating disorders (bulimia nervosa, anorexia nervosa), cachexia (wasting) and pancreatic dysfunction, as diagnostic and/or pharmaceutical composition.

Mitochondria are the energy suppliers of animal cells. Most of the energy available from metabolising foodstuffs like carbohydrates, fats etc. is used to create a proton gradient across the inner mitochondrial membrane. This proton gradient drives the enzyme ATP synthetase that produces ATP, the cells major fuel substance (Mitchell P, Science 206, 1979, 1148-1159). In the mitochondria of brown adipose tissue exists a protein (Uncoupling Protein 1) that tunnels protons through the inner mitochondrial membrane (review in Klingenberg M, Huang SG, Biochim Biophys Acta 1999, 1415(2):271-96). The energy stored in the proton gradient is thereby released as heat and not used for ATP synthesis.

When the energy intake of an animal exceeds expenditure surplus energy can be stored as fat in adipose tissue. The generation of a proton leak across the inner mitochondrial membrane by the activation of uncoupling proteins would reduce caloric efficiency and thus avoid the accumulation of excess body fat (obesity) that is detrimental to the animals health. In human, however, brown adipose tissue is almost absent in adults. Therefore UCP1 was not considered to be a major factor in the formation or prevention of human obesity. Recently the discovery of further proteins of similar sequence (UCP2-UCP5) that are widely expressed in human tissues (e.g. white adipose tissue, muscle) made this members of the UCP family to important targets for pharmaceutical research (reviewed in Adams SH, Nutr 2000, 130(4):711-4). Interestingly, and as reviewed in Ricquier, Biochem J. 345 (2000), 161-179, further homologues have been identified, like, inter alia, the plant UCPs StUCP (from Solanum tuberculosum) and AtUCP (Arabidopsis thaliana). Although the in vivo function of these proteins is still unknown, the possibility to influence UCP activity would be a conceivable therapy for the treatment or prevention of obesity and related diseases.

Mitochondria have a very specialized function in energy conversion and said function is reflected in their morphological structure, namely the distinct internal membrane. This internal membrane does not only provide the framework for electron-transport processes but also creates a large internal compartment in each organelle in which highly specialized enzymes are confined. Therefore, there is a strong relationship between mitochondrial energy metabolism and the biochemical/biophysical properties of these organelles.

The technical problem underlying the invention was to provide for means and methods for modulating the biological/biochemical activities of mitochondria and, thereby, modulating metabolic conditions in eukaryotic cells which influence energy expenditure, body temperature, thermogenesis, cellular metabolism to an excessive or deficient supply of substrate(s) in order to regulate the ATP level, the NAD⁺/NADH ratio, and/or superoxide production.

The solution to this technical problem is achieved by providing the embodiments characterized in the claims. As documented in the appended examples, the present invention provides for genes and gene products which are either directly or indirectly involved in membrane stability and/or function of organelles, in particular of mitochondria. In particular, the present invention provides for soup1 genes and gene products. It is envisaged that mutations in the herein described genes and gene products (encoding SOUP1-polypeptides) lead to phenotypic and/or physiological changes which may comprise a modified and altered mitochondrial activity. This, in turn, may lead to, inter alia, an altered energy metabolism, altered thermogenesis and/or altered energy homeostasis. As demonstrated in the appended examples, the here described polypeptide (and encoding nucleic acid molecule) was able to modify, e.g. suppress a specific eye phenotype in Drosophila which was due to the overexpression of the Drosophila melanogaster gene dUCPy. The overexpression of dUCP (with homology to human UCPs) in the compound eye of Drosophila led to a clearly visible eye defect (see appended examples and figures) which can be used as a "read-out" for a genetical "modifier screen". In said "modifier screen" thousands of different genes are mutagenized to activate their expression in the eye. Should one of the mutagenized genes interact with dUCPy and modify its activity an enhancement or suppression of the eye defect will occur. Since such flies are easily to discern they can be selected to isolate the interacting gene. As shown in the appended examples, a gene was deduced that can suppress the eye defect induced by the activity of dUCPy. This gene is called Suppressor Of Uncoupling Protein 1 (SOUP1).

More specifically, in a first aspect the present invention relates to the use of
(i) a nucleic acid molecule encoding a polypeptide, contributing by means of protein-protein interactions as well as protein-lipid interactions to membrane stability and/or contributing to membrane function of organelles by having transport functions, regulator functions of other membrane proteins or modifier functions of other (membrane) proteins and/or other functions,
(ii) a polypeptide encoded by said nucleic acid,
(iii) a fusion protein comprising said polypeptide,
(iv) an antibody, fragment or derivative thereof or an aptamer specifically recognizing said nucleic acid, polypeptide or fusion protein,
(v) an antisense oligonucleotide, ribozyme, hybridization probe or amplification primer of said nucleic acid molecule
for the manufacture of a diagnostic and/or pharmaceutical composition for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction, wherein said nucleic acid molecule
(a) remains hybridized at 65°C in a solution containing 0.2 x SSC and 0.1 % SDS to a nucleic acid molecule encoding the amino acid sequence of SEQ ID NO: 10, SEQ ID NO: 12 and/or SEQ ID NO: 14 and/or the complementary strand thereof;
(b) remains hybridized at 65°C in a solution containing 0.2 x SSC and 0.1% SDS to a nucleic acid molecule as depicted in SEQ ID NO: 6, SEQ ID NO: 9, SEQ ID NO: 11 and/or SEQ ID NO: 13 and/or the complementary strand thereof;
(c) is degenerate with respect to the nucleic acid molecule of (a) or (b);
(d) encodes a polypeptide which is at least 90% identical to the amino acid sequence as depicted in any one of SEQ ID NOs: 10, 12 or 14; or
(e) has the sequence as depicted in SEQ ID NOs: 9, 11, 13 or 51.

The term "membrane stability" as used herein comprises not only the overall stability but also comprises local stabilities on membranes of organelles, for example of the inner and outer membrane, but in particular of the inner membrane. The term "membrane stability" relates, therefore, to structural features of the membranes, provided by protein-protein interactions as well as by protein-lipid interactions leading to a defined membrane composition.

The term "contributing to membrane function of organelles" as employed herein above relates to functions of the above defined polypeptide comprising, inter alia, transport functions (like active and passive transport of ions, metabolites, vitamines, etc.), regulator functions of other membrane proteins (like transporters, carriers) or modifier functions of other (membrane) proteins (like enhancement/suppressor functions) and/or other functions as defined herein below.

The term "organelles" as employed herein not only relates to mitochondria but also to further organelles, like peroxisomes or plant cell organelles, e.g. chloroplasts.

The terms "hybridizes" and "hybridizing" as employed in context of the present invention preferably relate to stringent conditions as, inter alia, defined herein above, e.g. 0.2 x SSC, 0.1% SDS at 65°C. Said conditions comprise hybridization conditions and particularly washing conditions. It is preferred that washing conditions are more stringent than hybridization conditions. By setting the conditions for hybridization, the person skilled in the art can determine if strictly complementary sequences or sequences with a higher or lower degree of homology are to be detected. The setting of conditions is well within the skill of the artisan and to be determined according to protocols described, for example, in Sambrook, Molecular Cloning, A Laboratory Manual, 2^{nd} edition (1989), Cold Spring Harbor Laboratory Press, Cold Spring Harbor, NY or Hames and Higgins, "Nucleic acid hybridization, a practical approach", IRL Press, Oxford (1985). Non-stringent hybridization conditions for the detection of homologous and not exactly complementary sequences may be set at 6 x SSC, 1% SDS at 65°C.

The molecules hybridizing to the nucleic acid molecules also comprise fragments, derivatives and allelic variants of the above described nucleic acid molecules which encode (poly)peptides regulating, causing or contributing to obesity described in the present invention. In this regard, fragments are defined as parts of the nucleic acid molecules, which are long enough in order to encode said (poly)peptides. The term "derivatives" means that the sequences of these hybridizing molecules differ from the sequences of the above-mentioned nucleic acid molecules at one or more positions and that they exhibit a high degree of homology to these sequences. For example, homology means a sequence identity to sequences as identified e.g. in SEQ ID NOs: 10, 12, 14 or 52 of at least 35%, in particular an identity of at least 45 %, preferably of more than 50%, more preferably more than 60%, more preferably more than 70%, more preferably more than 80% and still more preferably a sequence identity of more than 90%. Considering e.g. a sequence identified as SEQ ID NO: 8 said homology means a sequence identity of at least 85%. For the sequence depicted e.g. in SEQ ID NO: 54, a sequence identity of at least 90% is considered "homologous". The person skilled in the art may employ computer programs and packages in order to determine homology values. Generally, nucleotide or amino acid sequence identities/homologies can be determined conventionally by using known computer programs such as BLASTIN, BLASTP, NALIGN, PALIGN or bl2seq using particular algorithms to find the best segment of homology between two segments.

As shown in the appended examples, in the context of the present invention the comparative analysis of the percentage of identities at the amino acid level are preferably obtained using the "bl2seq" program from NCBl using the following parameters: Open Gap Cost: 11 and Gap Extension Cost: 1. However, the program allows any positive integer for said value(s).

Furthermore, in the context of the present invention comparative analysis of the percentage of identities at the nucleotide level of the different nucleic acid sequences, on the other hand, are preferably obtained using the "matcher" program of the EMBOSS package using the following parameters: Gap penalty value: 16 and Gap length value: 4 alternatively, the program accepts any positive integer. It was found that these parameters are the best suited to calculate the percentage of identity over the reference nucleotide or amino acid sequences, especially considering the different levels of homology among the different sequences analyzed. Any deviations occurring when comparing with the above described nucleic acid molecules may be caused by deletion, substitution, insertion or recombination.

Moreover, homology means that functional and/or structural equivalence exists between the respective nucleic acid molecules or the proteins they encode. The nucleic acid molecules, which are homologous to the above described molecules and represent derivatives of these molecules, are generally variations of these molecules that constitute modifications which exert the same biological function. These variations may be naturally occurring variations, for example sequences derived from other organisms, or mutations, whereby these mutations may have occurred naturally or they may have been introduced by means of a specific mutagenesis. Moreover, the variations may be synthetically produced sequences. The allelic variants may be naturally occurring as well as synthetically produced variants or variants produced by recombinant DNA techniques.

The proteins encoded by the various variants of the above described nucleic acid molecules exhibit certain common characteristics. Biological activity, molecular weight, immunological reactivity, conformation etc. may belong to these characteristics as well as physical properties such as the mobility in gel electrophoresis, chromatographic characteristics, sedimentation coefficients, solubility, spectroscopic properties, stability, pH-optimum, temperature-optimum, etc.

In a preferred embodiment the above described nucleic acid molecule is DNA. In this context, it is understood that the term "nucleic acid molecule" comprises coding and, wherever applicable, non-coding sequences, like, inter alia, 5' and 3' non-coding sequences. Said 5' and/or 3' non-coding regions may comprise (specific) regulatory sequences ensuring initiation of transcription and optionally poly-A signals ensuring termination of transcription and/or stabilization of the transcript. Additional 5' and 3' non-coding regions may comprise promoters and/or transcriptional as well as translational enhancers. Furthermore, the term "nucleic acid molecule" may comprise intron(s) and splice variants, where applicable.

The term DNA as used herein comprises, inter alia, single-stranded or double-stranded DNA, e.g., synthetic DNA, cDNA and genomic DNA. Furthermore, the above described nucleic acid molecule may also be a RNA molecule such as mRNA. In accordance with the present invention, the term "nucleic acid molecule" comprises also any feasible derivative of a nucleic acid to which a nucleic acid probe may hybridize. Said nucleic acid probe itself may be a derivative of a nucleic acid molecule capable of hybridizing to said nucleic acid molecule or said derivative thereof. The term "nucleic acid molecule" further comprises peptide nucleic acids (PNAs) containing DNA analogs with amide backbone linkages (Nielsen, Science 254 (1991), 1497-1500).

In this context it has to be stressed that the above described nucleic acid molecules may also be chemically synthesized, using, inter alia, synthesizers which are known in the art and commercially available, like, e.g. the ABI 394 DNA-RAN-synthesizers.

It is preferred that the above described nucleic acid molecule encodes a polypeptide contributing to membrane stability and/or function of organelles, wherein said polypeptide contributing to membrane stability and/or function in organelles is expressed in mitochondria and/or peroxisomes. It is particularly preferred that said polypeptide participates in the maintenance of said membrane.

Furthermore, it is envisaged that the above described nucleic acid molecule encodes a polypeptide, wherein said polypeptide contributing to membrane stability and/or function in organelles is a transporter molecule and/or a regulator of a transporter molecule. It is, e.g., envisaged that the polypeptide encoded by the above described nucleic acid molecule regulates, directly or indirectly, carrier and/or transport molecules capable of transporting molecules like ions, metabolites or vitamins across membranes and/or that said polypeptide is such a transporter/carrier molecule.

It is particularly preferred that the above described nucleic acid molecule encodes a polypeptide as defined herein above, wherein said polypeptide is a modifying polypeptide. Particularly preferred modifying polypeptides comprise modifiers of mitochondrial proteins, for example the modification of a member of the UCP family.

Said member(s) of the UCP (uncoupling protein) family are known in the art and comprise, UCP1, UCP2, UCP3, UCP4, UCP5, StUCP or AtUCP, see, inter alia, Ricquier (2000), loc. cit. The above mentioned modification of mitochondrial proteins, and in particular of UCPs, may occur by direct interaction with said protein or, also, by supplying/importing/exporting ions, metabolites or vitamins and the like (or by blocking these processes) which are necessary for the function or activity of said mitochondrial protein or which are generated by the activity of said mitochondrial protein. Therefore, said "modification" also relates to transport- and supply-phenomena. Furthermore, said "modification" comprises the control of the function of one or more proteins/polypeptides, preferably of members of the UCP family. Most preferred are "modifications" comprising events which influence the metabolism of the cell, in particular the energy metabolism.

The present invention encompasses also, as pointed out herein above, "variants" of the nucleic acid molecules described herein.

The term "variant" means in this context that the nucleotide and their encoded amino acid sequence, respectively, of these polynucleotides differs from the sequences of the above described nucleic acid molecules and (poly)peptides contributing to membrane stability and/or function of organelles in one or more nucleotide positions and are highly homologous to said nucleic acid molecules. Homology is understood as defined herein above. The deviations from the sequences of the nucleic acid molecules described above can, for example, be the result of nucleotide substitution(s), deletion(s), addition(s), insertion(s) and/or recombination(s). Homology can further imply that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other mammals or mutations. The term "variants" in this context furthermore comprises, inter alia, allelic variations or splice variants as described herein above. Naturally occurring SOUP1 protein or soup1 gene variants are called "allelic variants", and refer to one of several alternate forms of a gene occupying a given locus on a chromosome of an organism. (Genes II, Lewin, B., ed., John Wiley & Sons, New York (1985) and updated versions). These allelic variants can vary at either the polynucleotide and/or (poly)peptide level. Alternatively, non-naturally occurring variants may be produced by mutagenesis techniques or by direct synthesis. Using known methods of protein engineering and recombinant DNA technology, variants may be generated to improve or alter the characteristics of the herein described SOUP1 proteins/soup1 genes. Therefore, the term "allelic variant" also comprises synthetically produced or genetically engineered variants.

The above described nucleic acid molecule may be of natural origin, synthetic or semisynthetic or it may be a derivative.

The nucleic acid molecule(s) used according to the invention may be a recombinantly produced chimeric nucleic acid molecule comprising any of the aforementioned nucleic acid molecules either alone or in combination. Preferably, said nucleic acid molecule is part of a vector. The vector may be, e.g., a plasmid, cosmid, virus, bacteriophage or another vector used e.g. conventionally in genetic engineering, and may comprise further genes such as marker genes which allow for the selection of said vector in a suitable host cell and under suitable conditions. Furthermore, the vector may, in addition to the above described nucleic acid sequences, comprise expression control elements, allowing proper expression of the coding regions in suitable hosts. Such control elements are known to the artisan and may include a promoter, a splice cassette, translation initiation codon, translation and insertion site for introducing an insert into the vector. Preferably, the above described nucleic acid molecule is operatively linked to said expression control sequences allowing expression in eukaryotic or prokaryotic cells.

Control elements ensuring expression in eukaryotic and prokaryotic cells are well known to those skilled in the art. As mentioned herein above, they usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in for example mammalian host cells comprise the CMV-HSV thymidine kinase promoter, SV40, RSV-promoter (Rous sarcoma virus), human elongation factor 1α-promoter, aPM-1 promoter (Schaffer, Biochem. Biophys. Res. Commun. 260 (1999), 416-425), or inducible promoter(s), like, metallothionein or tetracyclin, or enhancers, like CMV enhancer or SV40-enhancer. For the expression in prokaryotic cells, a multitude of promoters including, for example, the tac-lac-promoter or the trp promoter, has been described. Besides elements which are responsible for the initiation of transcription such regulatory elements may also comprise transcription termination signals, such as SV40-poly-A site or the tk-poly-A site, downstream of the polynucleotide. In this context, suitable expression vectors are known in the art such as Okayama-Berg cDNA expression vector pcDV1 (Pharmacia), pRc/CMV, pcDNA1, pcDNA3 (In-vitrogene), pSPORT1 (GIBCO BRL), Casper, Casper-HS43, pUAST, or prokaryotic expression vectors, such as lambda gt11. Beside the above described nucleic acid molecules, the vector may further comprise nucleic acid sequences encoding for secretion signals. Such sequences are well known to the person skilled in the art. Furthermore, depending on the expression system used leader sequences capable of directing the (poly)peptide to a cellular compartment may be added to the coding sequence of the above described nucleic acid molecules and are well known in the art. The leader sequence (s) is (are) assembled in appropriate phase with translation, initiation and termination sequences, and preferably, a leader sequence capable of directing secretion of translated protein, or a protein thereof, into the periplasmic space or extracellular medium. Optionally, the heterologous sequence can encode a fusionprotein including an C- or N-terminal identification peptide imparting desired characteristics, e.g., stabilization or simplified purification of expressed recombinant product. Once the vector has been incorporated into the appropriate host, the host is maintained under conditions suitable for high level expression of the nucleotide sequences, and, as desired, the collection and purification of the (poly)peptide(s) or fragments thereof may follow.

Furthermore, the vector may also be a gene transfer or gene targeting vector. Gene therapy, which is based on introducing therapeutic genes into cells by ex-vivo or in-vivo techniques is one of the most important applications of gene transfer. Suitable vectors, methods or gene-delivering systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813, Isner, Lancet 348 (1996), 370-374; Muhlhauser, Circ. Res. 77 (1995), 1077-1086; Onodua, Blood 91 (1998), 30-36; Verzeletti, Hum. Gene Ther. 9 (1998), 2243-2251; Verma, Nature 389 (1997), 239-242; Anderson, Nature 392 (Supp. 1998), 25-30; Wang, Gene Therapy 4 (1997), 393-400; Wang, Nature Medicine 2 (1996), 714-716; WO 94/29469; WO 97/00957; US 5,580,859; US 5,589,466; US 4,394,448 or Schaper, Current Opinion in Biotechnology 7 (1996), 635-640, and references cited therein. In particular, said vectors and/or gene delivery systems are also described in gene therapy approaches in adipocyte (see, inter alia, US 5,869,037 or Zhou, PNAS USA96 (1999), 2391-2395) or in the hypothalamus (see, inter alia, Geddes, Front Neuroendocrinol. 20 (1999), 296-316 or Geddes, Nat. Med. 3 (1997), 1402-1404). The nucleic acid molecules and vectors described above may be designed for direct introduction or for introduction via liposomes, viral vectors (e.g. adenoviral, retroviral), electroporation, ballistic (e.g. gene gun) or other delivery systems into the cell. Additionally, a baculoviral system can be used as eukaryotic expression system for the above described nucleic acid molecules.

As already mentioned above, the above described nucleic acid molecule and/or the above described vectors/hosts may be particularly useful as pharmaceutical compositions. Said pharmaceutical compositions may be employed in diagnostic and/or therapeutic approaches, e.g. in gene therapy approaches. In this context, it is envisaged that the nucleic acid molecules and/or vectors described above may be employed to modulate, alter and/or modify the cellular expression and/or intracellular concentration of the above described (poly)peptide(s) or of (a) fragment thereof. Said modulation, alteration and/or modification may lead to up- or downregulation of the SOUP1 (poly)peptide and/or the gene product of the herein described SOUP1 gene. Furthermore, said therapeutic approache(s) may lead to an alteration and/or modulation of the availability of active SOUP1 (poly) peptide/protein/gene product. In this context, the term "active" refers to the ability to perform its (normal) cellular function in an organism.

For gene therapy applications, nucleic acids encoding the above described (poly)peptide or fragments thereof may be cloned into a gene delivering system, such as a virus and the virus used for infection and conferring disease ameliorating or curing effects in the infected cells or organism.

As mentioned herein above, the nucleic acid molecule(s) and/or vector(s) may be employed in order to modulate/alter the gene expression or intracellular concentration of SOUP1 protein/(poly)peptide. Said modulation/alteration may also be achieved by antisense-approaches.

Antisense modulation of SOUP1 expression may employ antisense nucleic acids operably linked to gene regulatory sequences. For example, cells are transfected with a vector comprising a soup1 sequence with a promoter sequence oriented such that transcription of the gene yields an antisense transcript capable of binding to endogenous soup1 encoding mRNA. Transcription of the antisense nucleic acid may be constitutive or inducible and the vector may provide for stable extrachromosomal maintenance and integration. Alternatively, single-stranded antisense nucleic acids that bind to genomic DNA or mRNA encoding a (poly)peptide as described above or a fragment thereof may be administered to the target cell, in or temporarily isolated from a host, at a concentration that results in a substantial reduction in expression of said (poly)peptide. Furthermore, it is envisaged that expression of the above described (poly)peptide may be influenced, suppressed by other means than antisense approaches. Therefore, reduced expression of the above described (poly)peptide may also be achieved by RNA-mediated gene interference, which applies double-stranded RNA instead of antisense nucleic acids (see, Sharp, Genes Dev. 13 (1999), 139-141). Gene suppression by double stranded RNA or RNAi-approach is also described in Hunter, Curr. Biol. 10 (2000), R137-R140.

The nucleic acid molecule described above may therefore be used for the construction of appropriate anti-sense oligonucleotides which are able to inhibit the function of the nucleic acid molecules which either encode wildtype or mutant versions of the SOUP1 (poly)peptide. Said anti-sense nucleotide comprises preferably at least 15 nucleotides, more preferably at least 20 nucleotides, even more preferably 30 nucleotides and most preferably at least 40 nucleotides.

In addition, ribozyme approaches are also envisaged in this invention. Ribozymes may specifically cleave the above described nucleic acid molecule.

In the context of the present invention ribozymes comprise, inter alia, hammerhead ribozymes, hammerhead ribozymes with altered core sequences or deoxyribozymes (see, e.g., Santoro, Proc. Natl. Acad. Sci. USA 94 (1997), 4262) and may comprise natural and in vitro selected and/or synthesized ribozymes. Nucleic acid molecules used according to the present invention which are complementary to nucleic acid molecules coding for proteins/(poly)peptides regulating, causing or contributing to obesity and/or encoding a mammalian (poly)peptide involved in the regulation of body weight (see herein below) may be used for the construction of appropriate ribozymes (see, e.g., EP-B1 0 291 533, EP-A1 0 321 201, EP-A2 0 360 257) which specifically cleave nucleic acid molecules as described above. Selection of the appropriate target sites and corresponding ribozymes can be done as described for example in Steinecke, Ribozymes, Methods in Cell Biology 50, Galbraith, eds. Academic Press, Inc. (1995), 449-460.

As already mentioned above, the above described nucleic acid molecules and/or the above described vectors may be contained in a host cell or host, i.e. to a host cell or host which is genetically modified with a nucleic acid molecule described above or with a vector comprising such a nucleic acid molecule. The term "genetically modified" means that the host cell or host comprises in addition to its natural genome a nucleic acid molecule or vector as described above which was introduced into the cell or host or into one of its predecessors/parents. The nucleic acid molecule or vector may be present in the genetically modified host cell or host either as an independent molecule outside the genome, preferably as a molecule which is capable of replication, or it may be stably integrated into the genome of the host cell or host.

The host cell may be any prokaryotic or eukaryotic cell. Suitable prokaryotic cells are those generally used for cloning like E. coli or Bacillus subtilis. Furthermore, eukaryotic cells comprise, for example, fungal or animal cells. Examples for suitable fungal cells are yeast cells, preferably those of the genus Saccharomyces and most preferably those of the species Saccharomyces cerevisiae. Suitable animal cells are, for instance, insect cells, vertebrate cells, preferably mammalian cells, such as e.g. CHO, Hela, NIH3T3, MOLT-4, Jurkat, K562, HepG2, 3T3-442A, 3T3-L1 (and derivatives thereof), HIB-1B (see Villena, Biochem J. 331 (1998), 121-127), HEK 293, PAZ6 (see, Strobel, Diabetologia 42 (1999), 527-533). Further suitable cell lines known in the art are obtainable from cell line depositories, like the American Type Culture Collection (ATCC).

Preferably, the host cell which is transformed with the above described vector is a mammalian cell, particularly an adipose cell, a brain cell, a hepatic cell, an epithelial cell, a blood cell or a cell (line) derived therefrom.

Non-human hosts are preferably non-human mammals, most preferably mice, rats, sheep, calves, dogs, monkeys or apes and may comprise Psammomis obesus. Said mammals may be indispensable for developing a cure, preferably a cure for obesity, adipositas, eating disorders and/or disorders leading to a pathological body mass/body weight. Furthermore, the hosts may be partially useful in producing the above described (poly)peptides (or fragments thereof). It is envisaged that said (poly)peptide (or fragments thereof) be isolated from said host.

The non-human host may also be a non-human transgenic animal as described herein below (see Example 10). Particularly, the present invention envisages non-human transgenic animals comprising a mutated form of the nucleic acid molecules described above or non-human transgenic animals wherein the nucleic acid molecule described above has been deleted and/or inactivated. Said deletion may be a partial deletion. Particularly preferred non-human transgenic animals are Drosophila, Nematodes (like C. elegans), mice, rat, sheep and the like.

According to the present invention, the (poly)peptide encoded by the nucleic acid molecule described above may be produced by culturing the above described host cells under suitable conditions that allow the synthesis of said (poly)peptide and recovering and/or isolating the (poly)peptide produced from the culture. The transformed host cells can be grown in fermentors and cultured according to techniques known in the art to achieve optimal cell growth. The above described (poly)peptide can then be isolated from the growth medium, cellular lysates, cellular membrane fractions or inclusion bodies. Once expressed, the protein can be purified according to standard procedures of the art, including ammonium sulfate precipitation, affinity columns, column chromatography, gel electrophoressis and the like; see, Scopes, "Protein Purification", Springer-Verlag, N.Y. (1982). For example, "Current Protocols in Molecular Biology" (2000, John Wiley and Sons) provide for purification protocols. Further purification schemes are known in the art and provide also for the purification of membrane proteins. For example purification from expression in yeast/yeast expression systems is described in Murdza-Inglis (1991), JBC 266, 11871-11875, purification/exrpression in bacteria has been disclosed in Kaplan (1996), J. Bioenerg. Biomembr. 28, 41-47 or in eukaryotic cells ( Casteilla (1990), PNAS 87, 5124-5128). Substantially pure proteins of at least about 60%, at least about 70%, at least about 80% or at least about 90 to 97% homogeneity are preferred, and 98 to 99% or more homogeneity are most preferred, for pharmaceutical uses. Once purified, partially or to homogeneity as desired, the proteins may then be used therapeutically (including extracorporeally) or in developing and performing assay procedures.

The term "(poly)peptide" as employed herein denotes either a peptide, a full-length protein or (a) fragment(s) thereof. A peptide is preferably a fragment of the above described (poly)peptide. The term "(poly)peptide" comprises (a) peptide(s) or (a) (poly)peptide(s) which encompass amino acid chains of any length, wherein the amino acid residues are linked by covalent peptide bonds. Preferably, said amino acid chains of a "peptide" comprise at least 10 amino acids, more preferably at least 20, more preferably at least 30, more preferably at least 40, even more preferably at least 50 and, most preferably at least 60 amino acids. It is even more preferred that the above described (poly)peptides comprise at least 100, more preferred at least 200, more preferred at least 300, more preferred at least 400, more preferred at least 500, even more preferred at least 600 amino acids.

The term "or (a) fragment(s) thereof" as employed in the present invention and in context with (poly)peptides described above comprises specific peptides, amino acid stretches of the (poly)peptides as disclosed herein. It is preferred that said "fragment(s) thereof" is/are functional fragment(s). The term "functional fragment" denotes a part of the above identified (poly) peptide which fulfils, at least in part, physiological and/or structural activities of the above described (poly)peptide. It is, however, also envisaged that said fragment functions as intervening and/or inhibiting molecule for the (poly) peptide described above. For example, it is envisaged that fragments of the (poly)peptide described above may structurally and/or physiologically interact with the (poly)peptide disclosed herein and thereby inhibit the function of said (poly)peptide.

The (poly)peptides disclosed herein may be recombinant (poly)peptides expressed in host cells like bacteria, yeasts, or other eukaryotic cells, like mammalian or insect cells. Alternatively, they may be isolated from viral preparations. In another embodiment of the present invention, synthetic (poly)peptides may be used. Therefore, such a (poly)peptide may be a (poly) peptide as encoded by the nucleic acid molecule described above which only comprises naturally occurring amino acid residues, but it may also be a (poly)peptide containing modifications. These include covalent derivatives, such as aliphatic esters or amides of a carboxyl group, O-acetyl derivatives of hydroxyl containing residues and N-acyl derivatives of amino group containing residues. Such derivatives can be prepared by linkage to reactable groups which are present in the side chains of amino acid residues and at the N- and C-terminus of the protein. Furthermore, the (poly)peptide can be radiolabeled or labeled with a detectable group, such as a covalently bound rare earth chelate, or conjugated to a fluorescent moiety. The (poly) peptide described herein can be, for example, the product of expression of a nucleotide sequence encoding such a (poly)peptide, a product of chemical modification or can be purified from natural sources, for example, viral preparations. Furthermore, it can be the product of covalent linkage of (poly) peptide domains.

The peptides/(poly)peptides may also be produced by biochemical or synthetic techniques. Those methods are known to those of ordinary skill in the art (see, e.g. Merrifield, J. Am. Chem. Soc. 85 (1963), 2149-2146; Stewart, "Solid Phase Peptide Synthesis", WH Freeman Co, San Francisco (1969); Scopes, "Protein Purification", Springer Verlag, New York, Heidelberg, Berlin (1987); Janson, "Protein Purification, Principles, High Resolution Methods and Applications", VCH Publishers, New York, Weinheim, Cambridge (1989); Wrede, "Concepts in Protein Engineering and Design", Walter de Gruyter, Berlin, New York (1994)).

Additionally, within the scope of the invention are peptides/(poly)peptides wherein the above mentioned amino acid(s) and/or peptide bonds have been replaced by functional analogs, inter alia by peptidomimetics. Peptidomimetics is well known in the art and corresponding art describing this method are mentioned below. Therefore, the present invention also encompasses functional derivatives and/or analogues of said peptides comprising a specific SOUP1-derived peptide. Further methods for the preparation of peptides/(poly)peptides are described in Sambrook et al., loc. cit., or in Oxender and Fox (1987) "Protein Engineering", Alan Liss Inc. New York. Protein preparation of chemical derivates and/or analogues are described in, for example, Beilstein "Handbook of Organic Chemistry", Springer Edition New York, or in "Organic Synthesis", Wiley, New York.

According to the present invention, the fusion protein comprising the polypeptide used according to the present invention can comprise at least one further domain, said domain being linked by covalent or non-covalent bonds. The linkage can be based on genetic fusion according to the methods known in the art (Sambrook et al., loc. cit., Ausubel, "Current Protocols in Molecular Biology", Green Publishing Associates and Wiley Interscience, N.Y. (1989)) or can be performed by, e.g., chemical crosslinking as described in, e.g., WO 94/04686. The additional domain present in the fusion protein comprising the above described (poly)peptide may preferably be linked by a flexible linker, advantageously a (poly)peptide linker, wherein said (poly)peptide linker preferably comprises plural, hydrophilic, peptide-bonded amino acids of a length sufficient to span the distance between the C-terminal end of said further domain and the N-terminal end of the peptide, (poly)peptide or antibody or vice versa. The above described fusion protein may further comprise a cleavable linker or cleavage site, which, for example, is specifically recognized and cleaved by proteinases or chemical agents. Additionally, said at least one further domain may be of a predefined specificity or function. In this context, it is understood that the (poly)peptides described above may be further modified by conventional methods known in the art. This allows for the construction of fusion proteins comprising the above described (poly)peptide and other functional amino acid sequences, e.g., organelle localization signals, transactivating domains, DNA-binding domains, hormone-binding domains, protein tags (e.g. GST, GFP, h-myc peptide, FLAG, HA peptide, Strep), transmembrane domains or fatty acid attachment motifs which may be derived from heterologous proteins.

The fusion protein may also be a mosaic (poly)peptide comprising at least two epitopes of the above described (poly)peptide wherein said mosaic (poly)peptide lacks amino acids normally intervening between the epitopes in the native SOUP1 protein.

Inter alia, such mosaic (poly)peptides are useful in the applications and methods described herein, since they may comprise within a single peptide or (poly)peptide a number of relevant epitopes possibly presented linearly or as multi-antigen peptide system in a case of lysines. Relevant epitopes can be separated by spacer regions.

It is in particular preferred that the fusion protein used according to the present invention comprising said polypeptide or (a) fragment(s) thereof comprise(s) at least one, preferably at least two, more preferably at least three, more preferably at least four, more preferably at least five and most preferably six amino acid sequences as depicted in any one of SEQ ID NOs: 15 to 50, 61 or 62. As disclosed herein above, said sequence relate to specifically deduced transmembrane regions of the SOUP1 proteins described herein. It is particularly preferred that the fusion protein comprises at least one and most preferably at least six amino acid sequences as depicted in any one of SEQ ID NOs: 27 to 50. It is also envisaged that said fusion protein comprises transmembrane regions which are derived from different species, e.g. from human, mouse or zebrafish. Yet, most preferred are fusion proteins which comprise transmembrane regions from one species.

The nucleic acid molecule, the (poly)peptide (as well as the antibody or fragment or derivative thereof, the aptamer or other receptor described herein), the fusion protein, the mosaic (poly)peptide or the anti-sense oligonucleotide used according to the present invention may be detectably labeled. A variety of techniques are available for labeling biomolecules, are well known to the person skilled in the art and are considered to be within the scope of the present invention. Such techniques are, e.g., described in Tijssen, "Practice and theory of enzyme immuno assays", Burden, RH and von Knippenburg (Eds), Volume 15 (1985), "Basic methods in molecular biology"; Davis LG, Dibmer MD; Battey Elsevier (1990), Mayer et al., (Eds) "Immunochemical methods in cell and molecular biology" Academic Press, London (1987), or in the series "Methods in Enzymology", Academic Press, Inc.

There are many different labels and methods of labeling known to those of ordinary skill in the art. Examples of the types of labels which can be used in the present invention include enzymes, radioisotopes (like ³²P or ¹²⁵|), colloidal metals, fluorescent compounds/fluorochromes (like fluorescein, rhodamine, Texas Red, etc.), chemiluminescent compounds, and chemi- or bioluminescent compounds (like dioxetanes, luminol or acridiniums).

Commonly used labels furthermore comprise, inter alia, enzymes (like horse radish peroxidase, β-galactosidase, alkaline phosphatase), biotin or digoxygenin. Labeling procedures, like covalent coupling of enzymes or biotinyl groups, iodinations, phosphorylations, biotinylations, random priming, nick-translations, tailing (using terminal transferases) are well known in the art.

Detection methods comprise, but are not limited to, autoradiography, fluorescence microscopy, direct and indirect enzymatic reactions, etc.

According to the present invention, an antibody or a fragment or derivative thereof or an antiserum or an aptamer or another receptor specifically recognizing an epitope on the nucleic acid, or the (poly)peptide as described above are also used. The general methodology for producing antibodies is well-known and has, for monoclonal antibodies, been described in, for example, Köhler and Milstein, Nature 256 (1975), 494 and reviewed in J.G.R. Hurrel, ed., "Monoclonal Hybridoma Antibodies: Techniques and Applications", CRC Press Inc., Boco Raron, FL (1982). In accordance with the present invention the term "antibody" relates to monoclonal or polyclonal antibodies. Polyclonal antibodies (antiserum) can be obtained according to conventional protocols. Antibody fragments or derivatives comprise F(ab')₂, Fab, Fv or scFv fragments; see, for example, Harlow and Lane, "Antibodies, A Laboratory Manual", CSH Press 1988, Cold Spring Harbor, NY. Preferably the antibody used according to the present invention is a monoclonal antibody. Furthermore, in accordance with the present invention, the derivatives can be produced by peptidomimetics. In the context of the present invention, the term "aptamer" comprises nucleic acids such as RNA, ssDNA (ss = single stranded), modified RNA, modified ssDNA or PNAs which bind a plurality of target sequences having a high specificity and affinity. Aptamers are well known in the art and, inter alia, described in Famulok, Curr. Op. Chem. Biol. 2 (1998), 320-327. The preparation of aptamers is well known in the art and may involve, inter alia, the use of combinatorial RNA libraries to identify binding sites (Gold, Ann. Rev. Biochem. 64 (1995), 763-797). Said other receptors may, for example, be derived from said antibody etc. by peptidomimetics. The specificity of the recognition implies that other known proteins, molecules are not bound. A suitable host for assessing the specificity would imply contacting the above recited compound comprising an epitope of the nucleic acid molecule or the (poly)peptide described above as well as corresponding compounds e.g. from protein or nucleic acid molecules known in the art, for example in an ELISA format and identifying those antibodies etc. that only bind to the compound but do not or to no significant extent cross-react with said corresponding compounds.

Moreover, an anti-sense oligonucleotide of an above described nucleic acid molecule is used according to the present invention. Said anti-sense oligonucleotide may be employed in scientific as well as in diagnostic or in therapeutic purposes.

The invention furthermore provides for the use of a non-human transgenic animal comprising a nucleic acid molecule encoding a polypeptide, contributing by means of protein-protein interactions as well as protein-lipid interactions to membrane stability and/or contributing to membrane function of organelles by having transport functions, regulator functions of other membrane proteins or modifier functions of other (membrane) proteins and/or other functions, wherein said nucleic acid molecule
(a) remains hybridized at 65°C in a solution containing 0.2 x SSC and 0.1% SDS to a nucleic acid molecule encoding the amino acid sequence of SEQ ID NO: 8, SEQ ID NO: 54, SEQ ID NO: 10, SEQ ID NO: 12, SEQ ID NO: 14 and/or SEQ ID NO: 52 and/or the complementary strand thereof;
(b) remains hybridized at 65°C in a solution containing 0.2 x SSC and 0.1% SDS to a nucleic acid molecule as depicted in SEQ ID NO: 6, 7 or 53, SEQ ID NO: 9, SEQ ID NO: 11, SEQ ID NO: 13 and/or SEQ ID NO: 51 and/or the complementary strand thereof;
(c) is degenerate with respect to the nucleic acid molecule of (a) or (b);
(d) encodes a polypeptide which is at least 90% identical to SEQ ID NO: 8;
(e) encodes a polypeptide which is at least 90% identical to SEQ ID NO: 54;
(f) encodes a polypeptide which is at least 90% identical to the amino acid sequence as depicted in any one of SEQ ID NOs: 10, 12, 14 or 52; or
(g) has the sequence as depicted in SEQ ID NOs: 9, 11, 13 or 51;
expressing a polypeptide encoded by said nucleic acid molecule or a fusion protein comprising said polypeptide; or
transfected with a vector comprising said nucleic acid molecule as a research model for obesity, adipositas, eating disorders, wasting and/or other disorders of body weight/body mass.

It is envisaged, for example, that the non-human transgenic animal over- or under-expresses the above described polypeptide. According to a preferred embodiment of the present invention, the above described nucleic acid molecule or a homolog, paralog or ortholog thereof is silenced and/or mutated.

The above mentioned non-human transgenic animal is preferably selected from the group consisting of mouse, rat, sheep, hamster, pig, dog, monkey, rabbit, calf, horse, nematodes, fly and fish.

The non-human transgenic animals disclosed herein, such as transgenic mice, rats, hamsters, dogs, monkeys, rabbits, pigs, C. elegans, Drosophila, fish (like zebrafish or torpedofish), may have one or several copies of the same or different nucleic acid molecules encoding one or several forms of the (poly)peptide described above, regulating, causing or contributing to obesity or involved in the regulation of body weight. As mentioned above, these animals are partially useful as research models for obesity, adipositas, eating disorders, wasting and/or other disorders of body weight/body mass as described herein. Furthermore, said non-human transgenic animals are well suited for, e.g., pharmacological studies of drugs in connection with mutant forms of the above described SOUP1 protein.

As already mentioned above, the nucleic acid molecule, the vector, the host, the polypeptide, the fusion protein, the antibody, fragment or derivative thereof or an aptamer or another receptor or the anti-sense oligonucleotide described above are suitable for controlling the function of a gene and/or a gene product which is influenced and/or modified by a polypeptide as defined herein, e.g. SOUP1. Said influence/modification may occur by direct interaction between proteins/protein fragments and/or by providing metabolic compounds, or ions that are necessary for the function, activity and/or expression of said gene and/or gene product. It is particularly preferred that said gene and/or gene product is a gene and/or gene product expressed in organelles. Said organelle may be, inter alia, a mitochondrium or a peroxisome.

Preferably, said gene and/or gene product is a member of the UCP family. Members of the UCP family are well known and described herein above.

The diagnostic and/or pharmaceutical composition provided by the present invention is for use e.g in human or veterinary medicine. According to the present invention, the composition may further comprise suitable carriers, diluents and/or adjuvants.

The diagnostic and/or pharmaceutical composition is for detecting and/or verifying an disorder in cells, cell masses, organs and/or subjects and/or for the treatment, alleviation and/or prevention of an disorder in cells, cell masses, organs and/or subjects. Said disorder is a metabolic disorder selected from obesity, adipositas, eating disorders (bulimia nervosa, anorexia nervosa), cachexia (wasting) and pancreatic dysfunction (like diabetes, in particular type 2 diabetes).

For example, it has been shown that UCPs are involved in pancreatic disorders, e.g. diabetes. A role for uncoupling proteins in diabetes was demonstrated by induction of UCP3 in Streptozotocin-induced diabetes in rodents (see, inter alia, Hidaka, Proc Soc Exp Biol Med 224: 172-177 (2000), Hidaka, Diabetes 48: 430-435 (1999)).

Furthermore it was shown that UCP2 expression in pancreatic beta-cells influences beta-cell function and insulin secretion (Wang, Diabetes 48: 1020-1025 (1999); Chan, Diabetes 48: 1482-1486 (1999)).

Reactive oxygen species (ROS) can lead to membrane dysfunction, DNA damage and inactivation of proteins. Pathological consequences include cancer, arthritis and neurodegenerative disease. ROS limiting metabolism is a major mechanism to protection from cellular damage. In particular obesity can cause increased oxidative stress (Hayes, Free Radic Res 31: 273-300 (1999); Yang, Arch Biochem Biophys 378: 259-268 (2000)).

In contrast, increased ROS production in macrophages can improve immune response. So are UCP2 knockout mice more resistant against infection with certain pathogens.

Therefore, the compounds disclosed herein, being capable of modifying, inter alia, UCPs may be well suited for the above identified purposes.

In a further embodiment, the present invention relates to the use of the nucleic acid molecule, the vector, the host, the polypeptide, the fusion protein, the antibody, fragment or derivative thereof or an aptamer or another receptor or the anti-sense oligonucleotide for identifying substances capable of interacting with the polypeptide as defined in herein. Said substance is capable of interacting with said polypeptide may be (an) antagonist(s) or (an) agonist(s).

In particular, the present invention provides for an in vitro method of identifying a polypeptide or (a) substance(s) involved in a metabolic disorder selected from obesity, adipositas, eating disorders (bulimia nervosa, anorexia nervosa), cachexia (wasting) and pancreatic dysfunction, comprising the steps of:
(a) testing a collection of polypeptides or substances for interaction with the polypeptide or the fusion protein described above or (a) fragment(s) thereof using a readout system; and
(b) identifying polypeptides or substances which test positive for interaction in step (a).

According to a preferred embodiment of the present invention, the above described method comprises the further step of (c) repeating steps (a) and (b) with the polypeptides identified one or more times wherein the newly identified polypeptide replaces the previously identified polypeptide as a bait for the identification of a further interacting polypeptide.

The polypeptide or substance identified by the method disclosed herein above may be, inter alia, a polypeptide or a substance interacting directly or indirectly (e.g. via linker proteins or via physiological parameters) with the above described polypeptide, i.e. with SOUP1 proteins and/or a fragment thereof. The term "substance" as used herein broadly relates to physiological and non-physiological, e.g. synthetic substances.

The term "fragment" as used in the specification relates to fragments of SOUP1 proteins which comprise at least 5, preferably at least 10, more preferably at least 15 and even more preferably at least 25 amino acids and/or further comprise at least one, preferably at least 2, more preferably at least 3, more preferably at least 4, more preferably at least 5 and most preferably at least 6 transmembrane regions. Yet, it is also envisaged that a fragment as used herein represents the N- or C-terminus of a SOUP1 protein.

Said testing for interaction of step (a) as described herein above may be carried out by methods known to the skilled artisan and were described herein. In particular these assays comprise biochemical, immunological and/or molecular biological assays.

Said interaction assays employing read-out systems are well known in the art and comprise, inter alia, two hybrid screenings (as, described, inter alia, in EP-0 963 376, WO 98/25947, WO 00/02911), GST-pull-down columns, co-precipitation assays from cell extracts as described, inter alia, in Kasus-Jacobi, Oncogene 19 (2000), 2052-2059, "interaction-trap" systems (as described, inter alia, in US 6,004,746), expression cloning (e.g. lamda gtll), phage display (as described, inter alia, in US 5,541,109), in vitro binding assays and the like. Further interaction assay methods and corresponding read out systems are, inter alia, described in US 5,525,490, WO 99/51741, WO 00/17221, WO 00/14271 or WO 00/05410.

Similarly, interacting molecules/(poly)peptides may be deduced by cell-based techniques well known in the art. These assays comprise, inter alia, the expression of reporter gene constructs or "knock-in" assays, as described, for, e.g., the identification of drugs/small compounds influencing the gene expression. Said "knock-in" assays may comprise "knock-in" in tissue culture cells, as well as in (transgenic) animals. Examples for successful "knock-ins" are known in the art (see, inter alia, Tanaka, J. Neurobiol. 41 (1999), 524-539 or Monroe, Immunity 11 (1999), 201-212). Furthermore, biochemical assays may be employed which comprise, but are not limited to, binding of the above described (poly)peptides (or (a) fragment (s) thereof) to other molecules/(poly)peptides, peptides or binding of the above described (poly)peptides (or (a) fragment(s) thereof) to itself (themselves) (dimerizations, oligomerizations, multimerizations) and assaying said interactions by, inter alia, scintillation proximity assay (SPA) or homogenous time-resolved fluorescence assay (HTRFA).

Further method(s) which may be employed comprises FRET (fluorescence resonance energy transfer; as described, inter alia, in Ng, Science 283 (1999), 2085-2089), or fluorescence polarization assays. These methods are well known in the art and inter alia described in Fernandez, Curr. Opin. Chem. Biol. 2 (1998), 547-603.

Said "testing of interaction" may also comprise the measurement of a complex formation. The measurement of a complex formation is well known in the art and comprises, inter alia, heterogeneous and homogeneous assays. Homogeneous assays comprise assays wherein the binding partners remain in solution and comprise assays, like agglutination assays. Heterogeneous assays comprise assays like, inter alia, immuno assays, for example, ELISAs, RiAs, IRMAs, FlAs, CLIAs or ECLs.

Further methods and assays for identifying interaction and/or binding partners of the above described (poly)peptides or for the identification of agents/compounds which are capable of interfering with the binding of the above described (poly)peptides with this (specific) intracellular binding partners/targets are disclosed herein below. Said additional and/or further method(s) and assay(s) may also be employed in the above described method for identifying a (poly)peptide involved in the regulation of body weight and/or capable of interacting with the SOUP1 (poly)peptide of the invention.

Any measuring or detection step of the method(s) of the present invention may be assisted by computer technology. For example, in accordance with the present invention, said detection and/or measuring step can be automated by various means, including image analysis, spectroscopy or flow cytometry.

In yet another embodiment, the present invention relates to the method(s) described herein above, which further comprises the step of identifying the nucleic acid molecule(s) encoding the one or more interacting (poly) peptides.

The identification of such nucleic acid molecule(s) is well known in the art and comprises, inter alia, the use of specific and/or degenerate primers. Furthermore, recombinant technologies as described in Sambrook, loc. cit. or in Glick (1994), "Molecular Biotechnology", ASM Press, Washington may be employed.

According to the present invention, the nucleic acid molecules as described herein or the polypeptides as described herein may also be used for the detection and/or isolation of genes and/or gene products involved in functional cascades of cell metabolism, in particular of energy metabolism.

Additionally, the present invention relates to an in vitro method of identifying a polypeptide involved in the regulation of body weight in a mammal, comprising the steps of
(a) contacting a collection of (poly)peptides with the polypeptide or the fusion protein described above under conditions that allow binding of said (poly)peptides;
(b) removing (poly)peptides from said collection of (poly)peptides that did not bind to said polypeptide or fusion protein in step (a); and
(c) identifying (poly)peptides that bind to said polypeptide or fusion protein.

The method as described herein above may be carried out by the person skilled in the art without further ado. Said "contacting" of step (a) may, inter alia, be carried out in solution employing (magnetic) beads coupled with the (poly)peptide or fusion protein. Non-bound (poly)peptides may be easily removed by methods known in the art, comprising, for example, magnetic separation, gravity, affinity column systems and corresponding washes and the like.

Methods for identifying bound (poly)peptides are well known in the art and comprise, inter alia, SDS PAGE analysis and Western blotting. Furthermore, techniques like 2D-gel electrophoresis, in-gel digests, microsequencing, N-terminal sequencing, MALDI-MS, analysis of peptides in mass spectroscopy, peptide mass fingerprinting, PSD-MALDI-MS and/or (micro-) HPLC.

Separated polypeptdies to be identified may be further analyzed by, inter alia, Edman-degradation, MALDI-MS methods, ladder sequencing (Thiede, FEBS 357 (1995), 65).

By use of the above described and mentioned methods (and others known in the art) amino acid sequences of the (poly)peptides to be identified can be deduced and sequenced. From these sequenced amino acid fragments, degenerative oligonucleotides may be deduced and synthesized that may be used to screen, for example, genomic or cDNA libraries to identify and clone the corresponding gene/cDNA.

Furthermore, phage display approaches may be employed in the method(s) of this invention. Phage display allows the identification of proteins that interact with a molecule of interest. Libraries of phage, each displaying a different peptide epitope are tested for binding to the molecule of interest. Bound phages can be purified and the insert encoding the peptide epitope may be sequenced. Phage display kit(s) are known in the art and commercially available, e.g., Display Systems Biotech Cat.No. 300-110.

The present invention relates, in yet another embodiment to the method(s) described herein, wherein said (poly)peptide or fusion protein is fixed to a solid support.

Solid supports are well known in the art and comprise, inter alia, commercially available column materials, polystyrene beads, latex beads, magnetic beads, colloid metal particles, glass and/or silicon chips and surfaces, nitrocellulase strips, membranes, sheets, duracytes, wells and walls of reaction trays, plastic tubes etc. Suitable methods for fixing/immobilizing said (poly)peptide(s) or fusion protein(s) are well known and include, but are not limited to ionic, hydrophobic, covalent interactions and the like.

In a particular preferred embodiment, said solid support is a gel filtration or an affinity chromatography material.

in a yet more preferred embodiment of the method of the invention as described herein above, said binding (poly)peptides are released prior to said identification in step (c).

Said release may be effected by elution. Such elution methods are well known in the art and comprise, inter alia, elution with solutions of different ionic strength or different pH, or with intercalating or competing agents/molecules/peptides.

Furthermore, in a yet more preferred embodiment, the present invention relates to the above described method of the invention, wherein said method further comprises the step of identifying the nucleic acid molecule(s) encoding the one or more binding (poly)peptides.

As pointed out herein above, said nucleic acid molecule(s) may be deduced, inter alia, by employing degenerate primers/oligonucleotides in order to detect the corresponding gene(s) and/or cDNA(s) or by expression cloning.

Further, the present invention relates to a method of identifying a compound for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction, wherein said compound influences the expression of the above described nucleic acid molecule, comprising the steps of
(a) contacting a host carrying an expression vector comprising the nucleic acid molecule described above or the nucleic acid molecule identified by the method of the invention operatively linked to a readout system with a compound or a collection of compounds;
(b) assaying whether said contacting results in a change of signal intensity provided by said readout system; and, optionally,
(c) identifying a compound within said collection of compounds that induces a change of signal in step (b);
wherein said change in signal intensity correlates with a change of expression of said nucleic acid molecule.

Furthermore, the present invention provides for a method of identifying a compound for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction, wherein said compound influences the activity of a (poly)peptide as defined herein above, comprising the steps of
(a) contacting a host cell or non-human host carrying an expression vector comprising the nucleic acid molecule described above operatively linked to a readout system and/or carrying a (poly)peptide as described above linked to a readout system with a compound or a collection of compounds;
(b) assaying whether said contacting results in a change of signal intensity provided by said readout system; and, optionally
(c) identifying a compound within said collection of compounds that induces a change of signal in step (b);
wherein said change in signal correlates with a change in activity of said (poly)peptide.

The term "activity" as used herein above in context of the method of the invention also comprises the "function" of (a) (poly)peptide(s) as disclosed herein. Said function may comprise, as mentioned herein above, enzymatic activities or other functions, like, inter alia, involvement in signalling pathways. Such activities and modulators of such activities may be determined and/or identified by convenient in vitro or in vivo assays as described herein or by variations thereof. The underlying technology is widely and commonly known to the person skilled in the art.

Readout systems operatively linked to the above described nucleic acid molecules or linked to the above described (poly)peptides are disclosed herein and comprise, but are not limited to, assays based on radioactive lables, luminescence, fluorescence, etc. Inter alia, said readout system may comprise fluorescence resonance energy transfer (FRET). The above described methods are particularly useful in (automated) high-throughput screenings. In context of this invention, the above mentioned "readout system opertatively linked to the nucleic acid molecules described herein" also comprises readout systems which are located on different molecules, e.g. nucleic acid molecules, like, inter alia, other plasmids, vectors etc.

Said host cell of step (a) of the methods described herein above may be a eukaryotic host cell. Said host cell may be a yeast cell or a plant cell. It is particularly preferred that said eukaroytic host cell is a mammalian host cell. However, said host cell may also be a prokaryotic cell, e.g. a bacterium.

Particularly preferred are prokaryotic (host) cells as described herein above.

The term "compound" in the method(s) of the invention includes a single substance or a plurality of substances which may or may not be identical. Said compound(s) may be comprised in, for example, samples, e.g., cell extracts from, e.g., plants, animals or microorganisms. Furthermore, said compound(s) may be known in the art but hitherto not known to be capable of influencing the activity of (a) (poly)peptide(s) disclosed herein or not known to be capable of influencing the expression of the nucleic acid molecule disclosed herein, respectively. The plurality of compounds may be, e.g., added to a sample in vitro, to the culture medium or injected into the cell.

If a sample (collection of compounds) containing (a) compound(s) is identified in the method(s) of the invention, then it is either possible to isolate the compound from the original sample identified as containing the compound in question or one can further subdivide the original sample, for example, if it consists of a plurality of different compounds, so as to reduce the number of different substances per sample and repeat the method with the subdivisions of the original sample. It can then be determined whether said sample or compound displays the desired properties by methods known in the art such as described herein. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art (see, e.g., EP-A-0 403 506). Furthermore, the person skilled in the art will readily recognize which further compounds and/or cells may be used in order to perform the methods of the invention, for example, host cells as described herein above or enzymes, if necessary, that, e.g., convert a precursor compound into the active compound which in turn influences the expression of the nucleic acid molecule described above and/or influences the activity of (a) (poly)peptide described above. Such adaptation of the method of the invention is well within the skill of the person skilled in the art and can be performed without undue experimentation.

Compounds which can be used in accordance with the method of the present invention include, inter alia, peptides, proteins, nucleic acids including cDNA expression libraries, antibodies, small organic compounds, ligands, PNAs and the like. Said compounds can also be functional derivatives or analogues of known activators or inhibitors. Methods for the preparation of chemical derivatives and analogues are well known to those skilled in the art and are described in, for example, Beilstein, loc. cit. Furthermore, said derivatives and analogues can be tested for their effects according to methods known in the art and/or as described herein. Furthermore, peptidomimetics and/or computer aided design of appropriate activators or inhibitors of the expression of the nucleic acid molecules of the invention or of the activity of (a) (poly)peptide disclosed herein can be used, for example, according to the methods described herein. Appropriate computer systems for the computer aided design of, e.g., proteins and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above described computer analysis can be used in combination with the method of the invention for, e.g., optimizing known compounds, substances or molecules. Appropriate compounds can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors or activators of SOUP1 protein or the soup1 nucleic acid molecule can be used for the design of peptidomimetic inhibitors or activators of the (poly)peptide disclosed herein to be tested in the method of the invention (Rose, Biochemistry 35 (1996), 12933-12944; Rutenber, Bioorg. Med. Chem. 4 (1996), 1545-1558).

In a particularly preferred embodiment, the above described methods of the invention are method(s) wherein said change in signal intensity is an increase in signal intensity or a decrease in signal intensity. The above described method(s) of the invention for identifying compounds influencing the expression of the nucleic acid molecule disclosed herein and/or the activity of the (poly)peptide disclosed herein may also be employed for screening of said compound(s).

Moreover, the invention provides for a non-therapeutic method of assessing the impact of the expression of one or more polypeptides or of one or more fusion proteins as described above in a non-human animal, comprising the steps of
(a) overexpressing or underexpressing a nucleic acid molecule coding for a polypeptide or a fusion protein as described above or a nucleic acid molecule identified by the method of the invention in said animal; and
(b) determining whether the weight of said animal has increased, decreased, whether metabolic changes are induced and/or whether the eating behaviour is modified.

Non-human transgenic animals as described herein above may be particularly useful for the above described methods of assessing the impact of the expression of one or more (poly)peptides. The above mentioned "underexpression" of the nucleic acid molecule comprises, inter alia, full deletions of both alleles, or the deletion of any one allele. Furthermore, said term comprises the generation of a mutation which leads to the expression of a less functional protein/(poly)peptide in the test animal.

Additionally, the present invention relates to a method of screening for an agent which modulates the interaction of a polypeptide as defined herein above capable of detecting, verifying, treating, alleviating and/or preventing a metabolic disorder selected from obesity, adipositas, eating disorders (bulimia nervosa, anorexia nervosa), cachexia (wasting) and pancreatic dysfunction with a binding target/agent, comprising the steps of
(a) incubating a mixture comprising
   (aa) a polypeptide or a fusion protein as described herein or a fragment thereof;
   (ab) a binding target/agent of said (poly)peptide or fusion protein or fragment thereof; and
   (ac) a candidate agent
   under conditions whereby said (poly)peptide, fusion protein or fragment thereof specifically binds to said binding target/agent at a reference affinity;
(b) detecting the binding affinity of said (poly)peptide, fusion protein or fragment thereof to said binding target/agent to determine an (candidate) agent-biased affinity; and
(c) determining a difference between (candidate) agent-biased affinity and the reference affinity.

As pointed out herein above, a specific binding target/agent of the above described (poly)peptide(s) may comprise molecules involved in signalling pathways and/or specific receptors contacting of the (poly)peptide. However, it is also envisaged that said binding target/agent of the above described (poly)peptide is said (poly)peptide itself, leading, inter alia, to dimerizations or multimerizations. Further (natural and artificial) binding targets/agents may be identified by methods known in the art and disclosed herein.

The "reference affinity" of the interaction of the above described (poly) peptides and its binding targets/agents may be established and/or deduced by methods known in the art. Said methods comprise, but are not limited to, in vitro and in vivo methods and may involve binding assays as described herein. In particular, said binding assays encompass any assay where the molecular interaction of the above described (poly)peptides with binding targets/agents be evaluated. Said binding target/agent may comprise natural (e.g. intracellular) binding targets/agents, such as, e.g., SOUP1-substrate, SOUP1 (poly)peptide itself, SOUP1 (poly)peptide regulators and/or molecules of signalling cascades. Within the scope of this invention are, however also non-natural binding partners of the above described (poly) peptide, which may comprise, e.g., antibodies or derivatives and/or fragments thereof, aptamers, as well as non-natural receptor molecules. Said binding targets/agents also comprise antagonists as well as agonists of the (poly)peptides disclosed herein.

Specific affinities, activities and/or function of the above described (poly) peptide(s) may be determined by convenient in vitro, cell-based or in vivo assays, e.g. in vitro binding assays, cell culture assays, in animals (e. g. gene therapy, transgenics), etc. Binding assays encompass any assay where the molecular interaction of a (poly)peptide as described above with a binding target is evaluated. The binding target may be a natural intracellular binding target such as oligomerization (dimerization, multimerization) of said (poly)peptide itself, a substrate or a regulating protein of said (poly)peptide or another regulator that directly modulates the activity or the (cellular) localization of said (poly)peptides. Further binding targets/agents comprise non-natural binding targets like (a) specific immune protein(s) such as an antibody, or an SOUP1 (poly)peptide specific agent such as those identified in screening assays as described below.

Specific screening assays are, inter alia, disclosed in US 5,854,003 or in US 5,639,858. Specific binding agents of the (poly)peptides disclosed herein may include SOUP1-specific receptors, such as those of the family of heptahelical receptors. Other natural SOUP1 binding targets are readily identified by screening cells, membranes and cellular extracts and fractions with the disclosed materials and methods and by other methods known in the art. For example, natural intracellular binding targets of the (poly)peptide described herein above may be identified with assays such as one-, two-, and three-hybrid screens. In addition, biochemical purification procedures, co-precipitation assays from cell extracts, interaction-trap" systems, expression cloning (e. g. in bacteria using lambda gt11 or in eukaryotic cell systems using plasmid expression vectors), phage display, and the like, may be utilised for identification of natural SOUP1 binding agents. Non-natural intracellular binding agents may be obtained in screens of chemical libraries such as described below, etc.

The invention provides efficient methods of identifying pharmacological agents, compounds or lead compounds for agents active at the level of cellular function that can be modulated by SOUP1. Generally, these screening methods involve assaying for compounds, which modulate interaction of the (poly)peptides disclosed herein with a natural SOUP1 binding target. A wide variety of assays for binding agents are provided including labeled in vitro protein-protein binding assays, immunoassays, cell based assays, etc. The methods are amenable to automated, cost-effective high-throughput screening of chemical libraries for lead compounds and have immediate application in a broad range of domestic and international pharmaceutical and biotechnology drug development programs. Identified reagents find use in the pharmaceutical industries for animal and human trials; for example, the reagents may be derivatised and rescreened in vitro and in vivo assays to optimise activity and minimise toxicity for pharmaceutical development.

In vitro binding assays employ a mixture of components including a (poly) peptide described above, which may be part of a fusion product with another peptide or (poly)peptide(s), e. g. a tag for detection or anchoring, etc. The (poly)peptides described above or fragment(s) thereof used in the methods are usually added in an isolated, partially pure or pure form and are typically recombinantly produced. The assay mixture also comprises a candidate pharmacological agent at different concentrations. Candidate agents encompass numerous chemical classes, though typically they are organic compounds; preferably small organic compounds. Small organic compounds have a molecular weight of more than 50 Da yet less than about 2,500 Da, preferably less than about 1,000 Da, more preferably, less than about 500 Da. Candidate agents comprise functional chemical groups necessary for structural interactions with proteins and/or DNA, and typically include at least an amine, carbonyl, hydroxyl or carboxyl group, preferably at least two of the functional chemical groups, more preferably at least three. The candidate agents often comprise cyclical carbon or heterocyclic structures and/or aromatic or polyaromatic structures substituted with one ore more of the aforementioned functional groups. Candidate agents are also found among biomolecules including peptides, saccharides, fatty acids, steroids, purine, pyrimidies, derivatives, structural analogues or combinations thereof, and the like. Where the agent is or is encoded by a transfected nucleic acid, said nucleic acid is typically DNA or RNA.

Candidate agents are obtained from a wide variety of sources including libraries of synthetic or natural compounds. For example, numerous means are available for random and directed synthesis of a wide variety of organic compounds and biomolecules. Alternatively, libraries of natural compounds in the form of bacterial, fungal, plant, and animal extracts are available or readily produced. Additionally, natural and synthetically produced libraries and compounds are readily modified through conventional chemical, physical, and biochemical means. In addition, known pharmacological agents may be subject to directed or random chemical modifications, such as acylation, alkylation, esterification, amidification, etc., to produce structural analogues.

A variety of other reagents may also be included in the mixture. These include reagents required as biochemical energy sources, e. g. ATP or ATP analogues, nucleic acids, e. g. in nucleic acids binding assays, salts, buffers, neutral proteins, e. g. albumin, detergents, etc., which may be used to facilitate optimal protein-protein and/or protein-nucleic acid binding and/or reduce non-specific or background interactions, etc. Also, reagents that otherwise improve the efficiency of the assay, such as protease inhibitors, nuclease inhibitors, antimicrobial agents, etc. may be used.

The resultant mixture is incubated under conditions whereby, but for the presence of the candidate pharmacological agent, the SOUP1 polypeptide specifically binds the cellular binding target, portion or analogue with a reference binding affinity. The mixture components can be added in any order that provides for the requisite binding and incubations may be performed at any temperature, which facilitates optimal binding. Incubation periods are likewise selected for optimal binding but also minimised to facilitate rapid, high-throughput screening. Generally a plurality of assay mixtures are run in parallel with different agent concentrations to obtain a differential response to the various concentrations. Typically, one of these concentrations serves as a negative control, i. e. at zero concentration or below the limits of assay detection.

After incubation, the agent-biased binding and/or affinity between the (poly) peptide disclosed herein and one or more binding targets is detected by any convenient way. For cell-free binding type assays, a separation step is often used to separate bound from unbound components. The separation step may be accomplished in a variety of ways. Conveniently, at least one of the components is immobilised on a solid substrate, which may be any solid from which the unbound components may be conveniently separated. The solid substrate may be made of a wide variety of materials and in a wide variety of shapes, e.g. microtiter plate, microbead, dipstick, resin particle, etc. The substrate is chosen to maximise the signal to noise ratios, primarily to minimise background binding, for ease of washing and cost.

Separation may be effected for example, by removing a bead or a dipstick from a reservoir, emptying or diluting a reservoir such as a microtiter plate well, rinsing a bead (e. g. beads with iron cores may be readily isolated and washed using magnets), particle, chromatographic column or filter with a wash solution or solvent. Typically, the separation step will include an extended rinse or wash or a plurality of rinses and washes. For example, where the solid substrate is a microtiter plate, the wells may be washed several times with a washing solution, which typically includes those components of the incubation mixture that do not participate in specific binding such as salts, buffer, detergent, non-specific protein, etc.

Alternatively, cell-free binding type assays may be performed in homogeneous formats that do not require a separation step, e.g. scintillation proximity assay (SPA), homogenous time-resolved fluorescence assay (HTRFA). Further methods which may be employed comprise fluorescence polarisation (FP) and fluorescence resonance energy transfer (FRET).

Detection may be effected in any convenient way. For cell based assays such as one, two, and three hybrid screens, the transcript resulting from SOUP1-target binding usually encodes a directly or indirectly detectable product (e.g. galactosidase activity, luciferase activity, etc.). For cell-free binding assays, one of the components usually comprises or is coupled to a label. A wide variety of labels may be employed - essentially any label that provides for detection of bound protein. The label may provide for direct detection as radioactivity, luminescence, polarisation of light, optical or electron density, etc. or indirect detection such as an epitope tag, an enzyme, etc. The label may be appended to the protein e. g. a phosphate group comprising a radioactive isotope of phosphorous, or incorporated into the protein structure, e. g. a methionine residue comprising a radioactive isotope of sulfur.

A variety of methods may be used to detect a specific label depending on the nature of the label and other assay components. For example, the label may be detected bound to a solid substrate or a portion of the bound complex containing the label may be separated from the solid substrate, and thereafter the label detected. Labels may be directly detected through optical or electron density, radiative emission, nonradiative energy transfer, emission of polarised light, etc., or indirectly detected with antibody conjugates, etc. For example, in the case of radioactive labels, emissions may be detected directly, e.g. with particle counters or indirectly, e.g. with scintillation cocktails and counters.

A difference in the binding affinity of the above described (poly)peptide to the target in the absence of the agent as compared with the binding affinity in the presence of the agent indicates that the agent modulates the binding of the SOUP1 polypeptide to the SOUP1 binding target. The difference, as used herein, is statistically significant and preferably represents at least a 50%, more preferably at least a 90% difference.

Analogously, in cell-based assays, a difference in SOUP1-dependent activity in the presence and absence of an agent indicates the agent modulates SOUP1 mediated cellular function or SOUP1 expression. Such cell-based approaches may involve transient or stable expression assays. In this method, cells are transfected with one or more constructs encoding in sum, a polypeptide comprising a portion of the above described (poly)peptide and a reporter under the transcriptional control of a soup1 responsive promotor. The cell may advantageously also be cotransfected with a construct encoding an SOUP1 activator, e. g. a receptor capable of stimulating SOUP1 activity, etc. Alternatively, the adipose promotor itself may be linked to a suitable reporter gene, e. g. luciferase, and used in cell-based assays to screen for compounds capable of modulating, via up- or down-regulation, adipose expression.

The methods described herein are particularly suited for automated high-throughput drug screening using robotic liquid dispensing workstations. Similar robotic automation is available for high-throughput cell plating and detection of various assay read-outs.

Candidate agents shown to modulate the expression of the nucleic acid molecules disclosed herein or association of (poly)peptides disclosed herein with a binding partner provide valuable reagents to the pharmaceutical industries for animal and human trials. Target therapeutic indications are limited only in that the target soup1 cellular function (e. g. gene expression or association with a binding partner) be subject to modulation. In particular, candidate agents obtained from drug screening assays and the subject compositions, e.g. as soup1-derived nucleic acids or therapeutic polypeptides, provide therapeutic applications in diseases associated with body-weight regulation and energy homeostatis, including treatment of obesity, disorders associated with wasting, such as cancer, infectious diseases and HIV infection, or bulimia. As will be discussed herein below, for therapeutic use, the compositions and agents may be administered by any convenient way, preferably parenterally, conveniently in a physiologically acceptable carrier, e.g. phosphate buffered saline, saline, deionized water, or the like. Other additives may be included, such as stabilisers, bactericides, etc. Typically, the compositions are added to a retained physiological fluid such a blood or synovial fluid. Generally, the amount administered will be empirically determined, depending, for example, upon the therapeutic objectives; the route of administration, and the condition of the patient. Typically, the clinician will administer a molecule disclosed herein until a dosage is reached that provides the required biological effect. The progress of this therapy is easily monitored by conventional assays.

A method of refining the compound or agent identified by the method of the invention comprises
(a) modeling said compound by peptidomimetics; and
(b) chemically synthesizing the modeled compound.

Peptidomimetics are well known in the art and disclosed, inter alia, in Beeley, Trends Biotech 12 (1994), 213-216, Wiley, Med. Res. Rev. 13 (1993), 327-384, Hruby, Biopolymers 43 (1997), 219-266, or references cited therein or references cited herein above.

Methods of the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Methods for the chemical synthesis and/or the preparation of chemical derivatives and analogues are well known to those skilled in the art and are described in, for example, Beilstein, loc. cit. and "Organic Synthesis", Wiley, New York, U.S.A., see supra.

It is envisaged in the present invention that the above mentioned peptidomimetics methods and/or methods for chemical synthesis, modification or for refining may also directly be employed on the compounds disclosed herein, e.g. on the (poly)peptides or on the fusionproteins described above.

According to the present invention, the manufacturing of the diagnostic and/or pharmaceutical composition disclosed herein comprises formulating the nucleic acid molecule, polypeptide, fusion protein, antibody, fragment, derivative, aptamer, antisense oligonucleotide, ribozyme, hybridization probe or amplification primer described above with a pharmaceutically acceptable carrier and/or diluent.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg per dose; however, doses below or above this exemplary range are envisioned, especially considering the aforementioned factors. If the regimen is a continuous infusion, it should also be in the range of 1 µg to 10 mg units per kilogram of body weight per minute, respectively. Progress can be monitored by periodic assessment. The compositions provided by the invention may be administered locally or systemically. The compositions may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition provided by the present invention may comprise further agents depending on the intended use of the pharmaceutical composition.

Diagnostic and/or pharmaceutical compositions comprising the compound(s) disclosed herein or the compound(s) or agent(s) identified by the method(s) of the invention may e.g. be produced by a method comprising the steps of
(a) modifying a compound disclosed herein, or a compound or agent identified by the method of the invention as a lead compound to achieve
   (i) modified site of action, spectrum of activity, organ specificity, and/or
   (ii) improved potency, and/or
   (iii) decreased toxicity (improved therapeutic index), and/or
   (iv) decreased side effects, and/or
   (v) modified onset of therapeutic action, duration of effect, and/or modified pharmakinetic parameters (resorption, distribution, metabolism and excretion), and/or
   (vii) modified physico-chemical parameters (solubility, hygroscopicity, color, taste, odor, stability, state), and/or
   (viii) improved general specificity, organ/tissue specificity, and/or
   (ix) optimized application form and route
   by
   (i) esterification of carboxyl groups, or
   (ii) esterification of hydroxyl groups with carbon acids, or
   (iii) esterification of hydroxyl groups to, e.g. phosphates, pyrophosphates or sulfates or hemi succinates, or
   (iv) formation of pharmaceutically acceptable salts, or
   (v) formation of pharmaceutically acceptable complexes, or
   (vi) synthesis of pharmacologically active polymers, or
   (vii) introduction of hydrophilic moieties, or
   (viii) introduction/exchange of substituents on aromates or side chains, change of substituent pattern, or
   (ix) modification by introduction of isosteric or bioisosteric moieties, or
   (x) synthesis of homologous compounds, or
   (xi) introduction of branched side chains, or
   (xii) conversion of alkyl substituents to cyclic analogues, or
   (xiii) derivatisation of hydroxyl group to ketales, acetales, or
   (xiv) N-acetylation to amides, phenylcarbamates, or
   (xv) synthesis of Mannich bases, imines, or
   (xvi) transformation of ketones or aldehydes to Schiffs bases, oximes, acetales, ketales, enolesters, oxazolidines, thiozolidines or combinations thereof; and
(b) formulating the product of said modification with a pharmaceutically acceptable carrier.

Pharmaceutical acceptable carriers are well known in the art, as described herein above. It is envisaged that also the compounds described herein, i.e. the (poly)peptides or fusionproteins described above, the nucleic acid molecules described above be employed in the above described method for producing a composition. Preferably, said composition(s) is/are a pharmaceutical composition(s) as described herein.

Moreover, it is conceivable to produce a composition comprising
(a) an inhibitor of the above described (poly)peptide or identified by the method or refined by the method of the invention;
(b) an inhibitor of the expression of the gene identified by the method described herein or the above described nucleic acid molecule; and/or
(c) a compound identified by the method of the invention.

Said inhibitor of the above described (poly)peptide may be a compound which functions as inhibitor of the wildtype (poly)peptide, the SOUP1 protein. Said inhibitor may lead to induction of weight loss, may influence regulatory cells (pancreatic beta-cells) and thereby improve beta-cell function or preventing insulin resistance, it may change ROS (reactive oxygen species) production leading to a decreased ROS concentration (causing reduced molecular damage in aging, cancerogenesis and increased ischemic tolerance). However opposite effects could occur due to tissue specific reactions and metabolic situation. Said inhibitor may also be an inhibitor specifically interacting with (a) mutated form(s) of the (poly)peptide disclosed herein and thereby lead to a decrease in body weight/body mass or to an maintainance of the current body weight/body mass.

It is to be understood that the term "inhibitor" of the (poly)peptide identified by the methods of the invention also relates to (an) inhibitor(s) which influence the activity and/or function of interacting (poly)peptides as identified by the method of the present invention. Said interaction may be direct or indirect. Said "inhibitor" may also interfere with and/or modify the interaction of the (poly)peptide described herein above with its binding targets/agents as defined herein. The above described applies mutatis mutandis for the term "inhibitor of the expression of the above described nucleic acid molecule or of the gene identified by the method(s) of the invention". Said inhibitor may interfere with transcriptional and/or translational processes.

Similarly, is is conceivable to produce a composition comprising
(a) a stimulator of the above described (poly)peptide or of the (poly)peptide identified or refined by the method(s) described herein above;
(b) a stimulator of the expression of the above described nucleic acid molecule or of the gene identified by the method(s) of the invention;
(c) a compound identified by the method(s) of the invention; and/or
(d) the above described vector.

The term "stimulation of the (poly)peptide" relates to a compound which functions as a stimulator (activator) of the above described (poly)peptides. Said stimulator/activator may lead to an induction of weight gain and may be useful for the treatment of wasting. It may also change the ROS production which may lead to increased efficacy in (a) immune response(s). Yet, opposite effects are also envisaged, due to tissue specific reactions and metabolic situations. The here described "stimulators" may, inter alia, lead to an increased interaction of the (poly)peptide disclosed herein with its binding target. The term also relates to a stimulator/activator of the mutated form(s) of the above described (poly)peptides. Said stimulator(s) of the mutated form(s) may lead to an increase in body weight/body mass or to an maintenance of the current body weight/body mass.

"Inhibitors" as well as "stimulators of the (poly)peptide disclosed herein" may be deduced and/or evaluated by methods known in the art and disclosed herein.

The term "stimulator of a (poly)peptide identified or refined by the method(s) of the present invention" relates also to a stimulator which influences the activity/function of (interacting) (poly)peptides as identified by the method of the present invention they may interact with said (poly)peptides in either direct or indirect fashion. As already mentioned for the term "inhibitor", as defined herein above, the above said applies, mutatis mutantis, for the term "stimulator of the expression of the nucleic acid molecule disclosed herein or of the gene identified by the method(s) of the invention".

The above mentioned "inhibitors" and "stimulators" not only relate to (poly) peptides, but may also comprise small molecules which bind to, interfere with and/or interact with the (poly)peptides and/or nucleic acid molecules disclosed herein or with (poly)peptides and/or genes identified by the method(s) of the invention. Examples of such small molecules comprise, but are not limited to small peptides, anorganic and/or organic substances or peptide-like molecules, like peptide-analogs comprising D-amino acids. Said "inhibitors" and "stimulators" may further comprise antibodies, derivatives and/or fragments thereof, aptamers or specific (oligo)nucleotides. The "inhibitors" and "stimulators" may also be part of the pharmaceutical and/or diagnostic compositions as disclosed herein.

As pointed out herein above, said "inhibitors" or "stimulators" may also comprise small organic compounds as defined herein above.

According to the present invention, it is furthermore envisaged that the pharmaceutical composition provided by the present invention may be used in combination with other agents employed in the treatment of body weight/mass disorders. Said agents may comprise, but are not limited to, agents reducing/enhancing food intake, agents blocking/activating nutrient absorption, agents increasing/decreasing thermogenesis, agents modulating fat and/or protein metabolism or storage, agents modulating the central contoller regulating body weight. Said agents may, inter alia, comprise, agents like sibutramine, orlistat, ephedrine or caffeine, diethylpropione, phentermine, fluoxetine, sertraline, or phenylpropanolamine.

Moreover, the present invention envisages that the diagnostic composition may comprise the components as defined herein above wherein said components are bound to/attached to and/or linked to a solid support as defined herein above. It is furthermore envisaged, that said diagnostic composition comprises the compound(s) described above on (micro-)chips. Therefore, the diagnostic composition may, inter alia, comprise the nucleic acid molecules disclosed herein on so-called "gene chips" or the (poly) peptides disclosed herein on so-called "protein-chips". Diagnostic gene chips may comprise a collection of the above described nucleic acid molecules that, e.g., specifically detect mutations in the SOUP1-gene of animals, in particular of humans. The diagnostic compositions and in particular the diagnostic gene chip as described herein above may be particularly useful for screening patients for (genetic) defects underlying, e.g. obesity, adipositase, disorders of body weight/body mass, or eating disorders.

It is preferred that said compounds to be employed in a diagnostic composition are detectably labeled. A variety of techniques are available for labeling biomolecules, are well known to the person skilled in the art and are considered to be within the scope of the present invention. Such techniques are, e.g., described in Tijssen, "Practice and theory of enzyme immuno assays", Burden, RH and von Knippenburg (Eds), Volume 15 (1985), "Basic methods in molecular biology"; Davis LG, Dibmer MD; Battey Elsevier (1990), Mayer et al., (Eds) "Immunochemical methods in cell and molecular biology" Academic Press, London (1987), or in the series "Methods in Enzymology", Academic Press, Inc.

There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds.

In a particular preferred embodiment, the present invention relates to the use of a fruit fly as defined in herein above for the detection of polypeptides capable of contributing to membrane stability and/or function in organelles, capable of modifying mitochondrial proteins, and/or capable of influencing cellular metabolism.

Furthermore, the invention provides for a kit for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction, as diagnostic and/or pharmaceutical composition, comprising at least one of a nucleic acid molecule, a vector, a host, a polypeptide, a fusion protein, an antibody or a fragment or derivative thereof or an antiserum, an aptamer or another receptor and an anti-sense oligonucleotide as disclosed above.

Advantageously, the kit of the present invention further comprises, optionally (a) reaction buffer(s), storage solutions and/or remaining reagents or materials required for the conduct of scientific or diagnostic assays or the like. Furthermore, parts of the kit of the invention can be packaged individually in vials or bottles or in combination in containers or multicontainer units.

The kit of the present invention may be advantageously used, inter alia, for carrying out the method of producing a (poly)peptide disclosed herein and could be employed in a variety of applications referred herein, e.g., as diagnostic kits, as research tools or vaccination tools. Additionally, the kit of the invention may contain means for detection suitable for scientific medical and/or diagnostic purposes. The manufacture of the kits follows preferably standard procedures which are known to the person skilled in the art.

The figures show:
Figure 1 shows the nucleotide and amino acid sequence of Drosophila UCPy. Shown are the full length cDNA (SEQ ID NO:3), the open reading frame (SEQ ID NO:4), and the deduced amino acid sequence (SEQ ID NO:5).
Figure 2 shows the overexpression of Drosophila UCPy in the eye. In the fly shown on the left part of the picture, the dUCPy expression is normal, and the eye is normally developed. In the fly shown on the right part of the picture, dUCPy was overexpressed, and the phenotype observed shows a reduction of the fly eye. If SOUP1 is co-expressed in this phenotype, the eye defect can be rescued. The eye of such rescue flies (not shown) is almost identical to the normal eye of the left fly.
Figure 3a. shows the cDNA of Drosophila melanogaster for Accession Number GH22139. Shown are the full length cDNA nt 1-2953 (SEQ ID NO:6), the open reading frame (SEQ ID NO:7), and the deduced amino acid sequence (SEQ ID NO:8).
Figure 3b. shows the cDNA of Drosophila melanogaster for the splice variant of Accession Number GH22139. Shown are the dSOUP-sp1 open reading frame (nt 688-1497 and 2482-2641 of SEQ ID NO:6), and the deduced amino acid sequence for the SOUP splice variant dSOUP-sp1.
Figure 3c. shows the gene prediction from the SOUP1 locus in public databases. Shown are the open reading frame of splice variant dSOUP-CG8026 (nt 688-1569, 2280-2320 and 2482-2641 of SEQ ID NO:6), and the deduced amino acid sequence for splice variant dSOUP-CG8026, as predicted from public databases.
Figure 4 shows the transmembrane domain plot of SOUP1 proteins.
   Figure 4a shows the transmembrane domain plot for Drosophila SOUP1,
   Figure 4b shows the transmembrane domain plot for human SOUP1-CG8026,
   Figure 4c shows the transmembrane domain plot for human SOUP1,
   Figure 4d shows the transmembrane domain plot for mouse SOUP1,
   Figure 4e shows the transmembrane domain plot for zebrafish SOUP1.
Figure 5 shows the transmembrane domains of SOUP1 variants.
   Figure 5a shows the comparison of different Drosophila SOUP1 variants. The nucleotide numbering relates to the sequence in SEQ ID NO: 6. The transmembrane domains of splice varians dSOUP1 and dSOUP1-sp1 are different. Splice variant dSOUP1-CG8026 would generate a protein with seven transmembrane domains.
      The transmembrane domains in dSOUP1, variant dSOUP1-spl, variant dSOUP1-CG8026, human SOUP1 (hSOUP1), and mouse (mSOUP1) are shown in figures 5b, 5c, 5d, 5e and 5f, respectively.
Figure 6. shows human SOUP1. Shown are the open reading frame (SEQ ID NO:9), and the deduced amino acid sequence for human SOUP (SEQ ID NO:10).
Figure 7. shows mouse SOUP1. Shown are the open reading frame (SEQ ID NO:11), and the deduced aminoacid sequence for mouse SOUP (SEQ ID NO:12).
Figure 8. shows the alternative mouse gen 6i. Shown are the open reading frame (SEQ ID NO:13), and the deduced aminoacid sequence for mouse 6i (SEQ ID NO:14).
Figure 9. shows Danio rero SOUP1. Shown are the open reading frame (SEQ ID NO:51), and the deduced aminoacid sequence for mouse SOUP (SEQ ID NO:52).
Figure 10 shows the expression of UCP2 in a beta-actin-mSOUP-flg transgenic mouse model *in vivo.*

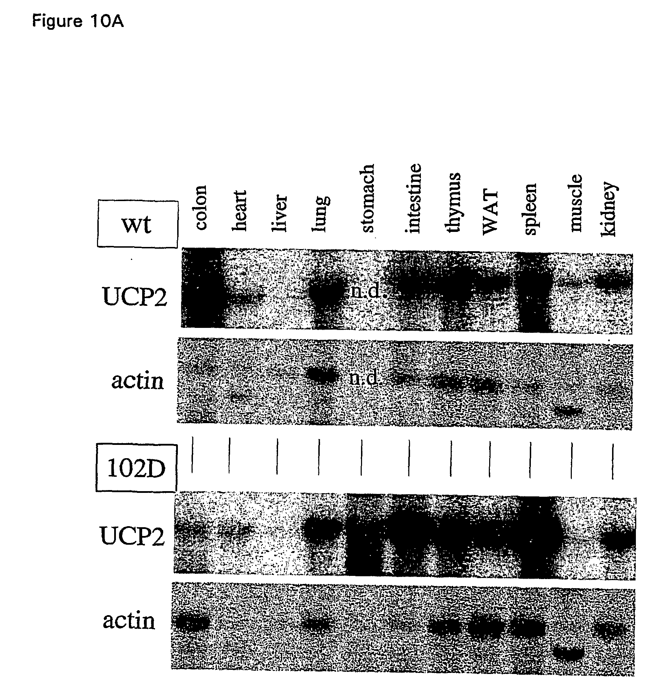

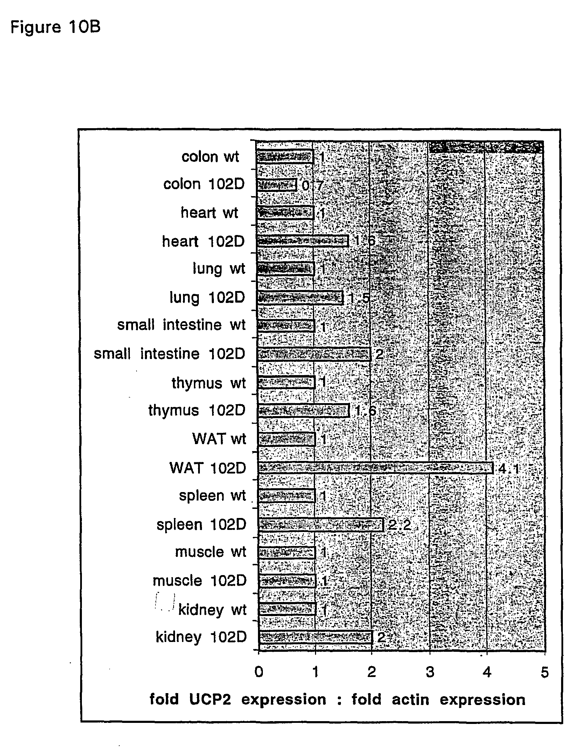

   Figure 10 a shows the northern analysis,
   Figure 10 b shows the quantification of the data shown in Figure 10 a.

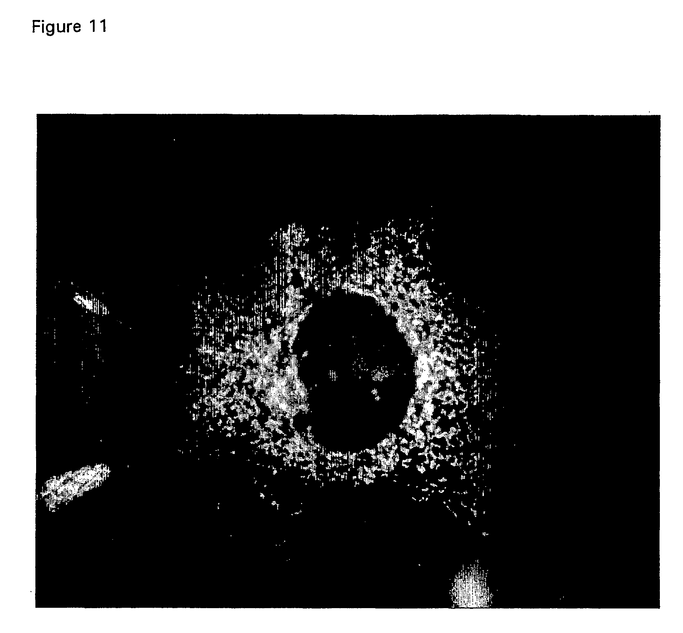

Figure 11 shows the localization of SOUP1 in mitochondria of NIH 3T3 cells. NIH 3T3 cells were transiently transfected with an expression vector for mouse Soup, fixed und immunostained with an antisera against mouse Soup (see Example 13).

The sequences show:

Sequences as shown in SEQ ID NOs: 15-26, 61 and 62 relate to transmembrane regions as deduced in a nucleic acid molecule obtainable from Drosophila melanogaster.

Sequences as shown in SEQ ID NOs: 27-38 relate to transmembrane domains as deduced in a nucleic acid molecule obtainable from Homo sapiens.

Sequences as shown in SEQ ID NOs: 39-50 relate to transmembrane domains of a protein deducible from Mus musculus.

The sequence as shown in SEQ ID NO: 8 depicts a polypeptide encoded, inter alia, by SEQ ID NO: 7 and represents a protein of Drosophila which has surprisingly been found to be involved in membrane function and/or stability of organelles and has, in particular, been found to be able to modify UCPs.

The amino acid sequence as depicted in SEQ ID NO: 54 (encoded, inter alia, by SEQ ID NO: 53) comprises a splice variant of the above described protein.

SEQ ID NO: 10 depicts the human homologue of SOUP1, SEQ ID NOs: 12 and 14 depict two variants of the mouse SOUP1, SEQ ID NO: 52 depicts SOUP1 of the zebrafish (Danio rero).

The examples illustrate the invention.

### Example 1: Cloning of a Drosophila melanogaster gene with homology to human Uncoupling Proteins (UCPs)

A BLAST homology search was performed in a public database (NCBI/NIH) looking for Drosophila genes with sequence homology to the human UCP2 and UCP3 genes. The search yielded sequence fragments of a family of Drosophila genes with UCP homology. They are clearly different to the next related mitochondrial proteins (oxoglutarate carrier).

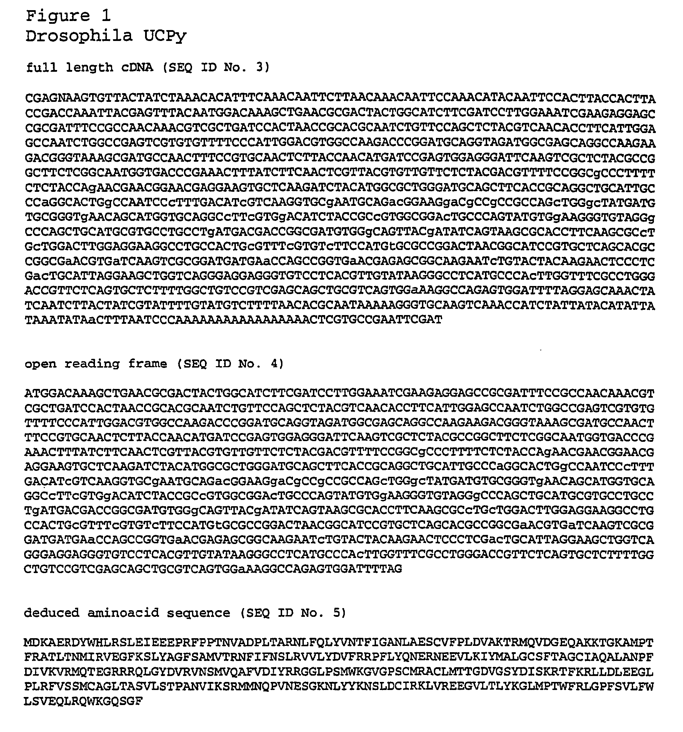

Using the sequence fragment of one of this genes (here called dUCPy) a PCR primer pair was generated (Upper 5'-CTAAACAAACAATTCCAAACATAG (SEQ ID NO: 1), Lower 5'-AAAAGACATAGAAAATACGATAGT (SEQ ID NO: 2)) and a PCR reaction performed on Drosophila cDNA using standard PCR conditions. The amplification product was radioactively labelled and used to screen a cDNA library made from adult Drosophila flies (Stratagene). A full-length cDNA clone was isolated, sequenced (figure 1) and used for further experiments.

### Example 2: Cloning of the dUCPy cDNA into an Drosophila expression vector

In order to test the effects of dUCPy expression in Drosophila cells the dUCPy cDNA was cloned into the expression vector pUAST (Ref.: Brand A & Perrimon N, Development 1993, 118:401-415) using the restriction sites Notl and Kpnl. The resulting expression construct was injected into the germline of Drosophila embryos and Drosophila strains with a stable integration of the construct were generated. Since the expression vector pUAST is activated by the yeast transcription factor Gal4 which is normally absent from Drosophila cells dUCPy is not yet expressed in these transgenic animals. If pUAST-dUCPy flies are crossed with a second Drosophila strain that expresses Gal4 in a tissue specific manner the offspring flies of this mating will express dUCPy in the Gal4 expressing tissue.

The cross of pUAST-dUCPy flies with a strain that expresses Gal4 in all cells of the body (under control of the actin promoter) showed no viable offspring. This means that dUCPy overexpression in all body cells is lethal. This finding is consistent with the assumption that dUCPy overexpression could lead to a collapse of the cellular energy production.

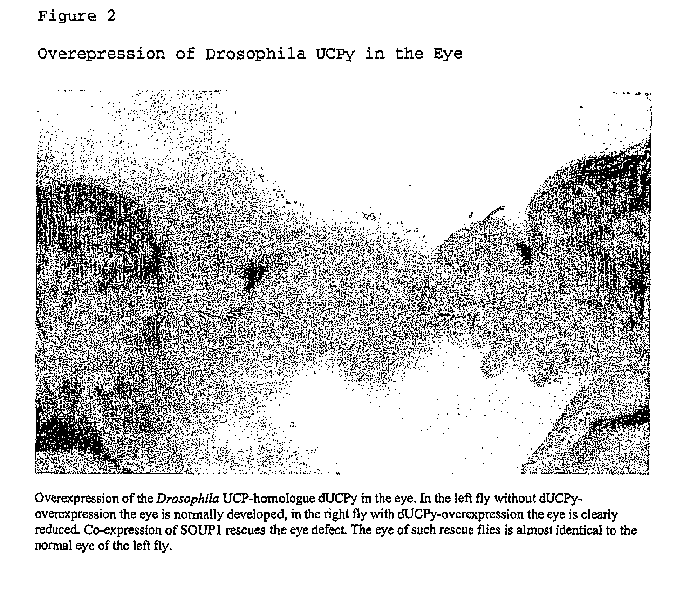

Expression of dUCPy in a non-vital organ like the eye (Gal4 under control of the eye-specific promoter of the "eyeless" gene) results in flies with visibly damaged eyes (figure 2). This easily visible eye phenotype is the basis of a genetic screen for gene products that can modify UCP activity.

### Example 3: dUCPy modifier screen

Parts of the genomes of the strain with Gal4 expression in the eye and the strain carrying the pUAST-dUCPy construct were combined on one chromosome using genomic recombination. The resulting fly strain has eyes that are permanently damaged by dUCPy expression. Flies of this strain were crossed with flies of a large collection of mutagenized fly strains. In this mutant collection a special expression system (EP-element, Ref.: Rrth P, Proc Natl Acad Sci U S A 1996, 93(22):12418-22) is integrated randomly in different genomic loci. The yeast transcription factor Gal4 can bind to the EP-element and activate the transcription of endogenous genes close the integration site of the EP-element. The activation of the genes therefore occurs in the same cells (eye) that overexpress dUCPy. Since the mutant collection contains several thousand strains with different integration sites of the EP-element it is possible to test a large number of genes whether their expression interacts with dUCPy activity. In case a gene acts as an enhancer of UCP activity the eye defect will be worsened; a suppressor will ameliorate the defect.

Using this screen a novel gene with suppressing activity was discovered that is here called Suppressor Of Uncoupling Protein (SOUP1). Expression of SOUP1 together with dUCPy in the Drosophila eye leads to a rescue of the dUCPy induced defect (figure 2).

### Example 4: Cloning of SOUP1 from Drosophila (dSOUP1)

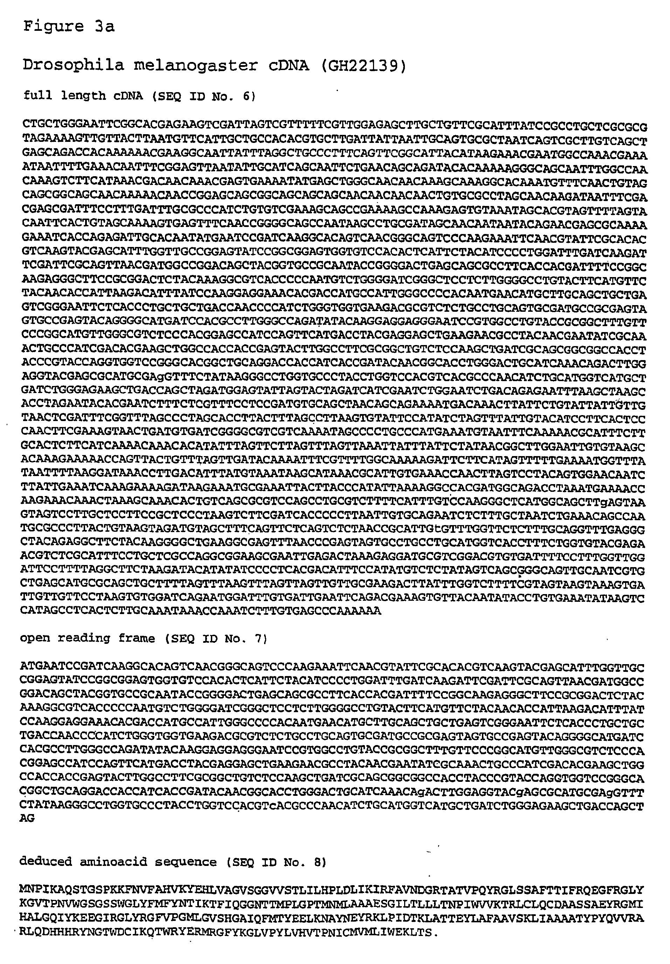

Genomic DNA neighbouring to the eye-defect rescuing EP-element was cloned and sequenced. This sequence was used for a BLAST search in a public Drosophila gene database. A short sequence fragment (EST) from cDNA clone GH22139 (Drosophila Genome Project) with identical sequence was discovered in the database search. This publicly available cDNA clone was ordered and then fully sequenced (figure 3a).

### Example 5: Overexpression of GH22139 in the Drosophila eye

To ensure that GH22139 encodes for the genetically identified dUCPy suppressor SOUP1 the GH22139 cDNA was cloned into the expression vector pUAST (using restriction sites Bglll and Xhol). By means of germ line injection transgenic animals were generated. pUAST-GH22139 transgenic flies were crossed with the flies expressing dUCPy in the eye. In the offspring of this cross (flies expressing dUCPy and GH22139) the eye defect was rescued. This proved that the GH22139 cDNA encodes for SOUP1.

### Example 6: Sequence analysis of dSOUP1

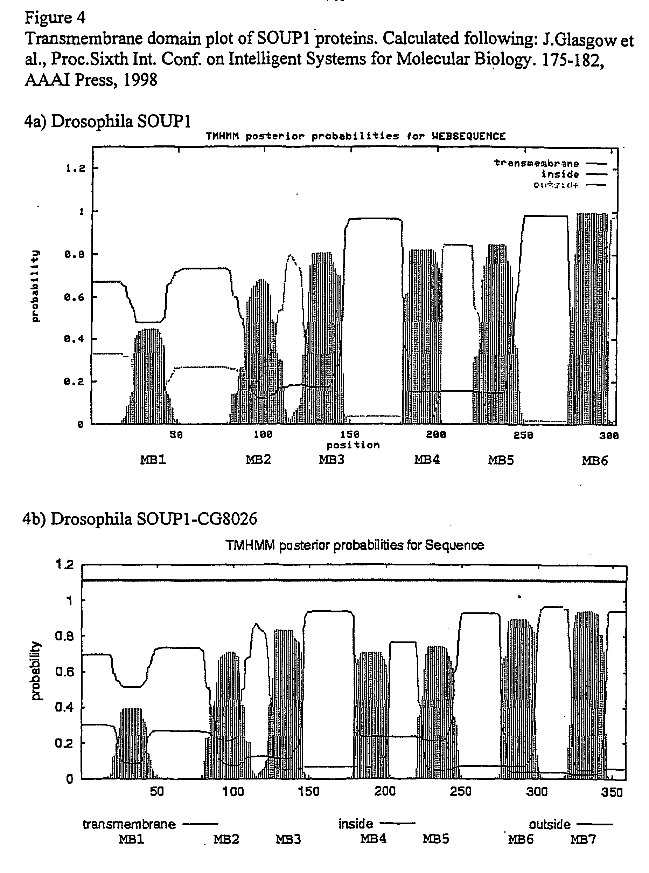

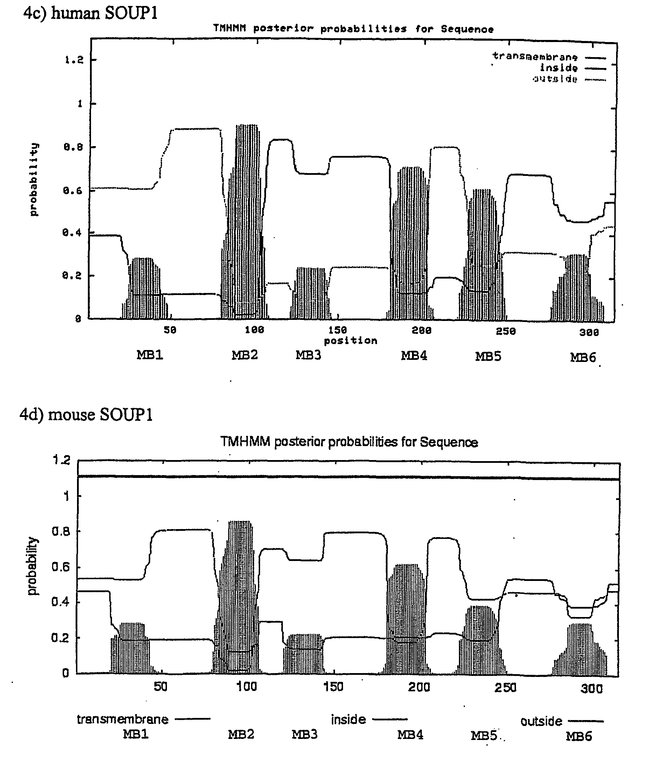

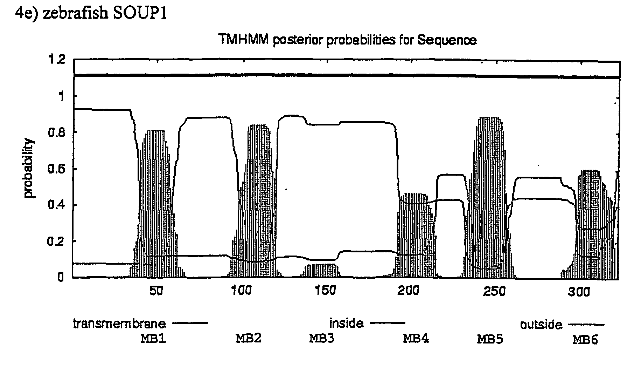

The analysis of the deduced amino acid sequence of the SOUP1 cDNA with bioinformatic tools (Ref.: J.Glasgow et al., Proc.Sixth Int. Conf. on Intelligent Systems for Molecular Biology. 175-182, AAAI Press, 1998) showed that the SOUP1 protein has the characteristic pattern of a mitochondrial carrier protein with 6 transmembrane domains (figure 4a).

### Example 7: Possible splice variants of dSOUP1 and comparison with a predicted gene in public databases

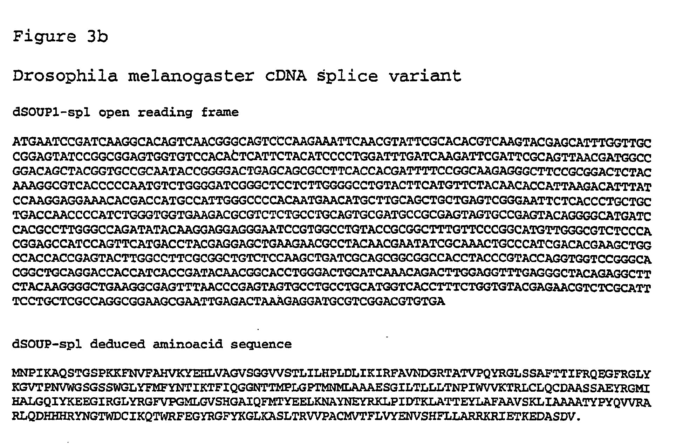

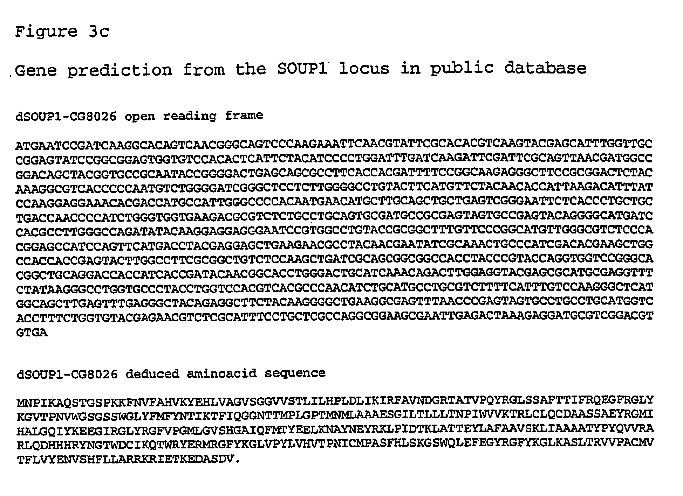

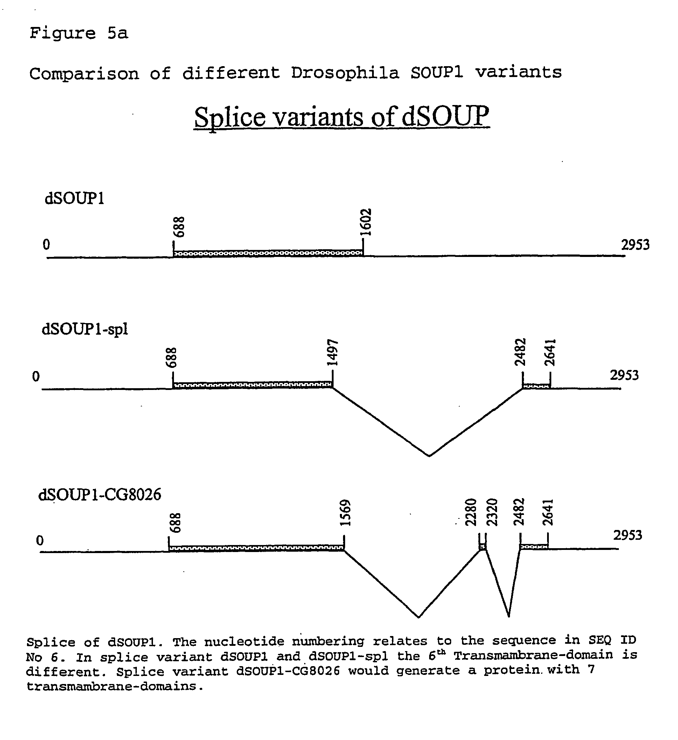

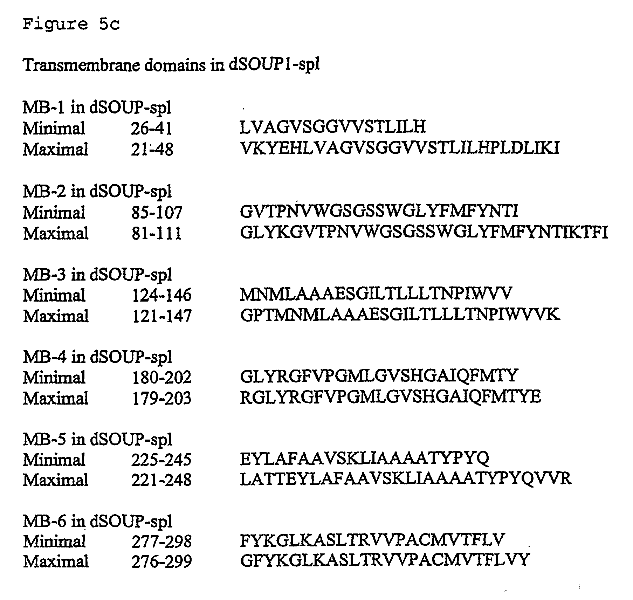

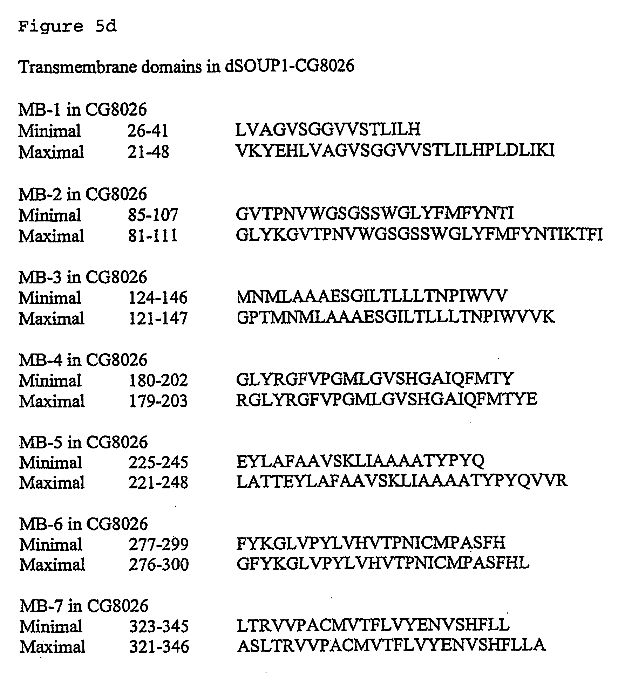

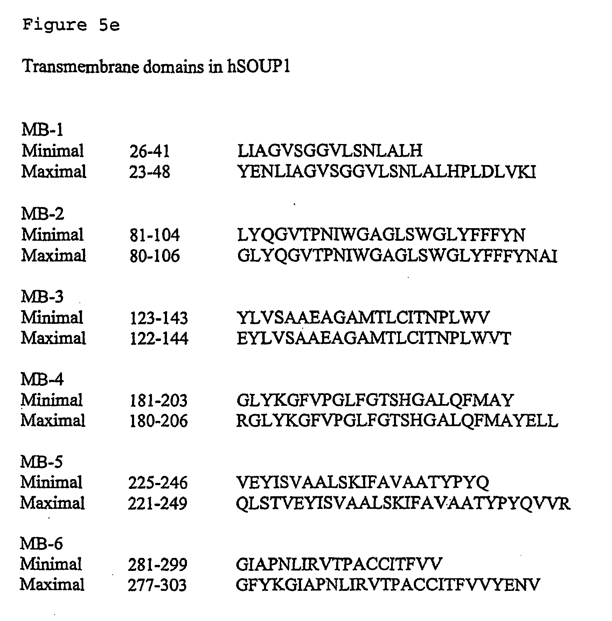

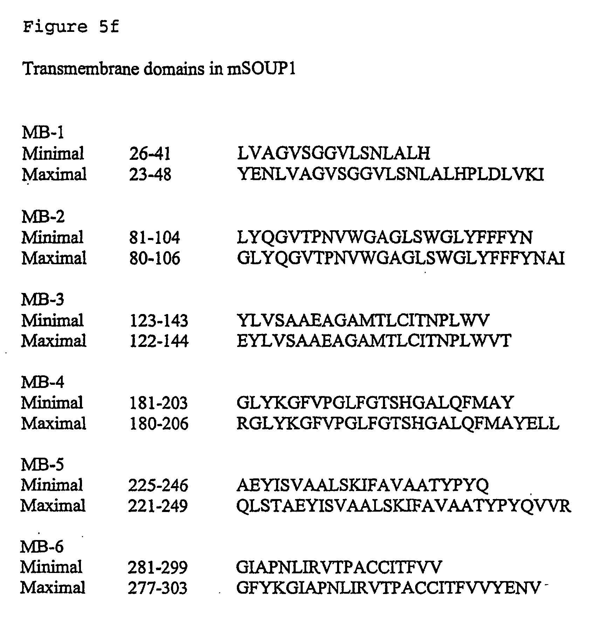

Sequence alignments, transmembrane-domain predictions and splice-predictions suggest that the Drosophila SOUP1 gene exists in different splice variants (Figure3b, c and Fig. 5a). The cDNA clone GH22139 can form two splice variants (dSOUP1 and dSOUP1-spl) with a different 6^{th} TM-domain. Searching the fully sequenced Drosophila genome in public databases revealed a gene prediction (dSOUP1-CG8026, Ref. Adams, M.D. et al., 2000 Science 287:2185) that suggests a splice variant with 7 TM-domains. The homologies between the variant are:

| amino acid homology in % | (identity/similarity) |
|---|---|
| dSOUP1- dSOUP1-CG8026 | (82/82) |
| dSOUP1 spl - dSOUP1-CG8026 | (89/89) |
| dSOUP1 - dSOUP1spl | (93/94) |

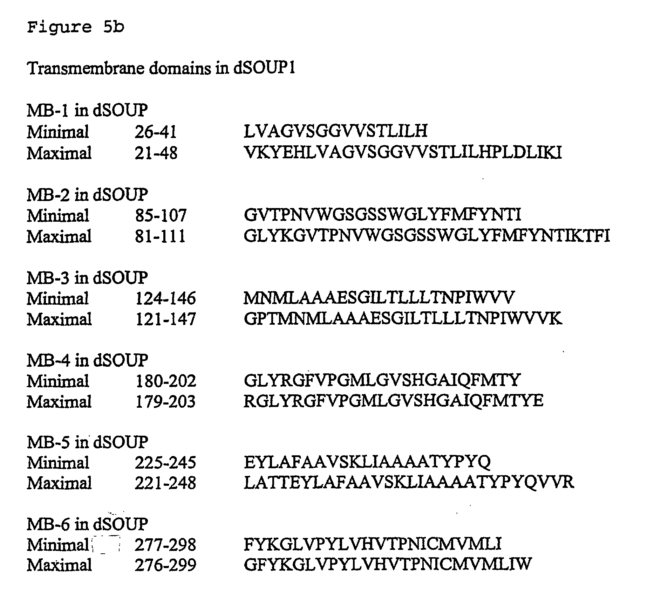

The amino acids of the predicted TM-domains of the different splice variant an species are depicted in Fig. 5b to f).

### Example 8: Cloning of SOUP1 homologues from human (hSOUP1), mouse (mSOUP1), and zebrafish (zSOUP1)

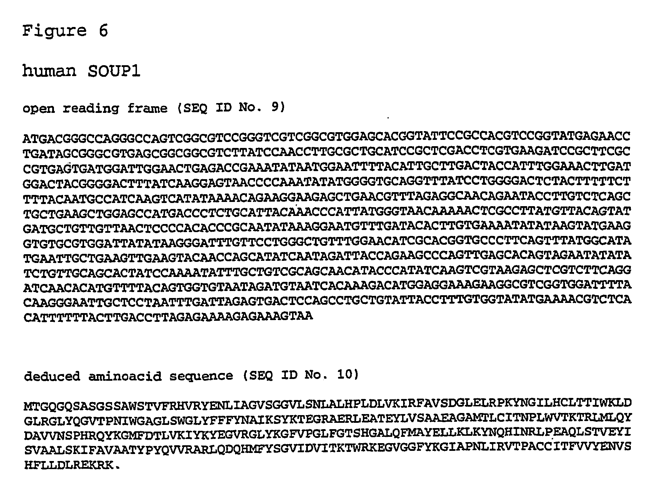

Using the complete cDNA sequence of Drosophila SOUP1 a BLAST search in a public database was performed. Several short, uncomplete sequence fragments from different species (zebra fish, chick, mouse, macaque, human) were found. Since none of the sequences in the database were complete the human sequence fragment was used to synthesize PCR-primers. The PCR amplification product was radioactively labeled and used as a probe to screen a human adipocyte cDNA library and a human hippocampus cDNA library. From both libraries cDNA clones could be isolated. The sequence of the human SOUP1 is shown in figure 6. It shows the characteristic 6 transmembrane domain pattern as well (figure 4c). The following PCR primers can be used to amplify the ORF of the human SOUP1 gene: Upper 5'- ATG GAC TAC GGG GAC TTT ATC AA (SEQ ID NO: 55), Lower 5'- ACC GAC GCC TTC TTT CCA ACT (SEQ ID NO: 56).

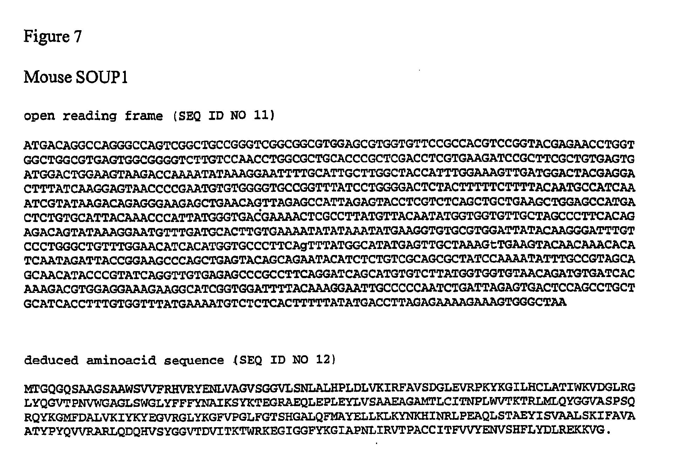

The identification of the mouse Soup1 gene was done in an equivalent approach. The sequence of mSOUP1 is shown in figure 7. It shows the characteristic 6 transmembrane domain pattern as well (figure 4d). The following PCR primers can be used to amplify the ORF of the mouse SOUP1 gene: Upper 5'-GGC GGC CAC TAC ATC ACG (SEQ ID NO: 57), Lower 5'-TGCCTCAAGAACATAGACTG (SEQ ID NO: 58).

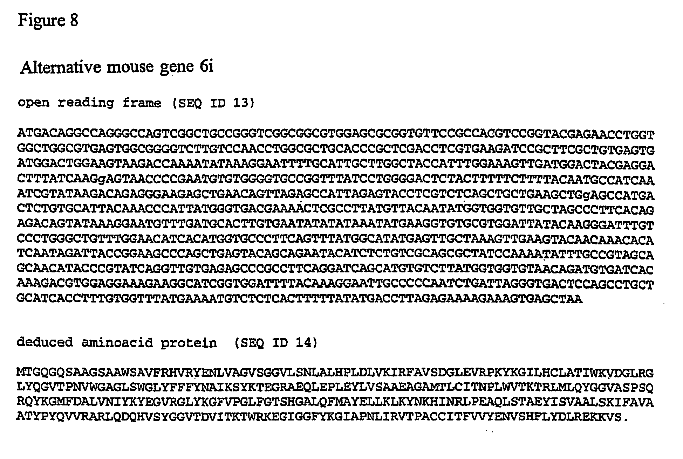

During the isolation of mouse SOUP1 cDNA clones the clone 6i was isolated that has an alternative sequence. The sequence of the open reading frame is shown in figure 8. The deduced amino-acid sequence differs in three positions from the other mouse SOUP1 clone.

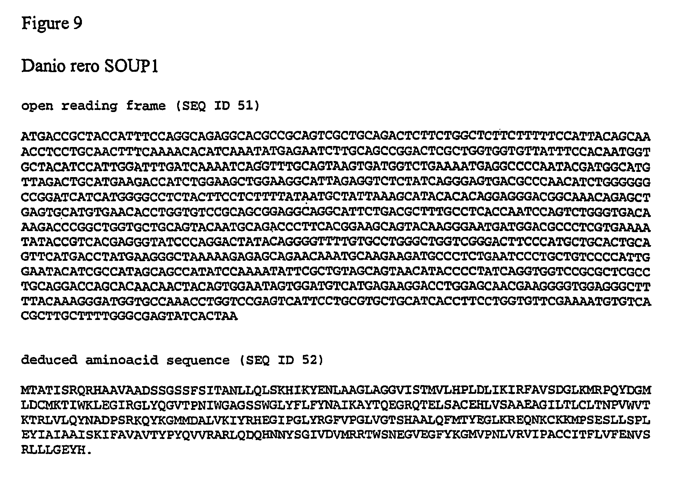

The identification of the zebrafish Soup1 gene was done in an equivalent approach. The sequence of zSOUP1 is shown in figure 9. It shows the characteristic 6 transmembrane domain pattern as well (figure 4e). The following PCR primers can be used to amplify the ORF of the zebrafisch SOUP1 gene: Upper 5'-ATGACCGCTACCATTTCCAGGCAGAGGCAC (SEQ ID NO: 59), Lower 5'-TTAGTGATACTCGCCCAAAAGCAAGCGTGA (SEQ ID NO: 60).

### Example 9: Generation of a flag-tagged mSOUP transgene

A carboxy-terminally flag-tagged *mSOUP* transgene (herein referred to as *mSOUPFlag)* was generated by performing RT-PCR on cDNA derived from mouse pancreas RNA using *mSOUP* gene specific primers *(mSOUP* forward primer: 5' GGCGGCCACTACATCACG3' (SEQ ID NO:63), flag-tagged mSOUP reverse primer: 5' CTACTTGTCATCATCGTCCTTGTAGTCGCTCACTTTCTTTTCTCT 3' (SEQ ID NO:64)).

### Example 10: Generation of beta-actin-mSOUPFIag transgenic mice

The *mSOUPFlag* (see Example 9) was expressed in mice under the control of the ubiquitous human beta-actin promoter using techniques known to those skilled in the art (for example, see, Gunnig et al. (1987). *Proc. Natl. Acad. Sci. USA* **84**,4831-4835). A transgenic construct DNA was injected into C57/BL6 mouse embryos (Harlan Winkelmann, Borchen, Germany) using standard techniques (see, for example, Brinster et al. (1985). *Proc. Natl. Acad. Sci. USA* **82**,4438-4442). Using this technique, eight independent mouse founderlines were generated.

### Example 11: mSOUP expression analysis via TaqMan analysis

The expression of the *mSOUPFlag* transgene was monitored by TaqMan analysis. For this analysis, 1-100 ng, preferably 50ng cDNA derived from different mouse tissues (for example, colon, heart, liver, lung, stomach, intestine, white adipose tissue (WAT), thymus, spleen, muscle, kidney) and a *mSOUP* specifc primer/probe pair were used *(mSOUP* forward primer (SEQ ID NO: 65): 5'-CTCTGTCGCAGCGCTATCC-3', *mSOUP* reverse primer (SEQ ID NO: 66): 5'-GAAGGCGGGCTCTCACAAC-3', *mSOUP* probe (SEQ ID NO: 67): 5'-AATATTTGCCGTAG CAGCAACATACCCGT-3').

TaqMan analysis was performed using standard techniques known to those skilled in the art.

In the eight independent mouse founder lines analysed, ectopic *mSOUP* expression using wild-type mice as a reference was detected in all tissues apart from brown adipose tissue (BAT). The two founder lines showing highest transgene expression levels were used for further analysis.

### Example 12: mUCP2 expression analysis via Northern blot analysis

To study the effect of ubiquitous, ectopic *mSOUPFlag* expression in a transgenic mouse model *in vivo, mUCP2* expression in actin*-mSOUPFlag* transgenic mice (and in control littermate) was monitored by Northern blot analysis using 20g total RNA and a 1.0kb *mUCP2* cDNA encompassing the complete UCP2 open reading frame as a probe. To ensure equal loading, the Northern blot was rehybridized with a 1.4kb *human-actin* cDNA as a probe. Northern blot analysis was performed using standard techniques (see, for example, Sambrook et al. (1989) Molecular Cloning, a laboratory manual. Second Edition, Cold Spring Harbor Laboratory Press).

Expression of mouse UCP2 was detected in all tissues tested (see FIGURE 10). Comparison of *mUCP2* expression levels in transgenic mice relative to control littermates revealed increased *mUCP2* expression in several different tissues analysed.

Quantification analysis using an Instant Imager and corresponding analysis software showed 1.5-4.1 fold increased *mUCP2* expression in lung, small intestine, thymus, WAT, spleen and kidney of actin-*mSOUPFlag* transgenic mice relative to the wild-type control littermates (see FIGURE 10B)

### Example 13: Fluorescence microscopy analysis of mouse Soup localisation in NIH3T3 cells.

NIH 3T3 cells were transiently transfected with an expression vector for mouse Soup, fixed und immunostained with an antisera against mouse Soup. The immunofluorescence was examined at 630x magnification and appropriate filters for the Cy3-labelled secondary antibody. It was shown that Soup is cleared localized in the mitochondria of NIH 3T3 cells (SEE FIGURE 11).

NIH3T3 cells were seeded into 24 wells plates containing Poly-D-Lysine coated coverslips (BD Biosciences, Erembodegem, Belgium) at 25.000 cells per well. The day after seeding, cells were transiently transfected with the Soup expression construct under the control of a CMV-Promoter with Lipofectamin Plus (InvitroGen, Karlsruhe, Germany) according to the manufacturer's instructions. 48 hours after transfection, cells were fixed in 4% para-formaldehyde and immunostained according to Dorner *et al.* (1998) J Biol Chem. **273**: 20267-75). In brief, cells were permeabilized with 0.75% Triton X-100 for 10 minutes in PBS, endogenous autofluorescence blocked by treatment of the cells with 0.1 NaBH₄ in PBS for 10 minutes. After a short PBS wash, cell were incubated in blocking buffer (PBS, 0.5 % BSA, 5% goat serum, 0.045% fish gelatine) for 1 hour. Primary antisera (1:25, overnight) and secondary antibody (anti-rabbit-Cy3, 1:200, 1 hour) were applied in blocking buffer followed by washes in blocking buffer. The cover slips were mounted on glass slides and immunostained cells were examined in an fluorescence microscope with the appropriate filter set for Cy3.

### Example 14: Anti mSOUP antibodies

Anti mouse SOUP antibodies were raised in rabbits by immunizing with the synthetic peptide SEQ ID NO:68: CYENVSHFLYDLREKKVS, corresponding to the C-terminus of mouse SOUP protein (C- a.a. 300-316 from SEQ ID NO:14). The antibodies were affinity purified using a SULFO-Link column (Order No. Pierce 44895) carrying the synthetic peptide SEQ ID NO:68: (CYENVSHFLYDLREKKVS), according to the manufacturers' protocol.

## Claims

1. Use of
(i) a nucleic acid molecule encoding a polypeptide, contributing by means of protein-protein interactions as well as protein-lipid interactions to membrane stability and/or contributing to membrane function of organelles by having transport functions, regulator functions of other membrane proteins or modifier functions of other (membrane) proteins and/or other functions,
(ii) a polypeptide encoded by said nucleic acid,
(iii) a fusion protein comprising said polypeptide,
(iv) an antibody, fragment or derivative thereof or an aptamer specifically recognizing said nucleic acid, polypeptide or fusion protein,
(v) an antisense oligonucleotide, ribozyme, hybridization probe or amplification primer of said nucleic acid molecule
for the manufacture of a diagnostic and/or pharmaceutical composition for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction, wherein said nucleic acid molecule
(a) remains hybridized at 65°C in a solution containing 0.2 x SSC and 0.1% SDS to a nucleic acid molecule encoding the amino acid sequence of SEQ ID NO: 10, SEQ ID NO: 12 and/or SEQ ID NO: 14 and/or the complementary strand thereof;
(b) remains hybridized at 65°C in a solution containing 0.2 x SSC and 0.1 % SDS to a nucleic acid molecule as depicted in SEQ ID NO: 6, SEQ ID NO: 9, SEQ ID NO: 11 and/or SEQ ID NO: 13 and/or the complementary strand thereof;
(c) is degenerate with respect to the nucleic acid molecule of (a) or (b);
(d) encodes a polypeptide which is at least 90% identical to the amino acid sequence as depicted in any one of SEQ ID NOs: 10, 12 or 14; or
(e) has the sequence as depicted in SEQ ID NOs: 9, 11 or 13.

2. The use of claim 1, wherein said nucleic acid molecule is DNA.

3. The use of claim 1 or 2, wherein said polypeptide is expressed in mitochondria and/or peroxisomes.

4. The use of claim 3, wherein said polypeptide participates in the maintenance of said membrane.

5. The use of any one of claims 1 to 4, wherein said polypeptide is a transporter molecule and/or a regulator of a transporter molecule.

6. The use of any one of claims 1 to 5, wherein said polypeptide is a modifying polypeptide, which controls the function of one or more proteins/polypeptides.

7. The use of claim 6, wherein said modifying polypeptide controls the function of the mitochondrial protein UCP2.

8. The use of any one of claims 1 to 7, wherein said nucleic acid molecule is comprised on a vector.

9. Use of a non-human transgenic animal comprising a nucleic acid molecule encoding a polypeptide, contributing by means of protein-protein interactions as well as protein-lipid interactions to membrane stability and/or contributing to membrane function of organelles by having transport functions, regulator functions of other membrane proteins or modifier functions of other (membrane) proteins and/or other functions, wherein said nucleic acid molecule
(a) remains hybridized at 65°C in a solution containing 0.2 x SSC and 0.1 % SDS to a nucleic acid molecule encoding the amino acid sequence of SEQ ID NO: 8, SEQ ID NO: 54, SEQ ID NO: 10, SEQ ID NO: 12, SEQ ID NO: 14 and/or SEQ ID NO: 52 and/or the complementary strand thereof;
(b) remains hybridized at 65°C in a solution containing 0.2 x SSC and 0.1% SDS to a nucleic acid molecule as depicted in SEQ ID NO: 6, 7 or 53, SEQ ID NO: 9, SEQ ID NO: 11, SEQ ID NO: 13 and/or SEQ ID NO: 51 and/or the complementary strand thereof;
(c) is degenerate with respect to the nucleic acid molecule of (a) or (b);
(d) encodes a polypeptide which is at least 90% identical to SEQ ID NO: 8;
(e) encodes a polypeptide which is at least 90% identical to SEQ ID NO: 54;
(f) encodes a polypeptide which is at least 90% identical to the amino acid sequence as depicted in any one of SEQ ID NOs: 10, 12, 14 or 52; or
(g) has the sequence as depicted in SEQ ID NOs: 9, 11, 13 or 51;
expressing a polypeptide encoded by said nucleic acid molecule or a fusion protein comprising said polypeptide; or transfected with a vector comprising said nucleic acid molecule as a research model for obesity, adipositas, eating disorders, wasting and/or other disorders of body weight/body mass.

10. The use of claim 9, wherein the nucleic acid molecule as defined in any one of claims 1 to 7 is silenced and/or mutated, as a research model for obesity, adipositas, eating disorders, wasting and/or other disorders of body weight/body mass.

11. The use of claim 9 or 10, wherein the animal is selected from the group consisting of mouse, rat, sheep, hamster, pig, dog, monkey, rabbit, calf, horse, nematodes, fly and fish.

12. An in vitro method of identifying a polypeptide or (a) substance(s) involved in a metabolic disorder selected from obesity, adipositas, eating disorders (bulimia nervosa, anorexia nervosa), cachexia (wasting) and pancreatic dysfunction, comprising the steps of:
(a) testing a collection of polypeptides or substances for interaction with the polypeptide or the fusion protein as defined in claim 1 using a readout system; and
(b) identifying polypeptides or substances which test positive for interaction in step (a).

13. An in vitro method of claim 12, comprising the further step of
(c) repeating steps (a) and (b) with the polypeptides identified one or more times wherein the newly identified polypeptide replaces the previously identified polypeptide as a bait for the identification of a further interacting polypeptide.

14. The method of claim 12 or 13, further comprising the step of identifying the nucleic acid molecule(s) encoding the one or more interacting (poly) peptides.

15. An in vitro method of identifying a polypeptide involved in the regulation of body weight in a mammal, comprising the steps of
(a) contacting a collection of (poly)peptides with the polypeptide or the fusion protein as defined in claim 1 under conditions that allow binding of said (poly)peptides;
(b) removing (poly)peptides from said collection of (poly)peptides that did not bind to said polypeptide or fusion protein as defined in claim 1 in step (a); and
(c) identifying (poly)peptides that bind to said polypeptide or the fusion protein as defined in claim 1.

16. The method of claim 15, wherein said polypeptide or fusion protein as defined in claim 1 is fixed to a solid support.

17. The method of claim 16, wherein said solid support is a gel filtration or an affinity chromatography material.

18. The method of any one of claims 15 to 17, wherein, prior to said identification in step (c), said binding (poly)peptides are released.

19. The method of claim 18, wherein said release is effected by elution.

20. The method of any one of claims 15 to 19, further comprising the step of identifying the nucleic acid molecule(s) encoding the one or more binding (poly)peptides.

21. A method of identifying a compound for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction, wherein said compound influences the expression of the nucleic acid molecule as defined in any one of claims 1 to 7, comprising the steps of
(a) contacting a host cell or non-human host carrying an expression vector comprising the nucleic acid molecule as defined in any one of claims 1 to 7 operatively linked to a readout system with a compound or a collection of compounds;
(b) assaying whether said contacting results in a change of signal intensity provided by said readout system; and, optionally,
(c) identifying a compound within said collection of compounds that induces a change of signal in step (b);
wherein said change in signal intensity correlates with a change of expression of said nucleic acid molecule.

22. A method of identifying a compound for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction,
wherein said compound influences the activity of a polypeptide as defined in claim 1, comprising the steps of
(a) contacting a host cell or non-human host carrying an expression vector comprising the nucleic acid molecule as defined in any one of claims 1 to 7 operatively linked to a readout system and/or carrying the polypeptide as defined in claim 1 linked to a readout system with a compound or a collection of compounds;
(b) assaying whether said contacting results in a change of signal intensity provided by said readout system; and, optionally
(c) identifying a compound within said collection of compounds that induces a change of signal in step (b);
wherein said change in signal correlates with a change in activity of said (poly)peptide.

23. The method of claim 21 or 22, wherein said host cell is an eukaryotic host cell.

24. The method of claim 23, wherein said eukaryotic host cell is a mammalian host cell.

25. The method of claim 21 or 22, wherein said host cell is a bacterium or a yeast.

26. The method of any one of claims 22 to 25, wherein said change in signal intensity is an increase in signal intensity

27. The method of any one of claims 22 to 25, wherein said change in signal intensity is a decrease in signal intensity.

28. A non-therapeutic method of assessing the impact of the expression of one or more polypeptides or of one or more fusion proteins as defined in claim 1 in a non-human animal, comprising the steps of
(a) overexpressing or underexpressing the nucleic acid molecule as defined in any one of claims 1 to 7 or the nucleic acid molecule identified by the method of claim 14 or 20 in said animal; and
(b) determining whether the weight of said animal has increased, decreased, whether metabolic changes are induced and/or whether the eating behaviour is modified.

29. A method of screening for an agent which modulates the interaction of a polypeptide as defined in claim 1 capable of detecting, verifying, treating, alleviating and/or preventing a metabolic disorder selected from obesity, adipositas, eating disorders (bulimia nervosa, anorexia nervosa), cachexia (wasting) and pancreatic dysfunction with a binding target/agent, comprising the steps of
(a) incubating a mixture comprising
(aa) a polypeptide or a fusion protein as defined in claim 1
(ab) a binding target/agent of said (poly)peptide or fusion protein or fragment thereof; and
(ac) a candidate agent
under conditions whereby said (poly)peptide, fusion protein or fragment thereof specifically binds to said binding target/agent at a reference affinity;
(b) detecting the binding affinity of said (poly)peptide, fusion protein or fragment thereof to said binding target to determine an (candidate) agent-biased affinity; and
(c) determining a difference between (candidate) agent-biased affinity and the reference affinity.

30. Kit for the detection and/or verification or for the treatment, alleviation and/or prevention of a metabolic disorder selected from obesity, adipositas, eating disorders (bulimia nervosa, anorexia nervosa), cachexia (wasting) and pancreatic dysfunction, as diagnostic and/or pharmaceutical composition, comprising at least one of
(a) a nucleic acid molecule as defined in any one of claims 1 to 7;
(b) a vector as defined in claim 8;
(c) a host cell or non-human host organism transformed with the vector as defined in claim 8,
(d) a polypeptide as defined in claim 1;
(e) a fusion protein as defined in claim 1;
(f) an antibody or a fragment or derivative thereof or an antiserum, or an aptamer as defined in claim 1; and
(g) an antisense oligonucleotide, ribozyme, hybridization probe or amplification primer as defined in claim 1.

## Patentansprüche

1. Verwendung
(i) eines Nukleinsäuremoleküls, welches ein Polypeptid kodiert, das mittels Protein-Protein-Wechselwirkungen sowie Protein-Lipid-Wechselwirkungen zu Membranstabilität und/oder zur Membranfunktion von Organellen beiträgt, indem es Transportfunktionen, regulatorische Funktionen anderer Membranproteine oder Modifikatorfunktionen anderer (Membran-)Proteine und/oder andere Funktionen aufweist,
(ii) eines von der Nukleinsäure kodierten Polypeptids,
(iii) eines Fusionsproteins umfassend das Polypeptid,
(iv) eines Antikörperfragments oder dessen Derivat oder eines Aptamers, das die Nukleinsäure, das Polypeptid oder das Fusionsprotein erkennt,
(v) eines Antisense-Oligonukleotids, einer Hybridisierungssonde oder eines Amplifikationsprimers des Nukleinsäuremoleküls
für die Herstellung einer diagnostischen und/oder pharmazeutischen Zusammensetzung für die Erkennung und/oder Verifizierung oder für die Behandlung, Linderung und/oder Vorbeugung einer Stoffwechselerkrankung, ausgewählt aus Übergewicht, Adipositas, Essstörungen (Bulimia nervosa, Anorexia nervosa), Kachexie (Auszehrung) und Pankreasfunktionsstörung, wobei das Nukleinsäuremolekül
(a) bei 65°C in einer Lösung, die 0,1 x SSC und 0,1% SDS enthält, an ein Nukleinsäuremolekül, welches die Aminosäuresequenz von SEQ ID Nr.: 10, SEQ ID Nr.: 12 und/oder SEQ ID Nr.: 14 kodiert, und/oder den komplementären Strang davon hybridisiert bleibt;
(b) bei 65°C in einer Lösung, die 0,2 x SSC und 0,1% SDS enthält, an ein Nukleinsäuremolekül wie dargestellt in SEQ ID Nr.: 6, SEQ ID Nr.: 9, SEQ ID Nr.: 11 und/oder SEQ ID Nr.: 13 und/oder an den komplementären Strang davon hybridisiert bleibt;
(c) in Bezug auf das Nukleinsäuremolekül von (a) oder (b) degeneriert ist;
(d) ein Polypeptid kodiert, das zu mindestens 90% identisch ist zu der Aminosäuresäuresequenz wie dargestellt in einer Beliebigen der SEQ ID Nr.: 10, 12 oder 14; oder
(e) die Sequenz wie in SEQ ID Nr. 9, 11 oder 13 gezeigt aufweist.

2. Verwendung nach Anspruch 1, wobei es sich bei dem Nukleinsäuremolekül um DNA handelt.

3. Verwendung nach Anspruch 1 oder 2, wobei das Polypeptid in Mitochondrien und/oder Peroxisomen exprimiert wird.

4. Verwendung nach Anspruch 3, wobei das Polypeptid an der Erhaltung der Membran beteiligt ist.

5. Verwendung nach einem der Ansprüche 1 bis 4, wobei es sich bei dem Polypeptid um ein Transportermolekül und/oder einen Regulator eines Transportermoleküls handelt.

6. Verwendung nach einem der Ansprüche 1 bis 5, wobei es sich bei dem Polypeptid um ein modifizierendes Polypeptid handelt, das die Funktion eines oder mehrerer Proteine/Polypeptide kontrolliert.

7. Verwendung nach Anspruch 6, wobei das modifizierende Polypeptid die Funktion des Mitochondrienproteins UCP2 kontrolliert.

8. Verwendung nach einem der Ansprüche 1 bis 7, wobei das Nukleinsäuremolekül auf einem Vektor vorliegt.

9. Verwendung eines nicht-humanen transgenen Tieres, welches ein Nukleinsäuremolekül aufweist, welches ein Polypeptid kodiert, das mittels Protein-Protein-Wechselwirkungen sowie Protein-Lipid-Wechselwirkungen zu Membranstabilität und/oder zur Membranfunktion von Organellen beiträgt, indem es Transportfunktionen, regulatorische Funktionen anderer Membranproteine oder Modifikatorfunktionen anderer (Membran-)Proteine und/oder andere Funktionen aufweist, wobei das Nukleinsäuremolekül
(a) bei 65°C in einer Lösung, die 0,2 x SSC und 0,1% SDS enthält, an ein Nukleinsäuremolekül, welches die Aminosäuresequenz von SEQ ID Nr.: 8, SEQ ID Nr.: 54, SEQ ID Nr.: 10, SEQ ID Nr.: 12, SEQ ID Nr.: 14 und/oder SEQ ID Nr.: 52 kodiert, und/oder den komplementären Strang davon hybridisiert bleibt;
(b) bei 65°C in einer Lösung, die 0,2 x SSC und 0,1% SDS enthält, an ein Nukleinsäuremolekül wie dargestellt in SEQ ID Nr.: 6, 7 oder 53, SEQ ID Nr.: 9, SEQ ID Nr.: 11, SEQ ID Nr.: 13 und/oder SEQ ID Nr.: 51 und/oder an den komplementären Strang davon hybridisiert bleibt;
(c) in Bezug auf das Nukleinsäuremolekül von (a) oder (b) degeneriert ist;
(d) ein Polypeptid kodiert, das zu mindestens 90% zu SEQ ID Nr.: 8 identisch ist;
(e) ein Polypeptid kodiert, das zu mindestens 90% zu SEQ ID Nr.: 54 identisch ist;
(f) ein Polypeptid kodiert, das zu mindestens 90% zu der in einer Beliebigen von SEQ ID Nr.: 10, 12, 14 oder 52 gezeigten Aminosäuresequenz identisch ist;
(g) die Sequenz wie in SEQ ID Nr. 9, 11, 13 oder 51 gezeigt aufweist,
wobei ein Polypeptid exprimiert wird, das von dem Nukleinsäuremolekül oder einem Fusionsprotein kodiert wird, welches das Polypeptid umfasst; oder das mit einem Vektor transfiziert ist, welcher das Nukleinsäuremolekül umfasst, als ein Forschungsmodell für Übergewicht, Adipositas, Essstörungen, Auszehrungs- und/oder andere Störungen des Körpergewichts/der Körpermasse.

10. Verwendung nach Anspruch 9, wobei das Nukleinsäuremolekül wie definiert in einem der Ansprüche 1 bis 7 stumm geschaltet und/oder mutiert ist als ein Forschungsmodell für Übergewicht, Adipositas, Essstörungen, Auszehrungs- und/oder andere Störungen des Körpergewichts/der Körpermasse.

11. Verwendung nach Anspruch 9 oder 10, wobei das Tier aus der Gruppe ausgewählt ist, die aus Maus, Ratte, Schaf, Hamster, Schwein, Hund, Affe, Kaninchen, Kalb, Pferd, Nematoden, Fliegen und Fisch besteht.

12. In-vitro-Verfahren des Identifizierens eines Polypeptids oder (a) einer Substanz / von Substanzen, die an einer Stoffwechselerkrankung beteiligt sind, welche aus Übergewicht, Adipositas, Essstörungen (Bulimia nervosa, Anorexia nervosa), Kachexie (Auszehrung) und Pankreasfunktionsstörung ausgewählt ist, welches folgende Schritte umfasst:
(a) Testen einer Zusammenstellung von Polypeptiden oder Substanzen für die Wechselwirkung mit dem Polypeptid oder dem Fusionsprotein wie definiert in Anspruch 1 unter Verwendung eines Auslesesystems; und
(b) Identifizieren von Polypeptiden oder Substanzen, die für eine Wechselwirkung in Schritt (a) positiv testen.

13. In-vitro-Verfahren nach Anspruch 12, das den weiteren Schritt des (c) ein- oder mehrmaligen Wiederholens der Schritte (a) und (b) umfasst, wobei das neu identifizierte Polypeptid das zuvor identifizierte Polypeptid als Köder für die Identifizierung eines weiteren wechselwirkenden Polypeptids ersetzt.

14. Verfahren nach Anspruch 12 oder 13, welches des Weiteren den Schritt des Identifizierens des Nukleinsäuremoleküls bzw. der Nukleinsäuremoleküle umfasst, welche (s) das eine oder mehrere wechselwirkende Peptide kodiert bzw. kodieren.

15. In-vitro-Verfahren des Identifizierens eines an der Regulierung von Körpergewicht in einem Säuger beteiligten Polypeptids, welches folgende Schritte umfasst:
(a) Kontaktieren einer Zusammenstellung von (Poly-) Peptiden mit dem Polypeptid oder dem Fusionsprotein wie definiert in Anspruch 1 unter Bedingungen, welche die Bindung der (Poly-)Peptide gestatten;
(b) Entfernen von (Poly-)Peptiden aus der Zusammenstellung von (Poly-)Peptiden, die in Schritt (a) nicht an das Polypeptid oder das Fusionsprotein wie definiert in Anspruch 1 gebunden haben; und
(c) Identifizieren von (Poly-)Peptiden, die an das Polypeptid oder das Fusionsprotein wie definiert in Anspruch 1 binden.

16. Verfahren nach Anspruch 15, wobei das Polypeptid oder das Fusionsprotein wie definiert in Anspruch 1 an einen festen Träger fixiert sind.

17. Verfahren nach Anspruch 16, wobei es sich bei dem festen Träger um ein Gelfiltrations- oder ein Affinitätschromatographiematerial handelt.

18. Verfahren nach einem der Ansprüche 15 bis 17, wobei die bindenden (Poly-)Peptide vor der Identifizierung in Schritt (c) freigesetzt werden.

19. Verfahren nach Anspruch 18, wobei die Freisetzung durch Elution bewirkt wird.

20. Verfahren nach einem der Ansprüche 15 bis 19, das des Weiteren den Schritt des Identifizierens des Nukleinsäuremoleküls bzw. der Nukleinsäuremoleküle umfasst, das bzw. die eines oder mehrere bindende (Poly-)Peptide kodiert bzw. kodieren.

21. Verfahren des Identifizierens einer Verbindung für die Erkennung und/oder Verifizierung oder für die Behandlung, Linderung und/oder Vorbeugung einer Stoffwechselerkrankung, ausgewählt aus Übergewicht, Adipositas, Essstörungen (Bulimia nervosa, Anorexia nervosa), Kachexie (Auszehrung) und Pankreasfunktionsstörung, wobei die Verbindung die Expression des Nukleinsäuremoleküls wie definiert in einem der Ansprüche 1 bis 7 beeinflusst, welches folgende Schritte umfasst:
(a) Kontaktieren einer Wirtszelle oder eines nicht-humanen Wirts, die bzw. der einen Expressionsvektor trägt, welcher das Nukleinsäuremolekül wie definiert in einem der Ansprüche 1 bis 7 aufweist, das funktional mit einem Auslesesystem mit einer Verbindung oder einer Zusammenstellung von Verbindungen verknüpft ist;
(b) Testen, ob das Kontaktieren zu einer Veränderung der Signalintensität führt, welche von dem Auslesesystem bereit gestellt wird; und gegebenenfalls
(c) Identifizieren einer Verbindung in der Zusammenstellung von Verbindungen, die eine Veränderung des Signals in Schritt (b) induziert;
wobei die Veränderung der Signalintensität mit einer Veränderung der Expression des Nukleinsäuremoleküls korreliert.

22. Verfahren des Identifizierens einer Verbindung für die Erkennung und/oder Verifizierung oder für die Behandlung, Linderung und/oder Vorbeugung einer Stoffwechselerkrankung, ausgewählt aus Übergewicht, Adipositas, Essstörungen (Bulimia nervosa, Anorexia nervosa), Kachexie (Auszehrung) und Pankreasfunktionsstörung, wobei die Verbindung die Aktivität eines Polypeptids wie definiert in Anspruch 1 beeinflusst, welches folgende Schritte umfasst:
(a) Kontaktieren einer Wirtszelle oder eines nicht-humanen Wirts, die bzw. der das Nukleinsäuremolekül wie definiert in einem der Ansprüche 1 bis 7 trägt, das funktional mit einem Auslesesystem verknüpft ist, und/oder das Polypeptid wie definiert in Anspruch 1 trägt, das mit einem Auslesesystem mit einer Verbindung oder einer Zusammenstellung von Verbindungen verknüpft ist;
(b) Testen, ob das Kontaktieren zu einer Veränderung der Signalintensität führt, welche von dem Auslesesystem bereit gestellt wird; und gegebenenfalls
(c) Identifizieren einer Verbindung in der Zusammenstellung von Verbindungen, die eine Veränderung des Signals in Schritt (b) induziert;
wobei die Veränderung des Signals mit einer Veränderung der Aktivität des (Poly-)Peptids korreliert.

23. Verfahren nach Anspruch 21 oder 22, wobei es sich bei der Wirtszelle um eine eukaryontische Wirtszelle handelt.

24. Verfahren nach Anspruch 23, wobei es sich bei der eukaryontischen Wirtszelle um eine Säugerwirtszelle handelt.

25. Verfahren nach Anspruch 21 oder 22, wobei es sich bei der Wirtszelle um ein Bakterium oder eine Hefe handelt.

26. Verfahren nach einem der Ansprüche 22 bis 25, wobei es sich bei der Veränderung der Signalintensität um eine Erhöhung der Signalintensität handelt.

27. Verfahren nach einem der Ansprüche 22 bis 25, wobei es sich bei der Veränderung der Signalintensität um eine Verringerung der Signalintensität handelt.

28. Nichttherapeutisches Verfahren des Beurteilens des Einflusses der Expression eines oder mehrere Polypeptide eines oder mehrerer Fusionsproteine wie definiert in Anspruch 1 in einem nicht-humanen Tier, welches folgende Schritte umfasst:
(a) Überexprimieren oder Unterexprimieren des Nukleinsäuremoleküls wie definiert in einem der Ansprüche 1 bis 7 oder des Nukleinsäuremoleküls, das von dem Verfahren nach Anspruch 14 oder 20 in dem Tier identifiziert wird, und
(b) Bestimmen, ob sich das Gewicht des Tieres erhöht, verringert hat, ob Stoffwechselveränderungen induziert wurden und/oder ob das Essverhalten verändert ist.

29. Verfahren des Testens auf einen Stoff, welcher die Wechselwirkung eines Polypeptids wie definiert in Anspruch 1 mit einem Bindungsziel/Bindemittel moduliert und welcher eine Stoffwechselerkrankung, ausgewählt aus Übergewicht, Adipositas, Essstörungen (Bulimia nervosa, Anorexia nervosa), Kachexie (Auszehrung) und Pankreasfunktionsstörung, erkennen, verifizieren, behandeln, lindern und/oder verhindern kann, welches folgende Schritte umfasst:
(a) Inkubieren eines Gemisches, umfassend
(aa) ein Polypeptid oder ein Fusionsprotein wie definiert in Anspruch 1
(ab) ein Bindungsziel/Bindemittel des (Poly-)Peptids oder Fusionsproteins oder eines Fragmentes davon; und
(ac) einen Kandidatenstoff
unter Bedingungen, bei denen das (Poly-)Peptid, Fusionsprotein oder Fragment davon spezifisch an das Bindungsziel/Bindemittel mit einer Bezugsaffinität bindet;
(b) Erkennen der Bindungsaffinität des (Poly-)Peptids, Fusionsproteins oder Fragmentes davon an das Bindungsziel zum Bestimmen einer Affinität, die von einem (Kandidaten-)Stoff beeinflusst wird; und
(c) Bestimmen eines Unterschiedes zwischen einer Affinität, die von einem (Kandidaten-)Stoff beeinflusst wird, und der Bezugsaffinität.

30. Kit für die Erkennung und/oder Verifizierung oder für die Behandlung, Linderung und/oder Vorbeugung einer Stoffwechselerkrankung, ausgewählt aus Übergewicht, Adipositas, Essstörungen (Bulimia nervosa, Anorexia nervosa), Kachexie (Auszehrung) und Pankreasfunktionsstörung, als diagnostische und/oder pharmazeutische Zusammensetzung, welches mindestens eines der Folgenden aufweist:
(a) ein Nukleinsäuremolekül wie definiert in einem der Ansprüche 1 bis 7;
(b) einen Vektor wie definiert in Anspruch 8;
(c) eine Wirtszelle oder einen nicht-humanen Wirtsorganismus, die mit dem Vektor wie definiert in Anspruch 8 transformiert sind;
(d) ein Polypeptid wie definiert in Anspruch 1;
(e) ein Fusionsprotein wie definiert in Anspruch 1;
(f) einen Antikörper oder ein Fragment oder Derivat davon oder ein Antiserum oder ein Aptamer wie definiert in Anspruch 1; und
(g) ein Antisense-Oligonukleotid, Ribozym, eine Hybridisierungssonde oder einen Amplifikationsprimer wie definiert in Anspruch 1.

## Revendications

1. Utilisation de
(i) une molécule d'acide nucléique codant pour un polypeptide, contribuant au moyen d'interactions protéine-protéine ainsi que d'interactions protéine-lipide à la stabilité des membranes et/ou contribuant à la fonction membranaire des organelles du fait de sa possession de fonctions de transport, de fonctions régulatrices d'autres protéines membranaires ou de fonctions modificatrices d'autres protéines (membranaires) et/ou d'autres fonctions,
(ii) un polypeptide codé par ledit acide nucléique,
(iii) une protéine de fusion comprenant ledit polypeptide,
(iv) un anticorps, un fragment ou un dérivé de celui-ci ou un aptamère reconnaissant spécifiquement ledit acide nucléique, ledit polypeptide ou ladite protéine de fusion.
(v) un oligonucléotide antisens, un ribozyme, une sonde d'hybridation ou une amorce d'amplification de ladite molécule d'acide nucléique,
pour la fabrication d'une composition de diagnostic et/ou pharmaceutique pour la détection et/ou la vérification ou pour le traitement, le soulagement et/ou la prévention d'un trouble métabolique choisi parmi l'obésité, l'adipose, les troubles de l'alimentation (la boulimie mentale, l'anorexie mentale), la cachexie (émaciation) et la dysfonction pancréatique, dans laquelle ladite molécule d'acide nucléique
(a) reste hybridée à 65°C dans une solution contenant du SSC 0,2 X et du SDS à 0,1 % à une molécule d'acide nucléique codant pour la séquence d'acides aminés de SEQ ID NO: 10, SEQ ID NO: 12 et/ou SEQ ID NO : 14 et/ou le brin complémentaire de celle-ci ;
(b) reste hybridée à 65°C dans une solution contenant du SSC 0,2 X et du SDS à 0,1 % à une molécule d'acide nucléique telle que représentée dans SEQ ID NO: 6, SEQ ID NO: 9, SEQ ID NO: 11 et/ou SEQ ID NO: 13 et/ou le brin complémentaire de celle-ci ;
(c) est dégénérée relativement à la molécule d'acide nucléique de (a) ou (b) ;
(d) code pour un polypeptide qui est identique à au moins 90 % à la séquence d'acides aminés telle que représentée dans l'une quelconque des SEQ ID NO : 10, 12 ou 14 ; ou
(e) a la séquence telle que présentée dans SEQ ID NO : 9, 11 ou 13.

2. Utilisation selon la revendication 1, dans laquelle ladite molécule d'acide nucléique est de l'ADN.

3. Utilisation selon la revendication 1 ou 2, dans laquelle ledit polypeptide est exprimé dans les mitochondries et/ou les peroxysomes.

4. Utilisation selon la revendication 3, dans laquelle ledit polypeptide participe à la conservation de ladite membrane.

5. Utilisation selon l'une quelconque des revendications 1 à 4, dans laquelle ledit polypeptide est une molécule de transport et/ou un régulateur d'une molécule de transport.

6. Utilisation selon l'une quelconque des revendications 1 à 5, dans laquelle ledit polypeptide est un polypeptide modificateur, qui contrôle la fonction d'un(e) ou plusieurs protéines/polypeptides.

7. Utilisation selon la revendication 6, dans laquelle ledit polypeptide modificateur contrôle la fonction de la protéine mitochondriale UCP2.

8. Utilisation selon l'une quelconque des revendications 1 à 7, dans laquelle ladite molécule d'acide nucléique est comprise sur un vecteur.

9. Utilisation d'un animal transgénique non humain comprenant une molécule d'acide nucléique codant pour un polypeptide, contribuant au moyen d'interactions protéine-protéine ainsi que d'interactions protéine-lipide à la stabilité des membranes et/ou contribuant à la fonction membranaire des organelles du fait de sa possession de fonctions de transport, de fonctions régulatrices d'autres protéines membranaires ou de fonctions modificatrices d'autres protéines (membranaires) et/ou d'autres fonctions, dans laquelle ladite molécule d'acide nucléique
(a) reste hybridée à 65°C dans une solution contenant du SSC 0,2 X et du SDS à 0,1 % à une molécule d'acide nucléique codant pour la séquence d'acides aminés de SEQ ID NO : 8, SEQ ID NO : 54, SEQ ID NO : 10, SEQ ID NO : 12, SEQ ID NO : 14 et/ou SEQ ID NO : 52 et/ou le brin complémentaire de celle-ci ;
(b) reste hybridée à 65°C dans une solution contenant du SSC 0,2 X et du SDS à 0,1 % à une molécule d'acide nucléique telle que représentée dans SEQ ID NO : 6, 7 ou 53, SEQ ID NO : 9, SEQ ID NO : 11, SEQ ID NO : 13 et/ou SEQ ID NO : 51 et/ou le brin complémentaire de celle-ci ;
(c) est dégénérée relativement à la molécule d'acide nucléique de (a) ou (b) ;
(d) code pour un polypeptide qui est identique à au moins 90 % à SEQ ID NO: 8;
(e) code pour un polypeptide qui est identique à au moins 90 % à SEQ ID NO : 54;
(f) code pour un polypeptide qui est identique à au moins 90 % à la séquence d'acides aminés telle que représentée dans l'une quelconque des SEQ ID NO : 10, 12, 14 ou 52 ; ou
(g) a la séquence telle que représentée dans SEQ ID NO : 9, 11, 13 ou 51 ;
exprimant un polypeptide codé par ladite molécule d'acide nucléique ou une protéine de fusion comprenant ledit polypeptide ; ou transfecté avec un vecteur comprenant ladite molécule d'acide nucléique en tant qu'un modèle de recherche pour l'obésité, l'adipose, les troubles de l'alimentation, l'émaciation et/ou d'autres troubles du poids corporel/de la masse corporelle.

10. Utilisation selon la revendication 9, dans laquelle la molécule d'acide nucléique selon l'une quelconque des revendications 1 à 7 est silencieuse et/ou mutée, en tant qu'un modèle de recherche pour l'obésité, l'adipose, les troubles de l'alimentation, l'émaciation et/ou d'autres troubles du poids corporel/de la masse corporelle.

11. Utilisation selon la revendication 9 ou 10, dans laquelle l'animal est choisi dans le groupe constitué par une souris, un rat, un mouton, un hamster, un porc, un chien, un singe, un lapin, un veau, un cheval, des nématodes, une mouche et un poisson.

12. Procédé *in vitro* d'identification d'un polypeptide ou d'une (de) substance(s) impliqué (e) (s) dans un trouble métabolique choisi parmi l'obésité, l'adipose, les troubles de l'alimentation (la boulimie mentale, l'anorexie mentale), la cachexie (émaciation) et la dysfonction pancréatique, le procédé comprenant les étapes consistant à:
(a) tester une collection de polypeptides ou de substances pour l'interaction avec le polypeptide ou la protéine de fusion selon la revendication 1 en utilisant un système de lecture ; et
(b) identifier les polypeptides ou substances qui sont positives au test d'interaction dans l'étape (a) .

13. Procédé *in vitro* selon la revendication 12, comprenant l'étape supplémentaire consistant à :
(c) répéter les étapes (a) et (b) avec les polypeptides identifiés une ou plusieurs fois dans lequel le polypeptide nouvellement identifié remplace le polypeptide préalablement identifié en tant qu'un appât pour l'identification d'un autre polypeptide d'interaction.

14. Procédé selon la revendication 12 ou 13, comprenant en outre l'étape consistant à identifier la (les) molécule(s) d'acide nucléique codant pour le ou les (poly)peptides d'interaction.

15. Procédé in vitro d'identification d'un polypeptide impliqué dans la régulation de la masse corporelle chez un mammifère, le procédé comprenant les étapes consistant à :
(a) mettre en contact une collection de (poly) peptides avec le polypeptide ou la protéine de fusion selon la revendication 1 dans des conditions qui permettent la liaison desdits (poly)peptides ;
(b) éliminer les (poly)peptides de ladite collection de (poly)peptides qui ne se lient pas au dit polypeptide ou à ladite protéine de fusion selon la revendication 1 dans l'étape (a) ; et
(c) identifier les (poly)peptides qui se lient au dit polypeptide ou à ladite protéine de fusion selon la revendication 1.

16. Procédé selon la revendication 15, dans lequel ledit polypeptide ou ladite protéine de fusion selon la revendication 1 est fixé(e) à un support solide.

17. Procédé selon la revendication 16, dans lequel ledit support solide est un matériau de filtration sur gel ou de chromatographie d'affinité.

18. Procédé selon l'une quelconque des revendications 15 à 17, dans lequel, avant ladite identification dans l'étape (c), lesdits (poly)peptides de liaison sont libérés.

19. Procédé selon la revendication 18, dans lequel ladite libération est réalisée par élution.

20. Procédé selon l'une quelconque des revendications 15 à 19, comprenant en outre l'étape consistant à identifier la (les) molécule(s) d'acide nucléique codant pour le ou les (poly)peptides de liaison.

21. Procédé d'identification d'un composé pour la détection et/ou la vérification ou pour le traitement, le soulagement et/ou la prévention d'un trouble métabolique choisi parmi l'obésité, l'adipose, les troubles de l'alimentation (la boulimie mentale, l'anorexie mentale), la cachexie (émaciation) et la dysfonction pancréatique, dans lequel ledit composé influence l'expression de la molécule d'acide nucléique selon l'une quelconque des revendications 1 à 7, le procédé comprenant les étapes consistant à:
(a) mettre en contact une cellule hôte ou un hôte non humain portant un vecteur d'expression comprenant la molécule d'acide nucléique selon l'une quelconque des revendications 1 à 7 liée de manière fonctionnelle à un système de lecture avec un composé ou une collection de composés ;
(b) tester si ladite mise en contact résulte en une modification de l'intensité du signal fournie par ledit système de lecture ; et, facultativement,
(c) identifier un composé à l'intérieur de ladite collection de composés qui induit une modification du signal dans l'étape (b) ;
dans lequel ladite modification de l'intensité du signal est corrélée à une modification de l'expression de ladite molécule d'acide nucléique.

22. Procédé d'identification d'un composé pour la détection et/ou la vérification ou pour le traitement, le soulagement et/ou la prévention d'un trouble métabolique choisi parmi l'obésité, l'adipose, les troubles de l'alimentation (la boulimie mentale, l'anorexie mentale), la cachexie (émaciation) et la dysfonction pancréatique, dans lequel ledit composé influence l'activité d'un polypeptide selon la revendication 1, le procédé comprenant les étapes consistant à:
(a) mettre en contact une cellule hôte ou un hôte non humain portant un vecteur d'expression comprenant la molécule d'acide nucléique selon l'une quelconque des revendications 1 à 7 liée de manière fonctionnelle à un système de lecture et/ou portant le polypeptide selon la revendication 1 lié à un système de lecture avec un composé ou une collection de composés ;
(b) tester si ladite mise en contact résulte en une modification de l'intensité du signal fournie par ledit système de lecture ; et, facultativement,
(c) identifier un composé à l'intérieur de ladite collection de composés qui induit une modification de signal dans l'étape (b);
dans lequel ladite modification du signal est corrélée à une modification de l'activité dudit (poly)peptide.

23. Procédé selon la revendication 21 ou 22, dans lequel ladite cellule hôte est une cellule hôte eucaryote.

24. Procédé selon la revendication 23, dans lequel ladite cellule hôte eucaryote est une cellule hôte de mammifère.

25. Procédé selon la revendication 21 ou 22, dans lequel ladite cellule hôte est une bactérie ou une levure.

26. Procédé selon l'une quelconque des revendications 22 à 25, dans lequel ladite modification de l'intensité du signal est une augmentation de l'intensité du signal.

27. Procédé selon l'une quelconque des revendications 22 à 25, dans lequel ladite modification de l'intensité du signal est une diminution de l'intensité du signal.

28. Procédé non thérapeutique d'évaluation de l'impact de l'expression d'un ou de plusieurs polypeptides ou d'une ou de plusieurs protéines de fusion selon la revendication 1 dans un animal non humain, le procédé comprenant les étapes consistant à:
(a) surexprimer ou sous-exprimer la molécule d'acide nucléique selon l'une quelconque de revendications 1 à 7 ou la molécule d'acide nucléique identifiée par le procédé selon la revendication 14 ou 20 dans ledit animal ; et
(b) déterminer si le poids dudit animal a augmenté, diminué, si des modifications métaboliques sont induites et/ou si le comportement alimentaire est modifié.

29. Procédé de criblage d'un agent qui module l'interaction d'un polypeptide selon la revendication 1 capable de détecter, vérifier, traiter, soulager et/ou prévenir un trouble métabolique choisi parmi l'obésité, l'adipose, les troubles de l'alimentation (la boulimie mentale, l'anorexie mentale), la cachexie (émaciation) et la dysfonction pancréatique avec une cible/agent de liaison, le procédé comprenant les étapes consistant à:
(a) faire incuber un mélange comprenant
(aa) un polypeptide ou une protéine de fusion selon la revendication 1 ;
(ab) une cible/agent de liaison dudit (poly) peptide ou de ladite protéine de fusion ou dudit fragment de ceux-ci ; et
(ac) un agent candidat
dans des conditions moyennant lesquelles ledit (poly) peptide, ladite protéine de fusion ou ledit fragment de ceux-ci se lie à ladite cible/au dit agent de liaison à une affinité de référence ;
(b) détecter l'affinité de liaison dudit (poly) peptide, de ladite protéine de fusion ou dudit fragment de ceux-ci à la dite cible de liaison pour déterminer une affinité modifiée par un agent (candidat); et
(c) déterminer une différence entre une affinité modifiée par un agent (candidat) et l'affinité de référence.

30. Kit pour la détection et/ou la vérification ou pour le traitement, le soulagement et/ou la prévention d'un trouble métabolique choisi parmi l'obésité, l'adipose, les troubles de l'alimentation (la boulimie mentale, l'anorexie mentale), la cachexie (émaciation) et la dysfonction pancréatique, sous la forme d'une composition de diagnostic et/ou pharmaceutique, comprenant au moins un de
(a) une molécule d'acide nucléique selon l'une quelconque des revendications 1 à 7 ;
(b) un vecteur selon la revendication 8 ;
(c) une cellule hôte ou un organisme hôte non humain transformé(e) avec le vecteur selon la revendication 8 ;
(d) un polypeptide selon la revendication 1 ;
(e) une protéine de fusion selon la revendication 1 ;
(f) an anticorps ou un fragment ou un dérivé de celui-ci ou un antisérum, ou un aptamère selon la revendication 1 ; et
(g) un oligonucléotide antisens, un ribozyme, une sonde d'hybridation ou une amorce,d'amplification selon la revendication 1.


### SEQUENCE LISTING

<110> DeveloGen AG
<120> Modifier of organelle metabolism
<130> 26407PWO
<140>
   <141>
<150> 00 125 693.2
   <151> 2000-11-23
<160> 68
<170> PatentIn Ver. 2.1
<210> 1
   <211> 24
   <212> DNA
   <213> Artificial Sequence
<220>
   <223> Description of Artificial Sequence: synthetic DNA
<400> 1
   ctaaacaaac aattccaaac atag 24
<210> 2
   <211> 24
   <212> DNA
   <213> Artificial Sequence
<220>
   <223> Description of Artificial Sequence: synthetic DNA
<400> 2
   aaaagacata gaaaatacga tagt 24
<210> 3
   <211> 1248
   <212> DNA
   <213> Drosophila melanogaster
<400> 3
   aagtgttact atctaaacac atttcaaaca attcttaaca aacaattcca aacatacaat 60
   tccacttacc acttaccgac caaattacga gtttacaatg gacaaagctg aacgcgacta 120
   ctggcatctt cgatccttgg aaatcgaaga ggagccgcga tttccgccaa caaacgtcgc 180
   tgatccacta accgcacgca atctgttcca gctctacgtc aacaccttca ttggagccaa 240
   tctggccgag tcgtgtgttt tcccattgga cgtggccaag acccggatgc aggtagatgg 300
   cgagcaggcc aagaagacgg gtaaagcgat gccaactttc cgtgcaactc ttaccaacat 360
   gatccgagtg gagggattca agtcgctcta cgccggcttc tcggcaatgg tgacccgaaa 420
   ctttatcttc aactcgttac gtgttgttct ctacgacgtt ttccggcgcc cttttctcta 480
   ccagaacgaa cggaacgagg aagtgctcaa gatctacatg gcgctgggat gcagcttcac 540
   cgcaggctgc attgcccagg cactggccaa tccctttgac atcgtcaagg tgcgaatgca 600
   gacggaagga cgccgccgcc agctgggcta tgatgtgcgg gtgaacagca tggtgcaggc 660
   cttcgtggac atctaccgcc gtggcggact gcccagtatg tggaagggtg tagggcccag 720
   ctgcatgcgt gcctgcctga tgacgaccgg cgatgtgggc agttacgata tcagtaagcg 780
   caccttcaag cgcctgctgg acttggagga aggcctgcca ctgcgtttcg tgtcttccat 840
   gtgcgccgga ctaacggcat ccgtgctcag cacgccggcg aacgtgatca agtcgcggat 900
   gatgaaccag ccggtgaacg agagcggcaa gaatctgtac tacaagaact ccctcgactg 960
   cattaggaag ctggtcaggg aggagggtgt cctcacgttg tataagggcc tcatgcccac 1020
   ttggtttcgc ctgggaccgt tctcagtgct cttttggctg tccgtcgagc agctgcgtca 1080
   gtggaaaggc cagagtggat tttaggagca aactatcaat cttactatcg tattttgtat 1140
   gtcttttaac acgcaataaa aagggtgcaa gtcaaaccat ctattataca tattataaat 1200
   ataactttaa tcccaaaaaa aaaaaaaaaa actcgtgccg aattcgat              1248
<210> 4
   <211> 1008
   <212> DNA
   <213> Drosophila melanogaster
<220>
   <221> CDS
   <222> (1)..(1005)
<400> 4
   atg gac aaa gct gaa cgc gac tac tgg cat ctt cga tcc ttg gaa atc    48
   Met Asp Lys Ala Glu Arg Asp Tyr Trp His Leu Arg Ser Leu Glu Ile
    1               5                  10                  15
   gaa gag gag ccg cga ttt ccg cca aca aac gtc gct gat cca cta acc    96
   Glu Glu Glu Pro Arg Phe Pro Pro Thr Asn Val Ala Asp Pro Leu Thr
               20                  25                  30
   gca cgc aat ctg ttc cag ctc tac gtc aac acc ttc att gga gcc aat   144
   Ala Arg Asn Leu Phe Gln Leu Tyr Val Asn Thr Phe Ile Gly Ala Asn
           35                  40                  45
   ctg gcc gag tcg tgt gtt ttc cca ttg gac gtg gcc aag acc cgg atg   192
   Leu Ala Glu Ser Cys Val Phe Pro Leu Asp Val Ala Lys Thr Arg Met
       50                  55                  60
   cag gta gat ggc gag cag gcc aag aag acg ggt aaa gcg atg cca act   240
   Gln Val Asp Gly Glu Gln Ala Lys Lys Thr Gly Lys Ala Met Pro Thr
   65                  70                  75                  80
   ttc cgt gca act ctt acc aac atg atc cga gtg gag gga ttc aag tcg   288
   Phe Arg Ala Thr Leu Thr Asn Met Ile Arg Val Glu Gly Phe Lys Ser
                   85                  90                  95
   ctc tac gcc ggc ttc tcg gca atg gtg acc cga aac ttt atc ttc aac   336
   Leu Tyr Ala Gly Phe Ser Ala Met Val Thr Arg Asn Phe Ile Phe Asn
               100                 105                 110
   tcg tta cgt gtt gtt ctc tac gac gtt ttc cgg cgc cct ttt ctc tac   384
   Ser Leu Arg Val Val Leu Tyr Asp Val Phe Arg Arg Pro Phe Leu Tyr
           115                 120                 125
   cag aac gaa cgg aac gag gaa gtg ctc aag atc tac atg gcg ctg gga   432
   Gln Asn Glu Arg Asn Glu Glu Val Leu Lys Ile Tyr Met Ala Leu Gly
       130                 135                 140
   tgc agc ttc acc gca ggc tgc att gcc cag gca ctg gcc aat ccc ttt   480
   Cys Ser Phe Thr Ala Gly Cys Ile Ala Gln Ala Leu Ala Asn Pro Phe
   145                 150                 155                 160
   gac atc gtc aag gtg cga atg cag acg gaa gga cgc cgc cgc cag ctg   528
   Asp Ile Val Lys Val Arg Met Gln Thr Glu Gly Arg Arg Arg Gln Leu
                   165                 170                 175
   ggc tat gat gtg cgg gtg aac agc atg gtg cag gcc ttc gtg gac atc   576
   Gly Tyr Asp Val Arg Val Asn Ser Met Val Gln Ala Phe Val Asp Ile
               180                 185                 190
   tac cgc cgt ggc gga ctg ccc agt atg tgg aag ggt gta ggg ccc agc      624
   Tyr Arg Arg Gly Gly Leu Pro Ser Met Trp Lys Gly Val Gly Pro Ser
           195                 200                 205
   tgc atg cgt gcc tgc ctg atg acg acc ggc gat gtg ggc agt tac gat      672
   Cys Met Arg Ala Cys Leu Met Thr Thr Gly Asp Val Gly Ser Tyr Asp
       210                 215                 220
   atc agt aag cgc acc ttc aag cgc ctg ctg gac ttg gag gaa ggc ctg      720
   Ile Ser Lys Arg Thr Phe Lys Arg Leu Leu Asp Leu Glu Glu Gly Leu
   225                 230                 235                 240
   cca ctg cgt ttc gtg tct tcc atg tgc gcc gga cta acg gca tcc gtg      768
   Pro Leu Arg Phe Val Ser Ser Met Cys Ala Gly Leu Thr Ala Ser Val
                   245                 250                 255
   ctc agc acg ccg gcg aac gtg atc aag tcg cgg atg atg aac cag ccg      816
   Leu Ser Thr Pro Ala Asn Val Ile Lys Ser Arg Met Met Asn Gln Pro
               260                 265                 270
   gtg aac gag agc ggc aag aat ctg tac tac aag aac tcc ctc gac tgc      864
   Val Asn Glu Ser Gly Lys Asn Leu Tyr Tyr Lys Asn Ser Leu Asp Cys
           275                 280                 285
   att agg aag ctg gtc agg gag gag ggt gtc ctc acg ttg tat aag ggc      912
   Ile Arg Lys Leu Val Arg Glu Glu Gly Val Leu Thr Leu Tyr Lys Gly
       290                 295                 300
   ctc atg ccc act tgg ttt cgc ctg gga ccg ttc tca gtg ctc ttt tgg      960
   Leu Met Pro Thr Trp Phe Arg Leu Gly Pro Phe Ser Val Leu Phe Trp
   305                 310                 315                 320
   ctg tcc gtc gag cag ctg cgt cag tgg aaa ggc cag agt gga ttt tag      1008
   Leu Ser Val Glu Gln Leu Arg Gln Trp Lys Gly Gln Ser Gly Phe
                   325                 330                 335
<210> 5
   <211> 335
   <212> PRT
   <213> Drosophila melanogaster
<400> 5
   Met Asp Lys Ala Glu Arg Asp Tyr Trp His Leu Arg Ser Leu Glu Ile
    1               5                  10                  15
   Glu Glu Glu Pro Arg Phe Pro Pro Thr Asn Val Ala Asp Pro Leu Thr
               20                  25                  30
   Ala Arg Asn Leu Phe Gln Leu Tyr Val Asn Thr Phe Ile Gly Ala Asn
           35                  40                  45
   Leu Ala Glu Ser Cys Val Phe Pro Leu Asp Val Ala Lys Thr Arg Met
       50                  55                  60
   Gln Val Asp Gly Glu Gln Ala Lys Lys Thr Gly Lys Ala Met Pro Thr
   65                  70                  75                  80
   Phe Arg Ala Thr Leu Thr Asn Met Ile Arg Val Glu Gly Phe Lys Ser
                   85                  90                  95
   Leu Tyr Ala Gly Phe Ser Ala Met Val Thr Arg Asn Phe Ile Phe Asn
               100                 105                 110
   Ser Leu Arg Val Val Leu Tyr Asp Val Phe Arg Arg Pro Phe Leu Tyr
           115                 120                 125
   Gln Asn Glu Arg Asn Glu Glu Val Leu Lys Ile Tyr Met Ala Leu Gly
       130                 135                 140
   Cys Ser Phe Thr Ala Gly Cys Ile Ala Gln Ala Leu Ala Asn Pro Phe
   145                 150                 155                 160
   Asp Ile Val Lys Val Arg Met Gln Thr Glu Gly Arg Arg Arg Gln Leu
                   165                 170                 175
   Gly Tyr Asp Val Arg Val Asn Ser Met Val Gln Ala Phe Val Asp Ile
               180                 185                 190
   Tyr Arg Arg Gly Gly Leu Pro Ser Met Trp Lys Gly Val Gly Pro Ser
           195                 200                 205
   Cys Met Arg Ala Cys Leu Met Thr Thr Gly Asp Val Gly Ser Tyr Asp
       210                 215                 220
   Ile Ser Lys Arg Thr Phe Lys Arg Leu Leu Asp Leu Glu Glu Gly Leu
   225                 230                 235                 240
   Pro Leu Arg Phe Val Ser Ser Met Cys Ala Gly Leu Thr Ala Ser Val
                   245                 250                 255
   Leu Ser Thr Pro Ala Asn Val Ile Lys Ser Arg Met Met Asn Gln Pro
               260                 265                 270
   Val Asn Glu Ser Gly Lys Asn Leu Tyr Tyr Lys Asn Ser Leu Asp Cys
           275                 280                 285
   Ile Arg Lys Leu Val Arg Glu Glu Gly Val Leu Thr Leu Tyr Lys Gly
       290                 295                 300
   Leu Met Pro Thr Trp Phe Arg Leu Gly Pro Phe Ser Val Leu Phe Trp
   305                 310                 315                 320
   Leu Ser Val Glu Gln Leu Arg Gln Trp Lys Gly Gln Ser Gly Phe
                   325                 330                 335
<210> 6
   <211> 2953
   <212> DNA
   <213> Drosophila melanogaster
<400> 6
   ctgctgggaa ttcggcacga gaagtcgatt agtcgttttt cgttggagag cttgctgttc 60
   gcatttatcc gcctgctcgc gcgtagaaaa gttgttactt aatgttcatt gctgccacac 120
   gtgcttgatt attaattgca gtgcgctaat cagtcgcttg tcagctgagc agaccacaaa 180
   aaacgaaggc aattatttag gctgcccttt cagttcggca ttacataaga aacgaatggc 240
   caaacgaaaa taattttgaa acaatttcgg agttaatatt gcatcagcaa ttctgaacag 300
   cagatacaca aaaagggcag caatttggcc aacaaagtct tcataaacga caacaaacga 360
   gtgaaaatat gagctgggca acaacaaagc aaaggcacaa atgtttcaac tgtagcagcg 420
   gcagcaacaa aaacaaccgg agcagcggca gcagcagcaa caacaacaac tgtgcgccta 480
   gcaacaagat aatttcgacg agcgatttcc tttgatttgc gcccatctgt gtcgaaagca 540
   gccgaaaagc caaagagtgt aaatagcacg tagttttagt acaattcact gtagcaaaag 600
   tgagtttcaa ccggggcagc caataagcct gcgatagcaa caataataca gaacgagcgc 660
   aaaagaaatc accagagatt gcacaatatg aatccgatca aggcacagtc aacgggcagt 720
   cccaagaaat tcaacgtatt cgcacacgtc aagtacgagc atttggttgc cggagtatcc 780
   ggcggagtgg tgtccacact cattctacat cccctggatt tgatcaagat tcgattcgca 840
   gttaacgatg gccggacagc tacggtgccg caataccggg gactgagcag cgccttcacc 900
   acgattttcc ggcaagaggg cttccgcgga ctctacaaag gcgtcacccc caatgtctgg 960
   ggatcgggct cctcttgggg cctgtacttc atgttctaca acaccattaa gacatttatc 1020
   caaggaggaa acacgaccat gccattgggc cccacaatga acatgcttgc agctgctgag 1080
   tcgggaattc tcaccctgct gctgaccaac cccatctggg tggtgaagac gcgtctctgc 1140
   ctgcagtgcg atgccgcgag tagtgccgag tacaggggca tgatccacgc cttgggccag 1200
   atatacaagg aggagggaat ccgtggcctg taccgcggct ttgttcccgg catgttgggc 1260
   gtctcccacg gagccatcca gttcatgacc tacgaggagc tgaagaacgc ctacaacgaa 1320
   tatcgcaaac tgcccatcga cacgaagctg gccaccaccg agtacttggc cttcgcggct 1380
   gtctccaagc tgatcgcagc ggcggccacc tacccgtacc aggtggtccg ggcacggctg 1440
   caggaccacc atcaccgata caacggcacc tgggactgca tcaaacagac ttggaggtac 1500
   gagcgcatgc gaggtttcta taagggcctg gtgccctacc tggtccacgt cacgcccaac 1560
   atctgcatgg tcatgctgat ctgggagaag ctgaccagct agatggagta ttagtactag 1620
   atcatcgaat ctggaatctg acagagaatt taagctaagc acctagaata cacgaatctt 1680
   tctcgtttcc tccgatgtgc agctaacagc agaaaatgac aaacttattc tgtattattg 1740
   ttgtaactcg atttcggttt agccctagca ccttacttta gccttaagtg tattccatat 1800
   ctagtttatt gtacatcctt cactcccaac ttcgaaagta actgatgtga tcggggcgtc 1860
   gtcaaaatag cccctgccca tgaaatgtaa tttcaaaaac gcatttcttg cactcttcat 1920
   caaaacaaac acatatttag ttcttagttt agttaaatta tttattctat aacggcttgg 1980
   aattgtgtaa gcacaaagaa aaaccagtta ctgtttagtt gatacaaaat ttcgttttgg 2040
   caaaaagatt cttcatagtt tttgaaaatg gtttataatt ttaaggataa accttgacat 2100
   ttatgtaaat aagcataaac gcattgtgaa accaacttag tcctacagtg gaacaatctt 2160
   attgaaatca aagaaaagat aagaaatgcg aaattactta cccatattaa aaggccacga 2220
   tggcagacct aaatgaaaac caagaaacaa actaaagcaa acactgtcag cgcgtccagc 2280
   ctgcgtcttt tcatttgtcc aagggctcat ggcagcttga gtaagtagtc cttgctcctt 2340
   ccgctcccta agtcttcgat caccccctta attgtgcaga atctctttgc taatctgaaa 2400
   cagccaatgc gcccttactg taagtagatg tagctttcag ttctcagtct ctaaccgcat 2460
   tgtgtttggt tctctttgca ggtttgaggg ctacagaggc ttctacaagg ggctgaaggc 2520
   gagtttaacc cgagtagtgc ctgcctgcat ggtcaccttt ctggtgtacg agaacgtctc 2580
   gcatttcctg ctcgccaggc ggaagcgaat tgagactaaa gaggatgcgt cggacgtgtg 2640
   attttccttt ggttggattc cttttaggct tctaagatac atatatcccc tcacgacatt 2700
   tccatatgtc tctatagtca gcgggcagtt gcaatcgtgc tgagcatgcg cagctgcttt 2760
   tagtttaagt ttagttagtt gttgcgaaga cttatttggt cttttcgtag taagtaaagt 2820
   gattgttgtt cctaagtgtg gatcagaatg gatttgtgat tgaattcaga cgaaagtgtt 2880
   acaatatacc tgtgaaatat aagtccatag cctcactctt gcaaataaac caaatctttg 2940
   tgagcccaaa aaa                                                    2953
<210> 7
   <211> 915
   <212> DNA
   <213> Drosophila melanogaster
<220>
   <221> CDS
   <222> (1)..(912)
<400> 7
   atg aat ccg atc aag gca cag tca acg ggc agt ccc aag aaa ttc aac     48
   Met Asn Pro Ile Lys Ala Gln Ser Thr Gly Ser Pro Lys Lys Phe Asn
   1               5                   10                  15
   gta ttc gca cac gtc aag tac gag cat ttg gtt gcc gga gta tcc ggc     96
   Val Phe Ala His Val Lys Tyr Glu His Leu Val Ala Gly Val Ser Gly
               20                  25                  30
   gga gtg gtg tcc aca ctc att cta cat ccc ctg gat ttg atc aag att     144
   Gly Val Val Ser Thr Leu Ile Leu His Pro Leu Asp Leu Ile Lys Ile
               35                  40                  45
   cga ttc gca gtt aac gat ggc cgg aca gct acg gtg ccg caa tac cgg    192
   Arg Phe Ala Val Asn Asp Gly Arg Thr Ala Thr Val Pro Gln Tyr Arg
        50                  55                  60
   gga ctg agc agc gcc ttc acc acg att ttc cgg caa gag ggc ttc cgc    240
   Gly Leu Ser Ser Ala Phe Thr Thr Ile Phe Arg Gln Glu Gly Phe Arg
    65                  70                  75                  80
   gga ctc tac aaa ggc gtc acc ccc aat gtc tgg gga tcg ggc tcc tct    288
   Gly Leu Tyr Lys Gly Val Thr Pro Asn Val Trp Gly Ser Gly Ser Ser
                    85                  90                  95
   tgg ggc ctg tac ttc atg ttc tac aac acc att aag aca ttt atc caa    336
   Trp Gly Leu Tyr Phe Met Phe Tyr Asn Thr Ile Lys Thr Phe Ile Gln
               100                 105                 110
   gga gga aac acg acc atg cca ttg ggc ccc aca atg aac atg ctt gca    384
   Gly Gly Asn Thr Thr Met Pro Leu Gly Pro Thr Met Asn Met Leu Ala
           115                 120                 125
   gct gct gag tcg gga att ctc acc ctg ctg ctg acc aac ccc atc tgg    432
   Ala Ala Glu Ser Gly Ile Leu Thr Leu Leu Leu Thr Asn Pro Ile Trp
       130                 135                 140
   gtg gtg aag acg cgt ctc tgc ctg cag tgc gat gcc gcg agt agt gcc    480
   Val Val Lys Thr Arg Leu Cys Leu Gln Cys Asp Ala Ala Ser Ser Ala
   145                 150                 155                 160
   gag tac agg ggc atg atc cac gcc ttg ggc cag ata tac aag gag gag    528
   Glu Tyr Arg Gly Met Ile His Ala Leu Gly Gln Ile Tyr Lys Glu Glu
                   165                 170                 175
   gga atc cgt ggc ctg tac cgc ggc ttt gtt ccc ggc atg ttg ggc gtc    576
   Gly Ile Arg Gly Leu Tyr Arg Gly Phe Val Pro Gly Met Leu Gly Val
               180                 185                 190
   tcc cac gga gcc atc cag ttc atg acc tac gag gag ctg aag aac gcc    624
   Ser His Gly Ala Ile Gln Phe Met Thr Tyr Glu Glu Leu Lys Asn Ala
           195                 200                 205
   tac aac gaa tat cgc aaa ctg ccc atc gac acg aag ctg gcc acc acc    672
   Tyr Asn Glu Tyr Arg Lys Leu Pro Ile Asp Thr Lys Leu Ala Thr Thr
       210                 215                 220
   gag tac ttg gcc ttc gcg gct gtc tcc aag ctg atc gca gcg gcg gcc    720
   Glu Tyr Leu Ala Phe Ala Ala Val Ser Lys Leu Ile Ala Ala Ala Ala
   225                 230                 235                 240
   acc tac ccg tac cag gtg gtc cgg gca cgg ctg cag gac cac cat cac    768
   Thr Tyr Pro Tyr Gln Val Val Arg Ala Arg Leu Gln Asp His His His
                   245                 250                 255
   cga tac aac ggc acc tgg gac tgc atc aaa cag act tgg agg tac gag    816
   Arg Tyr Asn Gly Thr Trp Asp Cys Ile Lys Gln Thr Trp Arg Tyr Glu
               260                 265                 270
   cgc atg cga ggt ttc tat aag ggc ctg gtg ccc tac ctg gtc cac gtc    864
   Arg Met Arg Gly Phe Tyr Lys Gly Leu Val Pro Tyr Leu Val His Val
           275                 280                 285
   acg ccc aac atc tgc atg gtc atg ctg atc tgg gag aag ctg acc agc    912
   Thr Pro Asn Ile Cys Met Val Met Leu Ile Trp Glu Lys Leu Thr Ser
       290                 295                 300
   tag                                                              915
<210> 8
   <211> 304
   <212> PRT
   <213> Drosophila melanogaster
<400> 8
   Met Asn Pro Ile Lys Ala Gln Ser Thr Gly Ser Pro Lys Lys Phe Asn
   1               5                   10                  15
   Val Phe Ala His Val Lys Tyr Glu His Leu Val Ala Gly Val Ser Gly
               20                  25                  30
   Gly Val Val Ser Thr Leu Ile Leu His Pro Leu Asp Leu Ile Lys Ile
           35                  40                  45
   Arg Phe Ala Val Asn Asp Gly Arg Thr Ala Thr Val Pro Gln Tyr Arg
       50                  55                  60
   Gly Leu Ser Ser Ala Phe Thr Thr Ile Phe Arg Gln Glu Gly Phe Arg
   65                  70                  75                  80
   Gly Leu Tyr Lys Gly Val Thr Pro Asn Val Trp Gly Ser Gly Ser Ser
                   85                  90                  95
   Trp Gly Leu Tyr Phe Met Phe Tyr Asn Thr Ile Lys Thr Phe Ile Gln
               100                 105                 110
   Gly Gly Asn Thr Thr Met Pro Leu Gly Pro Thr Met Asn Met Leu Ala
           115                 120                 125
   Ala Ala Glu Ser Gly Ile Leu Thr Leu Leu Leu Thr Asn Pro Ile Trp
       130                 135                 140
   Val Val Lys Thr Arg Leu Cys Leu Gln Cys Asp Ala Ala Ser Ser Ala
   145                 150                 155                 160
   Glu Tyr Arg Gly Met Ile His Ala Leu Gly Gln Ile Tyr Lys Glu Glu
                   165                 170                 175
   Gly Ile Arg Gly Leu Tyr Arg Gly Phe Val Pro Gly Met Leu Gly Val
               180                 185                 190
   Ser His Gly Ala Ile Gln Phe Met Thr Tyr Glu Glu Leu Lys Asn Ala
           195                 200                 205
   Tyr Asn Glu Tyr Arg Lys Leu Pro Ile Asp Thr Lys Leu Ala Thr Thr
       210                 215                 220
   Glu Tyr Leu Ala Phe Ala Ala Val Ser Lys Leu Ile Ala Ala Ala Ala
   225                 230                 235                 240
   Thr Tyr Pro Tyr Gln Val Val Arg Ala Arg Leu Gln Asp His His His
                   245                 250                 255
   Arg Tyr Asn Gly Thr Trp Asp Cys Ile Lys Gln Thr Trp Arg Tyr Glu
               260                 265                 270
   Arg Met Arg Gly Phe Tyr Lys Gly Leu Val Pro Tyr Leu Val His Val
           275                 280                 285
   Thr Pro Asn Ile Cys Met Val Met Leu Ile Trp Glu Lys Leu Thr Ser
       290                 295                 300
<210> 9
   <211> 948
   <212> DNA
   <213> Homo sapiens
<220>
   <221> CDS
   <222> (1) .. (945)
<400> 9
   atg acg ggc cag ggc cag tcg gcg tcc ggg tcg tcg gcg tgg agc acg    48
   Met Thr Gly Gln Gly Gln Ser Ala Ser Gly Ser Ser Ala Trp Ser Thr
    1               5                  10                  15
   gta ttc cgc cac gtc cgg tat gag aac ctg ata gcg ggc gtg agc ggc    96
   Val Phe Arg His Val Arg Tyr Glu Asn Leu Ile Ala Gly Val Ser Gly
               20                  25                  30
   ggc gtc tta tcc aac ctt gcg ctg cat ccg ctc gac ctc gtg aag atc   144
   Gly Val Leu Ser Asn Leu Ala Leu His Pro Leu Asp Leu Val Lys Ile
           35                  40                  45
   cgc ttc gcc gtg agt gat gga ttg gaa ctg aga ccg aaa tat aat gga   192
   Arg Phe Ala Val Ser Asp Gly Leu Glu Leu Arg Pro Lys Tyr Asn Gly
       50                  55                  60
   att tta cat tgc ttg act acc att tgg aaa ctt gat gga cta cgg gga   240
   Ile Leu His Cys Leu Thr Thr Ile Trp Lys Leu Asp Gly Leu Arg Gly
   65                  70                  75                  80
   ctt tat caa gga gta acc cca aat ata tgg ggt gca ggt tta tcc tgg   288
   Leu Tyr Gln Gly Val Thr Pro Asn Ile Trp Gly Ala Gly Leu Ser Trp
                   85                  90                  95
   gga ctc tac ttt ttc ttt tac aat gcc atc aag tca tat aaa aca gaa   336
   Gly Leu Tyr Phe Phe Phe Tyr Asn Ala Ile Lys Ser Tyr Lys Thr Glu
               100                 105                 110
   gga aga gct gaa cgt tta gag gca aca gaa tac ctt gtc tca gct gct   384
   Gly Arg Ala Glu Arg Leu Glu Ala Thr Glu Tyr Leu Val Ser Ala Ala
           115                 120                 125
   gaa gct gga gcc atg acc ctc tgc att aca aac cca tta tgg gta aca   432
   Glu Ala Gly Ala Met Thr Leu Cys Ile Thr Asn Pro Leu Trp Val Thr
       130                 135                 140
   aaa act cgc ctt atg tta cag tat gat gct gtt gtt aac tcc cca cac   480
   Lys Thr Arg Leu Met Leu Gln Tyr Asp Ala Val Val Asn Ser Pro His
   145                 150                 155                 160
   ccg caa tat aaa gga atg ttt gat aca ctt gtg aaa ata tat aag tat   528
   Pro Gln Tyr Lys Gly Met Phe Asp Thr Leu Val Lys Ile Tyr Lys Tyr
                   165                 170                 175
   gaa ggt gtg cgt gga tta tat aag gga ttt gtt cct ggg ctg ttt gga   576
   Glu Gly Val Arg Gly Leu Tyr Lys Gly Phe Val Pro Gly Leu Phe Gly
               180                 185                 190
   aca tcg cac ggt gcc ctt cag ttt atg gca tat gaa ttg ctg aag ttg     624
   Thr Ser His Gly Ala Leu Gln Phe Met Ala Tyr Glu Leu Leu Lys Leu
           195                 200                 205
   aag tac aac cag cat atc aat aga tta cca gaa gcc cag ttg agc aca     672
   Lys Tyr Asn Gln His Ile Asn Arg Leu Pro Glu Ala Gln Leu Ser Thr
       210                 215                 220
   gta gaa tat ata tct gtt gca gca cta tcc aaa ata ttt gct gtc gca     720
   Val Glu Tyr Ile Ser Val Ala Ala Leu Ser Lys Ile Phe Ala Val Ala
   225                 230                 235                 240
   gca aca tac cca tat caa gtc gta aga gct cgt ctt cag gat caa cac     768
   Ala Thr Tyr Pro Tyr Gln Val Val Arg Ala Arg Leu Gln Asp Gln His
                   245                 250                 255
   atg ttt tac agt ggt gta ata gat gta atc aca aag aca tgg agg aaa     816
   Met Phe Tyr Ser Gly Val Ile Asp Val Ile Thr Lys Thr Trp Arg Lys
               260                 265                 270
   gaa ggc gtc ggt gga ttt tac aag gga att gct cct aat ttg att aga     864
   Glu Gly Val Gly Gly Phe Tyr Lys Gly Ile Ala Pro Asn Leu Ile Arg
           275                 280                 285
   gtg act cca gcc tgc tgt att acc ttt gtg gta tat gaa aac gtc tca     912
   Val Thr Pro Ala Cys Cys Ile Thr Phe Val Val Tyr Glu Asn Val Ser
       290                 295                 300
   cat ttt tta ctt gac ctt aga gaa aag aga aag taa                     948
   His Phe Leu Leu Asp Leu Arg Glu Lys Arg Lys
   305                 310                 315
<210> 10
   <211> 315
   <212> PRT
   <213> Homo sapiens
<400> 10
   Met Thr Gly Gln Gly Gln Ser Ala Ser Gly Ser Ser Ala Trp Ser Thr
    1               5                  10                  15
   Val Phe Arg His Val Arg Tyr Glu Asn Leu Ile Ala Gly Val Ser Gly
               20                  25                  30
   Gly Val Leu Ser Asn Leu Ala Leu His Pro Leu Asp Leu Val Lys Ile
           35                  40                  45
   Arg Phe Ala Val Ser Asp Gly Leu Glu Leu Arg Pro Lys Tyr Asn Gly
       50                  55                  60
   Ile Leu His Cys Leu Thr Thr Ile Trp Lys Leu Asp Gly Leu Arg Gly
   65                  70                  75                  80
   Leu Tyr Gln Gly Val Thr Pro Asn Ile Trp Gly Ala Gly Leu Ser Trp
                   85                  90                  95
   Gly Leu Tyr Phe Phe Phe Tyr Asn Ala Ile Lys Ser Tyr Lys Thr Glu
               100                 105                 110
   Gly Arg Ala Glu Arg Leu Glu Ala Thr Glu Tyr Leu Val Ser Ala Ala
           115                 120                 125
   Glu Ala Gly Ala Met Thr Leu Cys Ile Thr Asn Pro Leu Trp Val Thr
       130                 135                 140
   Lys Thr Arg Leu Met Leu Gln Tyr Asp Ala Val Val Asn Ser Pro His
   145                 150                 155                 160
   Pro Gln Tyr Lys Gly Met Phe Asp Thr Leu Val Lys Ile Tyr Lys Tyr
                   165                 170                 175
   Glu Gly Val Arg Gly Leu Tyr Lys Gly Phe Val Pro Gly Leu Phe Gly
               180                 185                 190
   Thr Ser His Gly Ala Leu Gln Phe Met Ala Tyr Glu Leu Leu Lys Leu
           195                 200                 205
   Lys Tyr Asn Gln His Ile Asn Arg Leu Pro Glu Ala Gln Leu Ser Thr
       210                 215                 220
   Val Glu Tyr Ile Ser Val Ala Ala Leu Ser Lys Ile Phe Ala Val Ala
   225                 230                 235                 240
   Ala Thr Tyr Pro Tyr Gln Val Val Arg Ala Arg Leu Gln Asp Gln His
                   245                 250                 255
   Met Phe Tyr Ser Gly Val Ile Asp Val Ile Thr Lys Thr Trp Arg Lys
               260                 265                 270
   Glu Gly Val Gly Gly Phe Tyr Lys Gly Ile Ala Pro Asn Leu Ile Arg
           275                 280                 285
   Val Thr Pro Ala Cys Cys Ile Thr Phe Val Val Tyr Glu Asn Val Ser
       290                 295                 300
   His Phe Leu Leu Asp Leu Arg Glu Lys Arg Lys
   305                 310                 315
<210> 11
   <211> 951
   <212> DNA
   <213> Mus musculus
<220>
   <221> CDS
   <222> (1)..(948)
<400> 11
   atg aca ggc cag ggc cag tcg gct gcc ggg tcg gcg gcg tgg agc gtg   48
   Met Thr Gly Gln Gly Gln Ser Ala Ala Gly Ser Ala Ala Trp Ser Val
    1               5                  10                  15
   gtg ttc cgc cac gtc cgg tac gag aac ctg gtg gct ggc gtg agt ggc   96
   Val Phe Arg His Val Arg Tyr Glu Asn Leu Val Ala Gly Val Ser Gly
               20                  25                  30
   ggg gtc ttg tcc aac ctg gcg ctg cac ccg ctc gac ctc gtg aag atc   144
   Gly Val Leu Ser Asn Leu Ala Leu His Pro Leu Asp Leu Val Lys Ile
           35                  40                  45
   cgc ttc gct gtg agt gat gga ctg gaa gta aga cca aaa tat aaa gga    192
   Arg Phe Ala Val Ser Asp Gly Leu Glu Val Arg Pro Lys Tyr Lys Gly
       50                  55                  60
   att ttg cat tgc ttg gct acc att tgg aaa gtt gat gga cta cga gga    240
   Ile Leu His Cys Leu Ala Thr Ile Trp Lys Val Asp Gly Leu Arg Gly
   65                  70                  75                  80
   ctt tat caa gga gta acc ccg aat gtg tgg ggt gcc ggt tta tcc tgg    288
   Leu Tyr Gln Gly Val Thr Pro Asn Val Trp Gly Ala Gly Leu Ser Trp
                   85                  90                  95
   gga ctc tac ttt ttc ttt tac aat gcc atc aaa tcg tat aag aca gag    336
   Gly Leu Tyr Phe Phe Phe Tyr Asn Ala Ile Lys Ser Tyr Lys Thr Glu
               100                 105                 110
   gga aga gct gaa cag tta gag cca tta gag tac ctc gtc tca gct gct    384
   Gly Arg Ala Glu Gln Leu Glu Pro Leu Glu Tyr Leu Val Ser Ala Ala
           115                 120                 125
   gaa gct gga gcc atg act ctg tgc att aca aac cca tta tgg gtg acg    432
   Glu Ala Gly Ala Met Thr Leu Cys Ile Thr Asn Pro Leu Trp Val Thr
       130                 135                 140
   aaa act cgc ctt atg tta caa tat ggt ggt gtt gct agc cct tca cag    480
   Lys Thr Arg Leu Met Leu Gln Tyr Gly Gly Val Ala Ser Pro Ser Gln
   145                 150                 155                 160
   aga cag tat aaa gga atg ttt gat gca ctt gtg aaa ata tat aaa tat    528
   Arg Gln Tyr Lys Gly Met Phe Asp Ala Leu Val Lys Ile Tyr Lys Tyr
                   165                 170                 175
   gaa ggt gtg cgt gga tta tac aag gga ttt gtc cct ggg ctg ttt gga    576
   Glu Gly Val Arg Gly Leu Tyr Lys Gly Phe Val Pro Gly Leu Phe Gly
               180                 185                 190
   aca tca cat ggt gcc ctt cag ttt atg gca tat gag ttg cta aag ttg    624
   Thr Ser His Gly Ala Leu Gln Phe Met Ala Tyr Glu Leu Leu Lys Leu
           195                 200                 205
   aag tac aac aaa cac atc aat aga tta ccg gaa gcc cag ctg agt aca    672
   Lys Tyr Asn Lys His Ile Asn Arg Leu Pro Glu Ala Gln Leu Ser Thr
       210                 215                 220
   gca gaa tac atc tct gtc gca gcg cta tcc aaa ata ttt gcc gta gca    720
   Ala Glu Tyr Ile Ser Val Ala Ala Leu Ser Lys Ile Phe Ala Val Ala
   225                 230                 235                 240
   gca aca tac ccg tat cag gtt gtg aga gcc cgc ctt cag gat cag cat    768
   Ala Thr Tyr Pro Tyr Gln Val Val Arg Ala Arg Leu Gln Asp Gln His
                   245                 250                 255
   gtg tct tat ggt ggt gta aca gat gtg atc aca aag acg tgg agg aaa    816
   Val Ser Tyr Gly Gly Val Thr Asp Val Ile Thr Lys Thr Trp Arg Lys
               260                 265                 270
   gaa ggc atc ggt gga ttt tac aaa gga att gcc ccc aat ctg att aga    864
   Glu Gly Ile Gly Gly Phe Tyr Lys Gly Ile Ala Pro Asn Leu Ile Arg
           275                 280                 285
   gtg act cca gcc tgc tgc atc acc ttt gtg gtt tat gaa aat gtc tct    912
   Val Thr Pro Ala Cys Cys Ile Thr Phe Val Val Tyr Glu Asn Val Ser
       290                 295                 300
   cac ttt tta tat gac ctt aga gaa aag aaa gtg ggc taa                    951
   His Phe Leu Tyr Asp Leu Arg Glu Lys Lys Val Gly
   305                 310                 315
<210> 12
   <211> 316
   <212> PRT
   <213> Mus musculus
<400> 12
   Met Thr Gly Gln Gly Gln Ser Ala Ala Gly Ser Ala Ala Trp Ser Val
   1               5                   10                  15
   Val Phe Arg His Val Arg Tyr Glu Asn Leu Val Ala Gly Val Ser Gly
               20                  25                  30
   Gly Val Leu Ser Asn Leu Ala Leu His Pro Leu Asp Leu Val Lys Ile
           35                  40                  45
   Arg Phe Ala Val Ser Asp Gly Leu Glu Val Arg Pro Lys Tyr Lys Gly
       50                  55                  60
   Ile Leu His Cys Leu Ala Thr Ile Trp Lys Val Asp Gly Leu Arg Gly
   65                  70                  75                  80
   Leu Tyr Gln Gly Val Thr Pro Asn Val Trp Gly Ala Gly Leu Ser Trp
                   85                  90                  95
   Gly Leu Tyr Phe Phe Phe Tyr Asn Ala Ile Lys Ser Tyr Lys Thr Glu
               100                 105                 110
   Gly Arg Ala Glu Gln Leu Glu Pro Leu Glu Tyr Leu Val Ser Ala Ala
           115                 120                 125
   Glu Ala Gly Ala Met Thr Leu Cys Ile Thr Asn Pro Leu Trp Val Thr
       130                 135                 140
   Lys Thr Arg Leu Met Leu Gln Tyr Gly Gly Val Ala Ser Pro Ser Gln
   145                 150                 155                 160
   Arg Gln Tyr Lys Gly Met Phe Asp Ala Leu Val Lys Ile Tyr Lys Tyr
                   165                 170                 175
   Glu Gly Val Arg Gly Leu Tyr Lys Gly Phe Val Pro Gly Leu Phe Gly
               180                 185                 190
   Thr Ser His Gly Ala Leu Gln Phe Met Ala Tyr Glu Leu Leu Lys Leu
           195                 200                 205
   Lys Tyr Asn Lys His Ile Asn Arg Leu Pro Glu Ala Gln Leu Ser Thr
       210                 215                 220
   Ala Glu Tyr Ile Ser Val Ala Ala Leu Ser Lys Ile Phe Ala Val Ala
   225                 230                 235                 240
   Ala Thr Tyr Pro Tyr Gln Val Val Arg Ala Arg Leu Gln Asp Gln His
                   245                 250                 255
   Val Ser Tyr Gly Gly Val Thr Asp Val Ile Thr Lys Thr Trp Arg Lys
               260                 265                 270
   Glu Gly Ile Gly Gly Phe Tyr Lys Gly Ile Ala Pro Asn Leu Ile Arg
   275 280 285
   Val Thr Pro Ala Cys Cys Ile Thr Phe Val Val Tyr Glu Asn Val Ser
   290 295 300
   His Phe Leu Tyr Asp Leu Arg Glu Lys Lys Val Gly
   305 310 315
<210> 13
   <211> 951
   <212> DNA
   <213> Mus musculus
<220>
   <221> CDS
   <222> (1)..(948)
<400> 13
   atg aca ggc cag ggc cag tcg gct gcc ggg tcg gcg gcg tgg agc gcg    48
   Met Thr Gly Gln Gly Gln Ser Ala Ala Gly Ser Ala Ala Trp Ser Ala
    1               5                  10                  15
   gtg ttc cgc cac gtc cgg tac gag aac ctg gtg gct ggc gtg agt ggc    96
   Val Phe Arg His Val Arg Tyr Glu Asn Leu Val Ala Gly Val Ser Gly
               20                  25                  30
   ggg gtc ttg tcc aac ctg gcg ctg cac ccg ctc gac ctc gtg aag atc    144
   Gly Val Leu Ser Asn Leu Ala Leu His Pro Leu Asp Leu Val Lys Ile
           35                  40                  45
   cgc ttc gct gtg agt gat gga ctg gaa gta aga cca aaa tat aaa gga    192
   Arg Phe Ala Val Ser Asp Gly Leu Glu Val Arg Pro Lys Tyr Lys Gly
       50                  55                  60
   att ttg cat tgc ttg gct acc att tgg aaa gtt gat gga cta cga gga    240
   Ile Leu His Cys Leu Ala Thr Ile Trp Lys Val Asp Gly Leu Arg Gly
   65                  70                  75                  80
   ctt tat caa gga gta acc ccg aat gtg tgg ggt gcc ggt tta tcc tgg    288
   Leu Tyr Gln Gly Val Thr Pro Asn Val Trp Gly Ala Gly Leu Ser Trp
                   85                  90                  95
   gga ctc tac ttt ttc ttt tac aat gcc atc aaa tcg tat aag aca gag    336
   Gly Leu Tyr Phe Phe Phe Tyr Asn Ala Ile Lys Ser Tyr Lys Thr Glu
               100                 105                 110
   gga aga gct gaa cag tta gag cca tta gag tac ctc gtc tca gct gct    384
   Gly Arg Ala Glu Gln Leu Glu Pro Leu Glu Tyr Leu Val Ser Ala Ala
           115                 120                 125
   gaa gct gga gcc atg act ctg tgc att aca aac cca tta tgg gtg acg    432
   Glu Ala Gly Ala Met Thr Leu Cys Ile Thr Asn Pro Leu Trp Val Thr
       130                 135                 140
   aaa act cgc ctt atg tta caa tat ggt ggt gtt gct agc cct tca cag    480
   Lys Thr Arg Leu Met Leu Gln Tyr Gly Gly Val Ala Ser Pro Ser Gln
   145                 150                 155                 160
   aga cag tat aaa gga atg ttt gat gca ctt gtg aat ata tat aaa tat    528
   Arg Gln Tyr Lys Gly Met Phe Asp Ala Leu Val Asn Ile Tyr Lys Tyr
                   165                     170                     175
   gaa ggt gtg cgt gga tta tac aag gga ttt gtc cct ggg ctg ttt gga      576
   Glu Gly Val Arg Gly Leu Tyr Lys Gly Phe Val Pro Gly Leu Phe Gly
               180                 185                 190
   aca tca cat ggt gcc ctt cag ttt atg gca tat gag ttg cta aag ttg      624
   Thr Ser His Gly Ala Leu Gln Phe Met Ala Tyr Glu Leu Leu Lys Leu
           195                 200                 205
   aag tac aac aaa cac atc aat aga tta ccg gaa gcc cag ctg agt aca      672
   Lys Tyr Asn Lys His Ile Asn Arg Leu Pro Glu Ala Gln Leu Ser Thr
       210                 215                 220
   gca gaa tac atc tct gtc gca gcg cta tcc aaa ata ttt gcc gta gca      720
   Ala Glu Tyr Ile Ser Val Ala Ala Leu Ser Lys Ile Phe Ala Val Ala
   225                 230                 235                 240
   gca aca tac ccg tat cag gtt gtg aga gcc cgc ctt cag gat cag cat      768
   Ala Thr Tyr Pro Tyr Gln Val Val Arg Ala Arg Leu Gln Asp Gln His
                   245                 250                 255
   gtg tct tat ggt ggt gta aca gat gtg atc aca aag acg tgg agg aaa      816
   Val Ser Tyr Gly Gly Val Thr Asp Val Ile Thr Lys Thr Trp Arg Lys
               260                 265                 270
   gaa ggc atc ggt gga ttt tac aaa gga att gcc ccc aat ctg att agg      864
   Glu Gly Ile Gly Gly Phe Tyr Lys Gly Ile Ala Pro Asn Leu Ile Arg
           275                 280                 285
   gtg act cca gcc tgc tgc atc acc ttt gtg gtt tat gaa aat gtc tct      912
   Val Thr Pro Ala Cys Cys Ile Thr Phe Val Val Tyr Glu Asn Val Ser
       290                 295                 300
   cac ttt tta tat gac ctt aga gaa aag aaa gtg agc taa                  951
   His Phe Leu Tyr Asp Leu Arg Glu Lys Lys Val Ser
   305                 310                 315
<210> 14
   <211> 316
   <212> PRT
   <213> Mus musculus
<400> 14
   Met Thr Gly Gln Gly Gln Ser Ala Ala Gly Ser Ala Ala Trp Ser Ala
    1               5                  10                  15
   Val Phe Arg His Val Arg Tyr Glu Asn Leu Val Ala Gly Val Ser Gly
               20                  25                  30
   Gly Val Leu Ser Asn Leu Ala Leu His Pro Leu Asp Leu Val Lys Ile
           35                  40                  45
   Arg Phe Ala Val Ser Asp Gly Leu Glu Val Arg Pro Lys Tyr Lys Gly
       50                  55                  60
   Ile Leu His Cys Leu Ala Thr Ile Trp Lys Val Asp Gly Leu Arg Gly
   65                  70                  75                  80
   Leu Tyr Gln Gly Val Thr Pro Asn Val Trp Gly Ala Gly Leu Ser Trp
                   85                  90                  95
   Gly Leu Tyr Phe Phe Phe Tyr Asn Ala Ile Lys Ser Tyr Lys Thr Glu
   100 105 110
   Gly Arg Ala Glu Gln Leu Glu Pro Leu Glu Tyr Leu Val Ser Ala Ala
   115 120 125
   Glu Ala Gly Ala Met Thr Leu Cys Ile Thr Asn Pro Leu Trp Val Thr
   130 135 140
   Lys Thr Arg Leu Met Leu Gln Tyr Gly Gly Val Ala Ser Pro Ser Gln
   145 150 155 160
   Arg Gln Tyr Lys Gly Met Phe Asp Ala Leu Val Asn Ile Tyr Lys Tyr
   165 170 175
   Glu Gly Val Arg Gly Leu Tyr Lys Gly Phe Val Pro Gly Leu Phe Gly
   180 185 190
   Thr Ser His Gly Ala Leu Gln Phe Met Ala Tyr Glu Leu Leu Lys Leu
   195 200 205
   Lys Tyr Asn Lys His Ile Asn Arg Leu Pro Glu Ala Gln Leu Ser Thr
   210 215 220
   Ala Glu Tyr Ile Ser Val Ala Ala Leu Ser Lys Ile Phe Ala Val Ala
   225 230 235 240
   Ala Thr Tyr Pro Tyr Gln Val Val Arg Ala Arg Leu Gln Asp Gln His
   245 250 255
   Val Ser Tyr Gly Gly Val Thr Asp Val Ile Thr Lys Thr Trp Arg Lys
   260 265 270
   Glu Gly Ile Gly Gly Phe Tyr Lys Gly Ile Ala Pro Asn Leu Ile Arg
   275 280 285
   Val Thr Pro Ala Cys Cys Ile Thr Phe Val Val Tyr Glu Asn Val Ser
   290 295 300
   His Phe Leu Tyr Asp Leu Arg Glu Lys Lys Val Ser
   305 310 315
<210> 15
   <211> 16
   <212> PRT
   <213> Drosophila melanogaster
<400> 15
   Leu Val Ala Gly Val Ser Gly Gly Val Val Ser Thr Leu Ile Leu His
   1 5 10 15
<210> 16
   <211> 28
   <212> PRT
   <213> Drosophila melanogaster
<400> 16
   Val Lys Tyr Glu His Leu Val Ala Gly Val Ser Gly Gly Val Val Ser
   1 5 10 15
   Thr Leu Ile Leu His Pro Leu Asp Leu Ile Lys Ile
   20 25
<210> 17
   <211> 23
   <212> PRT
   <213> Drosophila melanogaster
<400> 17
   Gly Val Thr Pro Asn Val Trp Gly Ser Gly Ser Ser Trp Gly Leu Tyr
   1 5 10 15
   Phe Met Phe Tyr Asn Thr Ile
   20
<210> 18
   <211> 31
   <212> PRT
   <213> Drosophila melanogaster
<400> 18
   Gly Leu Tyr Lys Gly Val Thr Pro Asn Val Trp Gly Ser Gly Ser Ser
   1 5 10 15
   Trp Gly Leu Tyr Phe Met Phe Tyr Asn Thr Ile Lys Thr Phe Ile
   20 25 30
<210> 19
   <211> 23
   <212> PRT
   <213> Drosophila melanogaster
<400> 19
   Met Asn Met Leu Ala Ala Ala Glu Ser Gly Ile Leu Thr Leu Leu Leu
   1 5 10 15
   Thr Asn Pro Ile Trp Val Val
   20
<210> 20
   <211> 27
   <212> PRT
   <213> Drosophila melanogaster
<400> 20
   Gly Pro Thr Met Asn Met Leu Ala Ala Ala Glu Ser Gly Ile Leu Thr
    1               5                  10                  15
   Leu Leu Leu Thr Asn Pro Ile Trp Val Val Lys
               20                  25
<210> 21
   <211> 23
   <212> PRT
   <213> Drosophila melanogaster
<400> 21
   Gly Leu Tyr Arg Gly Phe Val Pro Gly Met Leu Gly Val Ser His Gly
    1               5                  10                  15
   Ala Ile Gln Phe Met Thr Tyr
               20
<210> 22
   <211> 25
   <212> PRT
   <213> Drosophila melanogaster
<400> 22
   Arg Gly Leu Tyr Arg Gly Phe Val Pro Gly Met Leu Gly Val Ser His
    1               5                  10                  15
   Gly Ala Ile Gln Phe Met Thr Tyr Glu
               20                  25
<210> 23
   <211> 21
   <212> PRT
   <213> Drosophila melanogaster
<400> 23
   Glu Tyr Leu Ala Phe Ala Ala Val Ser Lys Leu Ile Ala Ala Ala Ala
    1               5                  10                  15
   Thr Tyr Pro Tyr Gln
               20
<210> 24
   <211> 28
   <212> PRT
   <213> Drosophila melanogaster
<400> 24
   Leu Ala Thr Thr Glu Tyr Leu Ala Phe Ala Ala Val Ser Lys Leu Ile
   1 5 10 15
   Ala Ala Ala Ala Thr Tyr Pro Tyr Gln Val Val Arg
   20 25
<210> 25
   <211> 22
   <212> PRT
   <213> Drosophila melanogaster
<400> 25
   Phe Tyr Lys Gly Leu Val Pro Tyr Leu Val His Val Thr Pro Asn Ile
   1 5 10 15
   Cys Met Val Met Leu Ile
   20
<210> 26
   <211> 24
   <212> PRT
   <213> Drosophila melanogaster
<400> 26
   Gly Phe Tyr Lys Gly Leu Val Pro Tyr Leu Val His Val Thr Pro Asn
   1 5 10 15
   Ile Cys Met Val Met Leu Ile Trp
   20
<210> 27
   <211> 16
   <212> PRT
   <213> Homo sapiens
<400> 27
   Leu Ile Ala Gly Val Ser Gly Gly Val Leu Ser Asn Leu Ala Leu His
   1 5 10 15
<210> 28
   <211> 26
   <212> PRT
   <213> Homo sapiens
<400> 28
   Tyr Glu Asn Leu Ile Ala Gly Val Ser Gly Gly Val Leu Ser Asn Leu
   1 5 10 15
   Ala Leu His Pro Leu Asp Leu Val Lys Ile
   20 25
<210> 29
   <211> 24
   <212> PRT
   <213> Homo sapiens
<400> 29
   Leu Tyr Gln Gly Val Thr Pro Asn Ile Trp Gly Ala Gly Leu Ser Trp
    1               5                  10                  15
   Gly Leu Tyr Phe Phe Phe Tyr Asn
                20
<210> 30
   <211> 27
   <212> PRT
   <213> Homo sapiens
<400> 30
   Gly Leu Tyr Gln Gly Val Thr Pro Asn Ile Trp Gly Ala Gly Leu Ser
    1               5                  10                  15
   Trp Gly Leu Tyr Phe Phe Phe Tyr Asn Ala Ile
                20                  25
<210> 31
   <211> 21
   <212> PRT
   <213> Homo sapiens
<400> 31
   Tyr Leu Val Ser Ala Ala Glu Ala Gly Ala Met Thr Leu Cys Ile Thr
    1               5                  10                  15
   Asn Pro Leu Trp Val
                20
<210> 32
   <211> 23
   <212> PRT
   <213> Homo sapiens
<400> 32
   Glu Tyr Leu Val Ser Ala Ala Glu Ala Gly Ala Met Thr Leu Cys Ile
    1               5                  10                  15
   Thr Asn Pro Leu Trp Val Thr
                20
<210> 33
   <211> 23
   <212> PRT
   <213> Homo sapiens
<400> 33
   Gly Leu Tyr Lys Gly Phe Val Pro Gly Leu Phe Gly Thr Ser His Gly
   1 5 10 15
   Ala Leu Gln Phe Met Ala Tyr
   20
<210> 34
   <211> 27
   <212> PRT
   <213> Homo sapiens
<400> 34
   Arg Gly Leu Tyr Lys Gly Phe Val Pro Gly Leu Phe Gly Thr Ser His
   1 5 10 15
   Gly Ala Leu Gln Phe Met Ala Tyr Glu Leu Leu
   20 25
<210> 35
   <211> 22
   <212> PRT
   <213> Homo sapiens
<400> 35
   Val Glu Tyr Ile Ser Val Ala Ala Leu Ser Lys Ile Phe Ala Val Ala
   1 5 10 15
   Ala Thr Tyr Pro Tyr Gln
   20
<210> 36
   <211> 29
   <212> PRT
   <213> Homo sapiens
<400> 36
   Gln Leu Ser Thr Val Glu Tyr Ile Ser Val Ala Ala Leu Ser Lys Ile
   1 5 10 15
   Phe Ala Val Ala Ala Thr Tyr Pro Tyr Gln Val Val Arg
   20 25
<210> 37
   <211> 19
   <212> PRT
   <213> Homo sapiens
<400> 37
   Gly Ile Ala Pro Asn Leu Ile Arg Val Thr Pro Ala Cys Cys Ile Thr
   1 5 10 15
   Phe Val Val
<210> 38
   <211> 27
   <212> PRT
   <213> Homo sapiens
<400> 38
   Gly Phe Tyr Lys Gly Ile Ala Pro Asn Leu Ile Arg Val Thr Pro Ala
    1               5                  10                  15
   Cys Cys Ile Thr Phe Val Val Tyr Glu Asn Val
               20                  25
<210> 39
   <211> 16
   <212> PRT
   <213> Mus musculus
<400> 39
   Leu Val Ala Gly Val Ser Gly Gly Val Leu Ser Asn Leu Ala Leu His
    1               5                  10                  15
<210> 40
   <211> 26
   <212> PRT
   <213> Mus musculus
<400> 40
   Tyr Glu Asn Leu Val Ala Gly Val Ser Gly Gly Val Leu Ser Asn Leu
    1               5                  10                  15
   Ala Leu His Pro Leu Asp Leu Val Lys Ile
               20                  25
<210> 41
   <211> 24
   <212> PRT
   <213> Mus musculus
<400> 41
   Leu Tyr Gln Gly Val Thr Pro Asn Val Trp Gly Ala Gly Leu Ser Trp
    1               5                  10                  15
   Gly Leu Tyr Phe Phe Phe Tyr Asn
               20
<210> 42
   <211> 27
   <212> PRT
   <213> Mus musculus
<400> 42
   Gly Leu Tyr Gln Gly Val Thr Pro Asn Val Trp Gly Ala Gly Leu Ser
   1               5                   10                  15
   Trp Gly Leu Tyr Phe Phe Phe Tyr Asn Ala Ile
               20                  25
<210> 43
   <211> 21
   <212> PRT
   <213> Mus musculus
<400> 43
   Tyr Leu Val Ser Ala Ala Glu Ala Gly Ala Met Thr Leu Cys Ile Thr
   1               5                   10                  15
   Asn Pro Leu Trp Val
               20
<210> 44
   <211> 23
   <212> PRT
   <213> Mus musculus
<400> 44
   Glu Tyr Leu Val Ser Ala Ala Glu Ala Gly Ala Met Thr Leu Cys Ile
   1               5                   10                  15
   Thr Asn Pro Leu Trp Val Thr
               20
<210> 45
   <211> 23
   <212> PRT
   <213> Mus musculus
<400> 45
   Gly Leu Tyr Lys Gly Phe Val Pro Gly Leu Phe Gly Thr Ser His Gly
   1               5                   10                  15
   Ala Leu Gln Phe Met Ala Tyr
               20
<210> 46
   <211> 27
   <212> PRT
   <213> Mus musculus
<400> 46
   Arg Gly Leu Tyr Lys Gly Phe Val Pro Gly Leu Phe Gly Thr Ser His
   1               5                   10                  15
   Gly Ala Leu Gln Phe Met Ala Tyr Glu Leu Leu
               20                  25
<210> 47
   <211> 22
   <212> PRT
   <213> Mus musculus
<400> 47
   Ala Glu Tyr Ile Ser Val Ala Ala Leu Ser Lys Ile Phe Ala Val Ala
    1               5                  10                  15
   Ala Thr Tyr Pro Tyr Gln
               20
<210> 48
   <211> 29
   <212> PRT
   <213> Mus musculus
<400> 48
   Gln Leu Ser Thr Ala Glu Tyr Ile Ser Val Ala Ala Leu Ser Lys Ile
    1               5                  10                  15
   Phe Ala Val Ala Ala Thr Tyr Pro Tyr Gln Val Val Arg
               20                  25
<210> 49
   <211> 19
   <212> PRT
   <213> Mus musculus
<400> 49
   Gly Ile Ala Pro Asn Leu Ile Arg Val Thr Pro Ala Cys Cys Ile Thr
    1               5                  10                  15
   Phe Val Val
<210> 50
   <211> 27
   <212> PRT
   <213> Mus musculus
<400> 50
   Gly Phe Tyr Lys Gly Ile Ala Pro Asn Leu Ile Arg Val Thr Pro Ala
    1               5                  10                  15
   Cys Cys Ile Thr Phe Val Val Tyr Glu Asn Val
               20                  25
<210> 51
   <211> 975
   <212> DNA
   <213> Danio rerio
<220>
   <221> CDS
   <222> (1)..(972)
<400> 51
   atg acc gct acc att tcc agg cag agg cac gcc gca gtc gct gca gac      48
   Met Thr Ala Thr Ile Ser Arg Gln Arg His Ala Ala Val Ala Ala Asp
    1               5                  10                  15
   tct tct ggc tct tct ttt tcc att aca gca aac ctc ctg caa ctt tca      96
   Ser Ser Gly Ser Ser Phe Ser Ile Thr Ala Asn Leu Leu Gln Leu Ser
               20                  25                  30
   aaa cac atc aaa tat gag aat ctt gca gcc gga ctc gct ggt ggt gtt     144
   Lys His Ile Lys Tyr Glu Asn Leu Ala Ala Gly Leu Ala Gly Gly Val
           35                  40                  45
   att tcc aca atg gtg cta cat cca ttg gat ttg atc aaa atc agg ttt     192
   Ile Ser Thr Met Val Leu His Pro Leu Asp Leu Ile Lys Ile Arg Phe
       50                  55                  60
   gca gta agt gat ggt ctg aaa atg agg ccc caa tac gat ggc atg tta     240
   Ala Val Ser Asp Gly Leu Lys Met Arg Pro Gln Tyr Asp Gly Met Leu
   65                  70                  75                  80
   gac tgc atg aag acc atc tgg aag ctg gaa ggc att aga ggt ctc tat     288
   Asp Cys Met Lys Thr Ile Trp Lys Leu Glu Gly Ile Arg Gly Leu Tyr
                   85                  90                  95
   cag gga gtg acg ccc aac atc tgg ggg gcc gga tca tca tgg ggc ctc     336
   Gln Gly Val Thr Pro Asn Ile Trp Gly Ala Gly Ser Ser Trp Gly Leu
               100                 105                 110
   tac ttc ctc ttt tat aat gct att aaa gca tac aca cag gag gga cgg     384
   Tyr Phe Leu Phe Tyr Asn Ala Ile Lys Ala Tyr Thr Gln Glu Gly Arg
           115                 120                 125
   caa aca gag ctg agt gca tgt gaa cac ctg gtg tcc gca gcg gag gca     432
   Gln Thr Glu Leu Ser Ala Cys Glu His Leu Val Ser Ala Ala Glu Ala
       130                 135                 140
   ggc att ctg acg ctt tgc ctc acc aat cca gtc tgg gtg aca aag acc     480
   Gly Ile Leu Thr Leu Cys Leu Thr Asn Pro Val Trp Val Thr Lys Thr
   145                 150                 155                 160
   cgg ctg gtg ctg cag tac aat gca gac cct tca cgg aag cag tac aag     528
   Arg Leu Val Leu Gln Tyr Asn Ala Asp Pro Ser Arg Lys Gln Tyr Lys
                   165                 170                 175
   gga atg atg gac gcc ctc gtg aaa ata tac cgt cac gag ggt atc cca     576
   Gly Met Met Asp Ala Leu Val Lys Ile Tyr Arg His Glu Gly Ile Pro
               180                 185                 190
   gga cta tac agg ggt ttt gtg cct ggg ctg gtc ggg act tcc cat gct     624
   Gly Leu Tyr Arg Gly Phe Val Pro Gly Leu Val Gly Thr Ser His Ala
           195                 200                 205
   gca ctg cag ttc atg acc tat gaa ggg cta aaa aga gag cag aac aaa     672
   Ala Leu Gln Phe Met Thr Tyr Glu Gly Leu Lys Arg Glu Gln Asn Lys
       210                 215                 220
   tgc aag aag atg ccc tct gaa tcc ctg ctg tcc cca ttg gaa tac atc     720
   Cys Lys Lys Met Pro Ser Glu Ser Leu Leu Ser Pro Leu Glu Tyr Ile
   225                 230                 235                 240
   gcc ata gca gcc ata tcc aaa ata ttc gct gta gca gta aca tac ccc     768
   Ala Ile Ala Ala Ile Ser Lys Ile Phe Ala Val Ala Val Thr Tyr Pro
                   245                 250                 255
   tat cag gtg gtc cgc gct cgc ctg cag gac cag cac aac aac tac agt     816
   Tyr Gln Val Val Arg Ala Arg Leu Gln Asp Gln His Asn Asn Tyr Ser
               260                 265                 270
   gga ata gtg gat gtc atg aga agg acc tgg agc aac gaa ggg gtg gag     864
   Gly Ile Val Asp Val Met Arg Arg Thr Trp Ser Asn Glu Gly Val Glu
           275                 280                 285
   ggc ttt tac aaa ggg atg gtg cca aac ctg gtc cga gtc att cct gcg     912
   Gly Phe Tyr Lys Gly Met Val Pro Asn Leu Val Arg Val Ile Pro Ala
       290                 295                 300
   tgc tgc atc acc ttc ctg gtg ttc gaa aat gtg tca cgc ttg ctt ttg     960
   Cys Cys Ile Thr Phe Leu Val Phe Glu Asn Val Ser Arg Leu Leu Leu
   305                 310                 315                 320
   ggc gag tat cac taa                                                 975
   Gly Glu Tyr His
<210> 52
   <211> 324
   <212> PRT
   <213> Danio rerio
<400> 52
   Met Thr Ala Thr Ile Ser Arg Gln Arg His Ala Ala Val Ala Ala Asp
   1 5 10 15
   Ser Ser Gly Ser Ser Phe Ser Ile Thr Ala Asn Leu Leu Gln Leu Ser
   20 25 30
   Lys His Ile Lys Tyr Glu Asn Leu Ala Ala Gly Leu Ala Gly Gly Val
   35 40 45
   Ile Ser Thr Met Val Leu His Pro Leu Asp Leu Ile Lys Ile Arg Phe
   50 55 60
   Ala Val Ser Asp Gly Leu Lys Met Arg Pro Gln Tyr Asp Gly Met Leu
   65 70 75 80
   Asp Cys Met Lys Thr Ile Trp Lys Leu Glu Gly Ile Arg Gly Leu Tyr
   85 90 95
   Gln Gly Val Thr Pro Asn Ile Trp Gly Ala Gly Ser Ser Trp Gly Leu
   100 105 110
   Tyr Phe Leu Phe Tyr Asn Ala Ile Lys Ala Tyr Thr Gln Glu Gly Arg
   115 120 125
   Gln Thr Glu Leu Ser Ala Cys Glu His Leu Val Ser Ala Ala Glu Ala
   130 135 140
   Gly Ile Leu Thr Leu Cys Leu Thr Asn Pro Val Trp Val Thr Lys Thr
   145                 150                 155                 160
   Arg Leu Val Leu Gln Tyr Asn Ala Asp Pro Ser Arg Lys Gln Tyr Lys
                   165                 170                 175
   Gly Met Met Asp Ala Leu Val Lys Ile Tyr Arg His Glu Gly Ile Pro
               180                 185                 190
   Gly Leu Tyr Arg Gly Phe Val Pro Gly Leu Val Gly Thr Ser His Ala
           195                 200                 205
   Ala Leu Gln Phe Met Thr Tyr Glu Gly Leu Lys Arg Glu Gln Asn Lys
       210                 215                 220
   Cys Lys Lys Met Pro Ser Glu Ser Leu Leu Ser Pro Leu Glu Tyr Ile
   225                 230                 235                 240
   Ala Ile Ala Ala Ile Ser Lys Ile Phe Ala Val Ala Val Thr Tyr Pro
                   245                 250                 255
   Tyr Gln Val Val Arg Ala Arg Leu Gln Asp Gln His Asn Asn Tyr Ser
               260                 265                 270
   Gly Ile Val Asp Val Met Arg Arg Thr Trp Ser Asn Glu Gly Val Glu
           275                 280                 285
   Gly Phe Tyr Lys Gly Met Val Pro Asn Leu Val Arg Val Ile Pro Ala
       290                 295                 300
   Cys Cys Ile Thr Phe Leu Val Phe Glu Asn Val Ser Arg Leu Leu Leu
   305                 310                 315                 320
   Gly Glu Tyr His
<210> 53
   <211> 969
   <212> DNA
   <213> Drosophila melanogaster
<220>
   <221> CDS
   <222> (1)..(969)
<400> 53
   atg aat ccg atc aag gca cag tca acg ggc agt ccc aag aaa ttc aac   48
   Met Asn Pro Ile Lys Ala Gln Ser Thr Gly Ser Pro Lys Lys Phe Asn
    1               5                  10                  15
   gta ttc gca cac gtc aag tac gag cat ttg gtt gcc gga gta tcc ggc   96
   Val Phe Ala His Val Lys Tyr Glu His Leu Val Ala Gly Val Ser Gly
               20                  25                  30
   gga gtg gtg tcc aca ctc att cta cat ccc ctg gat ttg atc aag att   144
   Gly Val Val Ser Thr Leu Ile Leu His Pro Leu Asp Leu Ile Lys Ile
           35                  40                  45
   cga ttc gca gtt aac gat ggc cgg aca gct acg gtg ccg caa tac cgg   192
   Arg Phe Ala Val Asn Asp Gly Arg Thr Ala Thr Val Pro Gln Tyr Arg
       50                  55                  60
   gga ctg agc agc gcc ttc acc acg att ttc cgg caa gag ggc ttc cgc    240
   Gly Leu Ser Ser Ala Phe Thr Thr Ile Phe Arg Gln Glu Gly Phe Arg
    65                 70                  75                  80
   gga ctc tac aaa ggc gtc acc ccc aat gtc tgg gga tcg ggc tcc tct    288
   Gly Leu Tyr Lys Gly Val Thr Pro Asn Val Trp Gly Ser Gly Ser Ser
                    85                  90                  95
   tgg ggc ctg tac ttc atg ttc tac aac acc att aag aca ttt atc caa    336
   Trp Gly Leu Tyr Phe Met Phe Tyr Asn Thr Ile Lys Thr Phe Ile Gln
               100                 105                 110
   gga gga aac acg acc atg cca ttg ggc ccc aca atg aac atg ctt gca    384
   Gly Gly Asn Thr Thr Met Pro Leu Gly Pro Thr Met Asn Met Leu Ala
           115                 120                 125
   gct gct gag tcg gga att ctc acc ctg ctg ctg acc aac ccc atc tgg    432
   Ala Ala Glu Ser Gly Ile Leu Thr Leu Leu Leu Thr Asn Pro Ile Trp
       130                 135                 140
   gtg gtg aag acg cgt ctc tgc ctg cag tgc gat gcc gcg agt agt gcc    480
   Val Val Lys Thr Arg Leu Cys Leu Gln Cys Asp Ala Ala Ser Ser Ala
   145                 150                 155                 160
   gag tac agg ggc atg atc cac gcc ttg ggc cag ata tac aag gag gag    528
   Glu Tyr Arg Gly Met Ile His Ala Leu Gly Gln Ile Tyr Lys Glu Glu
                   165                 170                 175
   gga atc cgt ggc ctg tac cgc ggc ttt gtt ccc ggc atg ttg ggc gtc    576
   Gly Ile Arg Gly Leu Tyr Arg Gly Phe Val Pro Gly Met Leu Gly Val
               180                 185                 190
   tcc cac gga gcc atc cag ttc atg acc tac gag gag ctg aag aac gcc    624
   Ser His Gly Ala Ile Gln Phe Met Thr Tyr Glu Glu Leu Lys Asn Ala
           195                 200                 205
   tac aac gaa tat cgc aaa ctg ccc atc gac acg aag ctg gcc acc acc    672
   Tyr Asn Glu Tyr Arg Lys Leu Pro Ile Asp Thr Lys Leu Ala Thr Thr
       210                 215                 220
   gag tac ttg gcc ttc gcg gct gtc tcc aag ctg atc gca gcg gcg gcc    720
   Glu Tyr Leu Ala Phe Ala Ala Val Ser Lys Leu Ile Ala Ala Ala Ala
   225                 230                 235                 240
   acc tac ccg tac cag gtg gtc cgg gca cgg ctg cag gac cac cat cac    768
   Thr Tyr Pro Tyr Gln Val Val Arg Ala Arg Leu Gln Asp His His His
                   245                 250                 255
   cga tac aac ggc acc tgg gac tgc atc aaa cag act tgg agg ttt gag    816
   Arg Tyr Asn Gly Thr Trp Asp Cys Ile Lys Gln Thr Trp Arg Phe Glu
               260                 265                 270
   ggc tac aga ggc ttc tac aag ggg ctg aag gcg agt tta acc cga gta    864
   Gly Tyr Arg Gly Phe Tyr Lys Gly Leu Lys Ala Ser Leu Thr Arg Val
           275                 280                 285
   gtg cct gcc tgc atg gtc acc ttt ctg gtg tac gag aac gtc tcg cat    912
   Val Pro Ala Cys Met Val Thr Phe Leu Val Tyr Glu Asn Val Ser His
       290                 295                 300
   ttc ctg ctc gcc agg cgg aag cga att gag act aaa gag gat gcg tcg    960
   Phe Leu Leu Ala Arg Arg Lys Arg Ile Glu Thr Lys Glu Asp Ala Ser
   305 310 315 320
   gac gtg tga 969
   Asp Val
<210> 54
   <211> 322
   <212> PRT
   <213> Drosophila melanogaster
<400> 54
   Met Asn Pro Ile Lys Ala Gln Ser Thr Gly Ser Pro Lys Lys Phe Asn
   1 5 10 15
   Val Phe Ala His Val Lys Tyr Glu His Leu Val Ala Gly Val Ser Gly
   20 25 30
   Gly Val Val Ser Thr Leu Ile Leu His Pro Leu Asp Leu Ile Lys Ile
   35 40 45
   Arg Phe Ala Val Asn Asp Gly Arg Thr Ala Thr Val Pro Gln Tyr Arg
   50 55 60
   Gly Leu Ser Ser Ala Phe Thr Thr Ile Phe Arg Gln Glu Gly Phe Arg
   65 70 75 80
   Gly Leu Tyr Lys Gly Val Thr Pro Asn Val Trp Gly Ser Gly Ser Ser
   85 90 95
   Trp Gly Leu Tyr Phe Met Phe Tyr Asn Thr Ile Lys Thr Phe Ile Gln
   100 105 110
   Gly Gly Asn Thr Thr Met Pro Leu Gly Pro Thr Met Asn Met Leu Ala
   115 120 125
   Ala Ala Glu Ser Gly Ile Leu Thr Leu Leu Leu Thr Asn Pro Ile Trp
   130 135 140
   Val Val Lys Thr Arg Leu Cys Leu Gln Cys Asp Ala Ala Ser Ser Ala
   145 150 155 160
   Glu Tyr Arg Gly Met Ile His Ala Leu Gly Gln Ile Tyr Lys Glu Glu
   165 170 175
   Gly Ile Arg Gly Leu Tyr Arg Gly Phe Val Pro Gly Met Leu Gly Val
   180 185 190
   Ser His Gly Ala Ile Gln Phe Met Thr Tyr Glu Glu Leu Lys Asn Ala
   195 200 205
   Tyr Asn Glu Tyr Arg Lys Leu Pro Ile Asp Thr Lys Leu Ala Thr Thr
   210 215 220
   Glu Tyr Leu Ala Phe Ala Ala Val Ser Lys Leu Ile Ala Ala Ala Ala
   225 230 235 240
   Thr Tyr Pro Tyr Gln Val Val Arg Ala Arg Leu Gln Asp His His His
   245 250 255
   Arg Tyr Asn Gly Thr Trp Asp Cys Ile Lys Gln Thr Trp Arg Phe Glu
   260 265 270
   Gly Tyr Arg Gly Phe Tyr Lys Gly Leu Lys Ala Ser Leu Thr Arg Val
   275 280 285
   Val Pro Ala Cys Met Val Thr Phe Leu Val Tyr Glu Asn Val Ser His
   290 295 300
   Phe Leu Leu Ala Arg Arg Lys Arg Ile Glu Thr Lys Glu Asp Ala Ser
   305 310 315 320
   Asp Val
<210> 55
   <211> 23
   <212> DNA
   <213> Artificial Sequence
<220>
   <223> Description of Artificial Sequence: PCR primers
<400> 55
   atggactacg gggactttat caa 23
<210> 56
   <211> 21
   <212> DNA
   <213> Artificial Sequence
<220>
   <223> Description of Artificial Sequence: PCR primers
<400> 56
   accgacgcct tctttccaac t 21
<210> 57
   <211> 18
   <212> DNA
   <213> Artificial Sequence
<220>
   <223> Description of Artificial Sequence: PCR primers
<400> 57
   ggcggccact acatcacg 18
<210> 58
   <211> 20
   <212> DNA
   <213> Artificial Sequence
<220>
   <223> Description of Artificial Sequence: PCR primers
<400> 58
   tgcctcaaga acatagactg 20
<210> 59
   <211> 30
   <212> DNA
   <213> Artificial Sequence
<220>
   <223> Description of Artificial Sequence: PCR primers
<400> 59
   atgaccgcta ccatttccag gcagaggcac 30
<210> 60
   <211> 30
   <212> DNA
   <213> Artificial Sequence
<220>
   <223> Description of Artificial Sequence: PCR primers
<400> 60
   ttagtgatac tcgcccaaaa gcaagcgtga 30
<210> 61
   <211> 22
   <212> PRT
   <213> Drosophila melanogaster
<400> 61
   Phe Tyr Lys Gly Leu Lys Ala Ser Leu Thr Arg Val Val Pro Ala Cys
   1               5                   10                  15
   Met Val Thr Phe Leu Val
               20
<210> 62
   <211> 24
   <212> PRT
   <213> Drosophila melanogaster
<400> 62
   Gly Phe Tyr Lys Gly Leu Lys Ala Ser Leu Thr Arg Val Val Pro Ala
    1               5                  10                  15
   Cys Met Val Thr Phe Leu Val Tyr
               20
<210> 63
   <211> 18
   <212> DNA
   <213> Artificial Sequence
<220>
   <223> Description of Artificial Sequence: mSOUP gene specific primer
<400> 63
   ggcggccact acatcacg 18
<210> 64
   <211> 45
   <212> DNA
   <213> Artificial Sequence
<220>
   <223> Description of Artificial Sequence: mSOUP gene specific primer
<400> 64
   ctacttgtca tcatcgtcct tgtagtcgct cactttcttt tctct 45
<210> 65
   <211> 19
   <212> DNA
   <213> Artificial Sequence
<220>
   <223> Description of Artificial Sequence: mSOUP gene specific primer
<400> 65
   ctctgtcgca gcgctatcc 19
<210> 66
   <211> 19
   <212> DNA
   <213> Artificial Sequence
<220>
   <223> Description of Artificial Sequence: mSOUP gene specific primer
<400> 66
   gaaggcgggc tctcacaac 19
<210> 67
   <211> 29
   <212> DNA
   <213> Artificial Sequence
<220>
   <223> Description of Artificial Sequence: mSOUP gene specific primer
<400> 67
   aatatttgcc gtagcagcaa catacccgt 29
<210> 68
   <211> 18
   <212> PRT
   <213> Artificial Sequence
<220>
   <223> Description of Artificial Sequence: synthetic pepide
<400> 68
   Cys Tyr Glu Asn Val Ser His Phe Leu Tyr Asp Leu Arg Glu Lys Lys
    1               5                  10                  15
   Val Ser